# EUROPEAN PATENT SPECIFICATION

(11) **EP 2 854 825 B1**
(45) Date of publication and mention of the grant of the patent: **31.10.2018**
(21) Application number: 13727699.4
(22) Date of filing: 14.05.2013
(51) Int. Cl.: A61K 35/48, A61P 11/00

(54) **hUTC MODULATION OF PRO-INFLAMMATORY MEDIATORS OF LUNG AND PULMONARY DISEASES AND DISORDERS**
HUTC-MODULATION VON ENTZÜNDUNGSFÖRDERNDEN MEDIATOREN DER LUNGE UND LUNGENERKRANKUNGEN SOWIE -STÖRUNGEN
MODULATION DE HUTC DE MÉDIATEURS PRO-INFLAMMATOIRES DE MALADIES ET DE TROUBLES DES POUMONS ET PULMONAIRES

(30) Priority: 14.05.2012 US 201213471095
(43) Date of publication of application: 08.04.2015
(73) Proprietor: DePuy Synthes Products, Inc., Raynham MA 02767 (US)
(72) Inventor: KIHM, Anthony J., Princeton, New Jersey 08540 (US)
(74) Representative: Carpmaels & Ransford LLP
(86) International application number: PCT/US2013/041002
(87) International publication number: WO 2013/173376

(56) References cited:
- US-A1- 2010 158 877
- WEISS ET AL: "Stem cells and cell therapies for cystic fibrosis and other lung diseases", PULMORNARY PHARMACOLOGY & THERAPEUTICS, ACADEMIC PRESS, GB, vol. 21, no. 4, 1 August 2008 (2008-08-01) , pages 588-594, XP022932220, ISSN: 1094-5539, DOI: 10.1016/J.PUPT.2007.11.004 [retrieved on 2008-01-18]
- JIANJUN LI ET AL: "Human umbilical cord mesenchymal stem cells reduce systemic inflammation and attenuate LPS-induced acute lung injury in rats", JOURNAL OF INFLAMMATION, BIOMED CENTRAL, LONDON, GB, vol. 9, no. 1, 13 September 2012 (2012-09-13), page 33, XP021121588, ISSN: 1476-9255, DOI: 10.1186/1476-9255-9-33
- EUN SUN KIM ET AL: "Intratracheal transplantation of human umbilical cord blood-derived mesenchymal stem cells attenuates Escherichia coli-induced acute lung injury in mice", RESPIRATORY RESEARCH, BIOMED CENTRAL LTD., LONDON, GB, vol. 12, no. 1, 15 August 2011 (2011-08-15), page 108, XP021091812, ISSN: 1465-9921, DOI: 10.1186/1465-9921-12-108
- Yuben Moodley ET AL: "Stem Cells, Tissue Engineering and Hematopoietic Elements Human Umbilical Cord Mesenchymal Stem Cells Reduce Fibrosis of Bleomycin-Induced Lung Injury", , 1 January 2009 (2009-01-01), pages 303-313080629, XP55306600, Retrieved from the Internet: URL:https://www.ncbi.nlm.nih.gov/pmc/artic les/PMC2708816/pdf/JPATH175000303.pdf
- WEISS MARK L ET AL: "Human umbilical cord matrix stem cells: preliminary characterization and effect of transplantation in a rodent model of Parkinson's disease", STEM CELLS, ALPHAMED PRESS, DAYTON, OH, US, vol. 24, no. 3, 1 March 2006 (2006-03-01), pages 781-792, XP002463574, ISSN: 1066-5099, DOI: 10.1634/STEMCELLS.2005-0330
- Hwai-Shi Wang ET AL: "Mesenchymal Stem Cells in the Wharton's Jelly of the Human Umbilical Cord", Stem Cells, vol. 22, no. 7, 1 December 2004 (2004-12-01), pages 1330-1337, XP055178069, ISSN: 1066-5099, DOI: 10.1634/stemcells.2004-0013
- MITCHELL K E ET AL: "Matrix cells from Wharton's jelly form neurons and glia", STEM CELLS, ALPHAMED PRESS, DAYTON, OH, US, vol. 21, no. 1, 1 January 2003 (2003-01-01), pages 50-60, XP002281007, ISSN: 1066-5099
- DATABASE MEDLINE [Online] US NATIONAL LIBRARY OF MEDICINE (NLM), BETHESDA, MD, US; 2012, GRUBEK-JAWORSKA HANNA ET AL: "IL-6 and IL-13 in induced sputum of COPD and asthma patients: correlation with respiratory tests.", Database accession no. NLM22311051
- DATABASE MEDLINE [Online] US NATIONAL LIBRARY OF MEDICINE (NLM), BETHESDA, MD, US; 13 March 1993 (1993-03-13), DONNELLY S C ET AL: "Interleukin-8 and development of adult respiratory distress syndrome in at-risk patient groups.", Database accession no. NLM8095568
- DATABASE MEDLINE [Online] US NATIONAL LIBRARY OF MEDICINE (NLM), BETHESDA, MD, US; November 2009 (2009-11), DI STEFANO A ET AL: "Association of increased CCL5 and CXCL7 chemokine expression with neutrophil activation in severe stable COPD.", Database accession no. NLM19703829
- KENNELLY HELEN ET AL: "Human mesenchymal stromal cells exert HGF dependent cytoprotective effects in a human relevant pre-clinical model of COPD.", 6 December 2016 (2016-12-06), SCIENTIFIC REPORTS 06 DEC 2016, VOL. 6, PAGE(S) 38207 ISSN: 2045-2322

### SEQUENCE LISTING

<110> KIHM, ANTHONY J.
<120> hUTC MODULATION OF PRO-INFLAMMATORY MEDIATORS OF LUNG AND PULMONARY
   DISEASES AND DISORDERS
<130> 18668-329484
<160> 10
<170> PatentIn version 3.5
<210> 1
   <211> 22
   <212> DNA
   <213> Artificial Sequence
<220>
   <223> Description of Artificial Sequence: Synthetic primer
<400> 1
   gagaaatcca aagagcaaat gg 22
<210> 2
   <211> 21
   <212> DNA
   <213> Artificial Sequence
<220>
   <223> Description of Artificial Sequence: Synthetic primer
<400> 2
   agaatggaaa actggaatag g 21
<210> 3
   <211> 20
   <212> DNA
   <213> Artificial Sequence
<220>
   <223> Description of Artificial Sequence: Synthetic primer
<400> 3
   tcttcgatgc ttcggattcc 20
<210> 4
   <211> 21
   <212> DNA
   <213> Artificial Sequence
<220>
   <223> Description of Artificial Sequence: Synthetic primer
<400> 4
   gaattctcgg aatctctgtt g 21
<210> 5
   <211> 21
   <212> DNA
   <213> Artificial Sequence
<220>
   <223> Description of Artificial Sequence: Synthetic primer
<400> 5
   ttacaagcag tgcagaaaac c 21
<210> 6
   <211> 22
   <212> DNA
   <213> Artificial Sequence
<220>
   <223> Description of Artificial Sequence: Synthetic primer
<400> 6
   agtaaacatt gaaaccacag cc 22
<210> 7
   <211> 20
   <212> DNA
   <213> Artificial Sequence
<220>
   <223> Description of Artificial Sequence: Synthetic primer
<400> 7
   tctgcagctc tgtgtgaagg 20
<210> 8
   <211> 22
   <212> DNA
   <213> Artificial Sequence
<220>
   <223> Description of Artificial Sequence: Synthetic primer
<400> 8
   cttcaaaaac ttctccacaa cc 22
<210> 9
   <211> 17
   <212> DNA
   <213> Artificial Sequence
<220>
   <223> Description of Artificial Sequence: Synthetic primer
<400> 9
   cccacgccac gctctcc 17
<210> 10
   <211> 19
   <212> DNA
   <213> Artificial Sequence
<220>
   <223> Description of Artificial Sequence: Synthetic primer
<400> 10
   tcctgtcagt tggtgctcc 19

## Description

### FIELD OF THE INVENTION

The invention relates a cell based therapy for modulation of proinflammatory mediators of lung and pulmonary diseases and disorders.

### BACKGROUND OF THE INVENTION

Lung diseases (both chronic and acute), disorders, and/or injuries remain a significant cause of morbidity and mortality throughout the world. Chronic obstructive pulmonary disease (COPD) is the fourth leading cause of death in the world (Spurzem and Rennard, Semin Respir Crit Care Med, 2005; 26: 142-153) and can be caused by anatomic narrowing of the airways or blocking of airways with mucus that interferes with normal breathing. Additionally, interstitial lung disease, also known as pulmonary fibrosis, is classified as a restrictive disease that includes a variety of chronic lung disorders. Management of chronic lung diseases includes drug therapy, oxygen therapy, surgery, and pulmonary rehabilitation.

While 90% of COPD patients are smokers, only 10% of smokers develop the disease, suggesting that genetic predisposition may be an important prognostic factor (Siafakas and Tzortzaki, Respir Med, 2002; 96: 615-24). Smoker's lung disease is characterized by chronic active inflammation, airway mucus hypersecretion, and emphysema (MacNee, Proc Am Thorac Soc. 2005; 2: 258-66) and is only partially reversible upon cessation of smoking (Spurzem and Rennard, Semin Respir Crit Care Med, 2005; 26: 142-153). Inflammation of the airways and lung parenchyma plays a major role in the pathogenesis of COPD. Cigarette smoke has been shown to induce pulmonary inflammation and ultimately lead to COPD even if cigarette smoke exposure stopped.

Emphysema is one of the major factors determining morbidity and mortality in COPD. Emphysema is defined as the enlargement of peripheral air space in the lung (including respiratory bronchioles and alveoli), which is accompanied by the destruction of alveolar wall structures, and is characterized, for example, by loss of lung tissue elasticity from destruction of structures supporting the lung tissues such as *e.g.* alveoli, and destruction of capillaries feeding the alveoli. Inflammatory enzymes, such as *e.g.* elastin, can cause this destruction. The incidence of emphysema has increased because of increasing environmental pollutants, cigarette smoking, and other exposure to noxious substances. Currently, the only remediation for severe emphysema is lung transplantation. Accordingly, a great need remains for an adequate and useful approach to treat, repair, and/or ameliorate lung damage in patients with emphysema, such as elastase-induced emphysema.

Acute lung injury (ALI) and acute respiratory distress syndrome (ARDS) are significant causes of morbidity and mortality in the intensive care setting and are characterized by the abrupt onset of hypoxemia with diffuse pulmonary edema in response to either direct injury (e.g., drowning, pneumonia, inhaled toxic gases, and pulmonary contusion) or indirect injury (e.g., severe sepsis, transfusion, shock, and pancreatitis). Mechanical ventilation and supportive care are current treatments for ALI and ARDS.

Restrictive lung disease is one of the most common causes of morbidity and mortality and has three primary etiologies: lung cancer, pneumonia, and pulmonary fibrosis. Idiopathic pulmonary fibrosis (IPF) is a crippling disease characterized by progressive dyspnea and is associated with a high mortality rate progressive fixed tissue fibrosis, architectural distortion, and loss of function (Ortiz L.A. et al., Proc Natl Acad Sci U.S.A., 2003; 100:8407-11). Presently, no effective therapies to reverse or retard the course of IPF are available. Most treatments, such as corticosteroids, immunosuppressive, immunomodulatory, or antifibrotic agents, seek to suppress inflammation, but none has been proven to alter disease progression. Therefore, a significant need exists for novel therapies aimed at slowing or halting fibrosis while enhancing endogenous lung repair and regeneration.

Numerous pro-inflammatory mediators have been implicated in pathology of a lung disease, disorder, and/or injury such as *e.g.* in respiratory disease. For example in pulmonary fibrosis, a chronic form of fibrosing interstitial pneumonia, an excess of profibrotic cytokines or a deficiency in antifibrotic cytokines has been implicated in the disease's pathologic process. (Zhao F. et al. Transplant Proceedings, 2008; 40(5):1700-1705).

As such, the reduction of production and/or inhibition of these cytokines and pro-inflammatory mediators may reduce the symptoms and/or pathology of a lung disease, disorder, and/or injury. Accordingly, there is currently a great need for treatments that reduce the production or inhibit the production of these pro-inflammatory mediators. Current treatments of lung diseases, disorders, and/or injuries such as *e.g.* COPD include inhaled or oral corticosteroids, bronchodilators and anticholinergics. In addition, the use of interleukin antagonists and antibodies against interleukins has been studied, including in clinical trials, for *e.g.* asthma. However, none of these treatments provide for reduction of production and/or inhibition of pro-inflammatory mediators involved in the symptoms and/or pathology of a lung disease, disorder, and/or injury.

Transplantation of stem cells can be utilized as a clinical tool for reconstituting a target tissue, thereby restoring physiologic and anatomic functionality. In the treatment of lung diseases (both chronic and acute), disorders, and/or injuries, the focus has been predominantly on using stem cell technology to regenerate or repair lung tissue damaged by lung disease, disorder, and/or injury.

Thus, there is need for a way of treating lung disease, disorders, and/or injuries by modulating the mediators associated with the pathology of the lung disease, disorder, and/or injury. In particular what is needed is a method of continually modulating the prof-inflammatory mediators associated with the pathology of a lung disease, disorder and/or injury,

### SUMMARY OF THE INVENTION

The invention provides umbilical cord tissue-derived cells for use in treating chronic obstructive pulmonary disease (COPD) by reducing the production of one or more pro-inflammatory mediators involved in the pathology of COPD, wherein the one or more pro-inflammatory mediators is selected from the group consisting of TNF-α, RANTES, IL-1β and combinations thereof, and wherein the cells are isolated from human umbilical cord tissue substantially free of blood, are capable of self-renewal and expansion in culture, lack the production of CD117 and CD45, and do not express hTERT or telomerase.

Herein disclosed are methods of modulating (*e.g*. reducing) the production of a pro-inflammatory mediator involved in the pathology of a lung disease, disorder, and/or injury in a patient having the lung disease, disorders, and/or injuries. Such diseases, disorders, and/or injuries include, but are not limited to, chronic obstructive pulmonary diseases (COPD) (*e.g.* chronic bronchitis, emphysema), pulmonary fibrosis, acute lung injury (ALI), acute respiratory distress syndrome (ARDS), and the damages associated thereto.

One embodiment of the disclosure is a method of modulating the production of one or more pro-inflammatory mediators involved in the pathology of a lung disease, disorder, and/or injury in a patient having the lung disease, disorder, and/or injury comprising administering to the patient an effective amount of umbilical cord tissue-derived cells. Another embodiment is a method of reducing the production of one or more pro-inflammatory mediators involved in the pathology of a lung disease, disorder, and/or injury in a patient having the lung disease, disorder, and/or injury comprising administering to the patient an effective amount of umbilical cord tissue-derived cells (*e.g.* in amount effective to reduce the production of the one or more pro-inflammatory mediators).

The methods utilize cells isolated from human umbilical cord tissue substantially free of blood, which are capable of self-renewal and expansion in culture, lack the production of CD117 or CD45, and do not express hTERT or telomerase. In one embodiment, the cells lack production of CD117 and CD45 and, optionally, also do not express hTERT and telomerase. In another embodiment, the cells do not express hTERT and telomerase. In yet another embodiment, the cells are isolated from human umbilical cord tissue substantially free of blood, are capable of self-renewal and expansion in culture, lack the production of CD117 or CD45, and do not express hTERT or telomerase and one or more following characteristics: express CD10, CD13, CD44, CD73, and CD90; do not express CD31 or CD34; express, relative to a human fibroblast, mesenchymal stem cell, or iliac crest bone marrow cell, increased levels of interleukin 8 or reticulon 1; and have the potential to differentiate into cells of at least a lung tissue.

The methods are suitable for modulating (*e.g*. reducing) the production of many of the pro-inflammatory mediators of lung diseases, disorders, and/or injuries. In one embodiment, the pro-inflammatory mediators are TNF-α, RANTES, MCP-1, IL-1β and combinations thereof. The pro-inflammatory mediator may be involved in the progress of the lung disease, disorder, and/or injury.

In one embodiment, the cells are administered with at least one other cell type and/or at least one other agent. The other cell type may be a lung cell such as *e.g.* a progenitor cell, a vascular smooth muscle cell, a vascular smooth muscle progenitor cell, a pericyte, vascular endothelial cell, a vascular endothelium progenitor cell, or other multipotent or pluripotent stem cell. The agent may be selected from an antithrombogenic agent, an anti-inflammatory agent, an immunosuppressive agent, an immunomodulatory agent, a pro-angiogenic agent, or an antiapoptotic agent.

Herein also disclosed is a method of modulating (*e.g*. reducing) the production of one or more pro-inflammatory mediators of a chronic obstructive pulmonary disease (such as *e.g.* emphysema or chronic bronchitis) in a patient having the chronic obstructive pulmonary disease, comprising an effective amount of umbilical cord tissue-derived cells, wherein said pro-inflammatory mediator mediates the progress of the chronic obstructive pulmonary disease, and wherein the cells are isolated from human umbilical cord tissue substantially free of blood, are capable of self-renewal and expansion in culture, lack the production of CD117 and CD45, and do not express hTERT or telomerase. One embodiment is a method of reducing the production of one or more pro-inflammatory mediators of a chronic obstructive pulmonary disease (such as *e.g.* emphysema or chronic bronchitis) in a patient having the chronic obstructive pulmonary disease, comprising an effective amount of umbilical cord tissue-derived cells, wherein said pro-inflammatory mediator mediates the progress of the chronic obstructive pulmonary disease, and wherein the cells are isolated from human umbilical cord tissue substantially free of blood, are capable of self-renewal and expansion in culture, lack the production of CD117 and CD45, and do not express hTERT or telomerase. The one or more pro-inflammatory mediators may be TNF-α, RANTES, MCP-1, IL-1β and combinations thereof.

In another embodiment, the cells may further have one or more of the following characteristics: express CD10, CD13, CD44, CD73, and CD90; do not express CD31 or CD34; express, relative to a human fibroblast, mesenchymal stem cell, or iliac crest bone marrow cell, increased levels of interleukin 8 or reticulon 1; and have the potential to differentiate into cells of at least a lung tissue.

In some embodiments, the umbilical cord tissue-derived cells are formulated in a pharmaceutical composition comprising a pharmaceutically acceptable carrier. Alternatively, the cells are formulated in a kit that contains a pharmaceutically acceptable carrier. The methods may inhibit the production of the one or more pro-inflammatory mediators.

Herein also disclosed is a method of inhibiting production of one or more pro-inflammatory mediators of chronic obstructive pulmonary disease in a patient having the chronic obstructive pulmonary disease, comprising an effective amount of umbilical cord tissue-derived cells. The one or more pro-inflammatory mediators may be selected from the group consisting of TNF-α, RANTES, MCP-1, IL-1β and combinations thereof. In one embodiment, the COPD is chronic bronchitis or emphysema.

The umbilical cord tissue-derived cells are isolated from human umbilical cord tissue substantially free of blood, are capable of self-renewal and expansion in culture, lack the production of CD117 and CD45, and do not express hTERT or telomerase. Optionally, the cells further have one or more of the following characteristics: express CD10, CD13, CD44, CD73, and CD90; do not express CD31 or CD34; express, relative to a human fibroblast, mesenchymal stem cell, or iliac crest bone marrow cell, increased levels of interleukin 8 or reticulon 1; and have the potential to differentiate into cells of at least a lung tissue.

In one embodiment, the cells are administered at the sites of the chronic obstructive pulmonary disease.

In certain embodiments, the cells are induced *in vitro* to differentiate into lung tissue cells, such as *e.g.* vascular smooth muscle, pericyte, or vascular endothelium lineage cells, prior to administration. In other embodiments, the cells are genetically engineered to produce a gene product that promotes treatment of a lung disease, disorder, and/or injury.

In some embodiments of the methods, the cells are administered with at least one other cell type, which may include lung tissue cells, such as *e.g.* lung progenitor cells, vascular smooth muscle cells, vascular smooth muscle progenitor cells, pericytes, vascular endothelial cells, vascular endothelium progenitor cells, or other multipotent or pluripotent stem cells. The other cell type can administered simultaneously with, before, or after, the umbilical cord tissue-derived cells.

In other embodiments, the cells are administered with at least one other agent, which may be an antithrombogenic agent, an anti-inflammatory agent, an immunosuppressive agent, an immunomodulatory agent, pro-angiogenic, or an antiapoptotic agent, for example. The other agent can be administered simultaneously with, before, or after, the umbilical cord tissue-derived cells.

The cells are preferably administered at or proximal to the sites of the lung disease, disorder, and/or injury, but can also be administered at locations distal to such sites. They can be administered by injection, infusion, a device implanted in the patient, or by implantation of a matrix or scaffold containing the cells. The cells may exert a trophic effect, such as proliferation, on the lung tissue of the patient. The cells may induce migration of lung tissue cells, such as *e.g.* vascular smooth muscle cells, vascular endothelial cells, lung progenitor cells, pericytes, vascular smooth muscle progenitor cells, or vascular endothelium progenitor cells to the site or sites of lung disease, disorder, and/or injury.

Herein also disclosed is a pharmaceutical composition for modulating the production of one or more pro-inflammatory mediators involved in the pathology of a lung disease, disorder, and/or injury comprising umbilical cord tissue-derived cells, wherein the cells are isolated from human umbilical cord tissue substantially free of blood, are capable of self-renewal and expansion in culture, lack the production of CD117 and CD45, and do not express hTERT or telomerase. In one embodiment, the pharmaceutical composition reduces the production of one or more pro-inflammatory mediators involved in the pathology of a lung disease, disorder, and/or injury comprising umbilical cord tissue-derived cells. In another embodiment, the pharmaceutical composition inhibits the production of one or more pro-inflammatory mediators involved in the pathology of a lung disease, disorder, and/or injury comprising umbilical cord tissue-derived cells. Other aspects of the disclosure feature treatment with pharmaceutical compositions and kits comprising products of the umbilical cord tissue-derived cells.

The pharmaceutical composition may comprise a pharmaceutically acceptable carrier, diluent, and/or buffer. The lung disease may be a chronic obstructive pulmonary disease such as *e.g.* chronic bronchitis or emphysema.

Yet another embodiment of the disclosure is a kit for modulating the production of one or more pro-inflammatory mediators involved in the pathology of a lung disease, disorder, and/or injury comprising a pharmaceutically acceptable carrier and umbilical cord tissue-derived cells. Another embodiment is kit for reducing the production of one or more pro-inflammatory mediators involved in the pathology of a lung disease, disorder, and/or injury comprising a pharmaceutically acceptable carrier and umbilical cord tissue-derived cells. In one embodiment, the umbilical cord tissue-derived cells are isolated from human umbilical cord tissue substantially free of blood, are capable of self-renewal and expansion in culture, lack the production of CD117 and CD45, and do not express hTERT or telomerase. The lung disease may be a chronic obstructive pulmonary disease such as *e.g.* chronic bronchitis or emphysema.

Other features and advantages of the invention will be understood by reference to the detailed description and examples that follow.

### BRIEF DESCRIPTION OF THE DRAWINGS

The foregoing summary, as well as the following detailed description of the invention, will be better understood when read in conjunction with the appended figures. For the purpose of illustrating the invention, the figures demonstrate embodiments of the present invention.

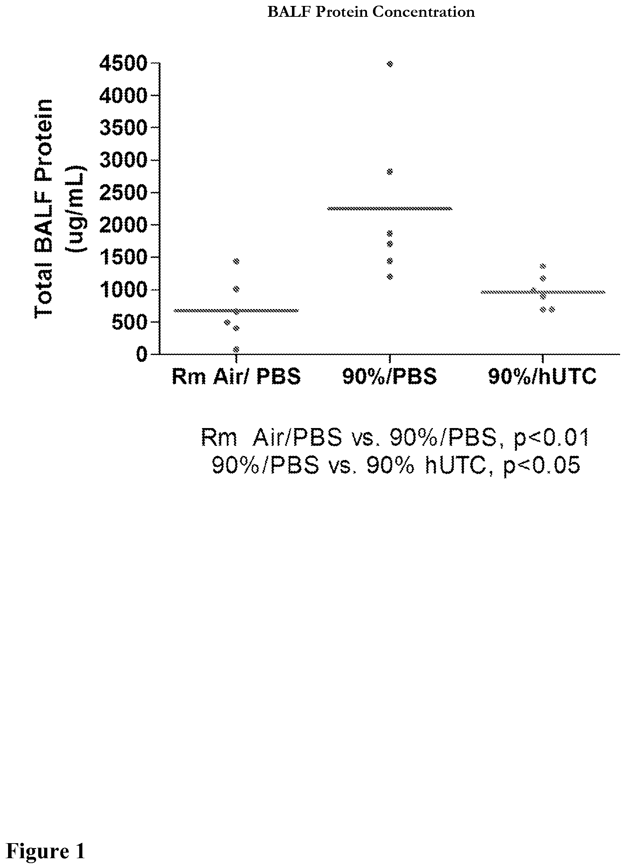

Figure 1 shows the BALF total protein concentration. Total protein was measured using Pierce BCA Protein Assay. Each data point represents measurements obtained from a single animal. The horizontal line represents the average of all measurements. Student T-test analysis was performed. The data is shown in tabular form below in Table 1.2.

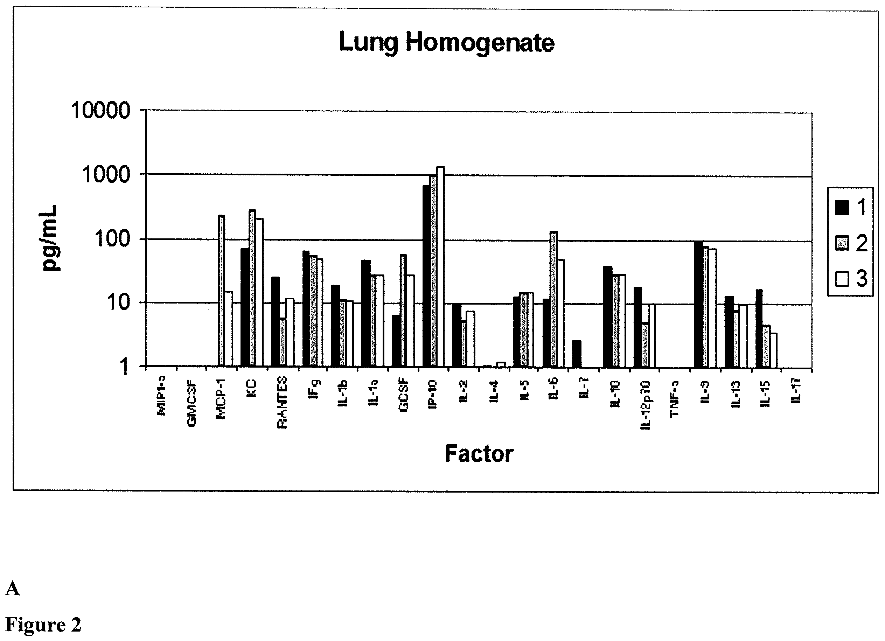

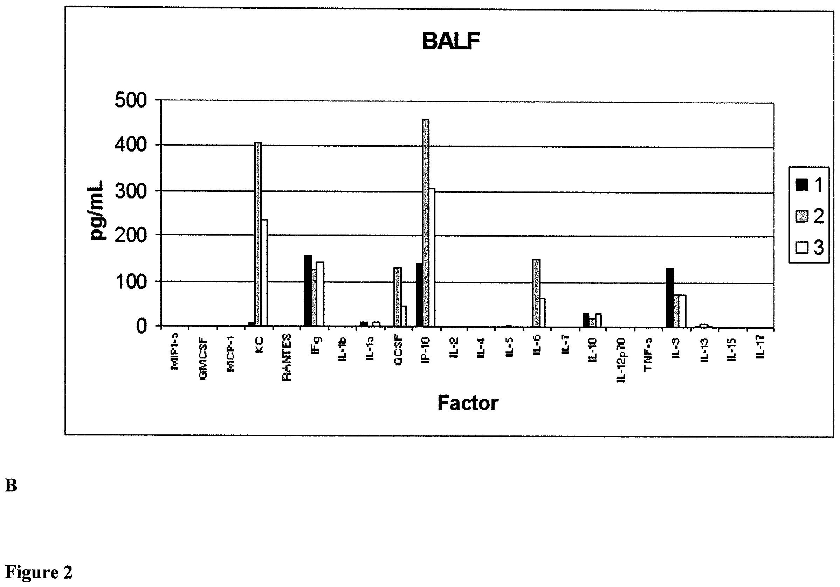

Figures 2 shows the results of the cytokine/chemokine analysis. Figure 2A shows a cytokine/chemokine analysis of Lung Homogenate: The concentrations of twenty-two different cytokines/chemokines were determined for lung homogenate using a mouse 22-multiplex bead kit (Millipore) following the manufacturer's protocol and analyzed using the BioRad Bioplex machine. Data bars represent the mean of six samples. The data is shown in tabular form in Tables 1-3 and 1-4. Figure 2B shows a cytokine/chemokine analysis of BALF. The concentrations of twenty-two different cytokines/chemokines were determined for BALF (Bronchoalveolar lavage fluid) using a mouse 22-multiplex bead kit (Millipore) following the manufacturer's protocol and analyzed using the BioRad Bioplex machine. Data bars represent the mean of six samples. The data is shown in tabular form in Tables 1-5 and 1-6.

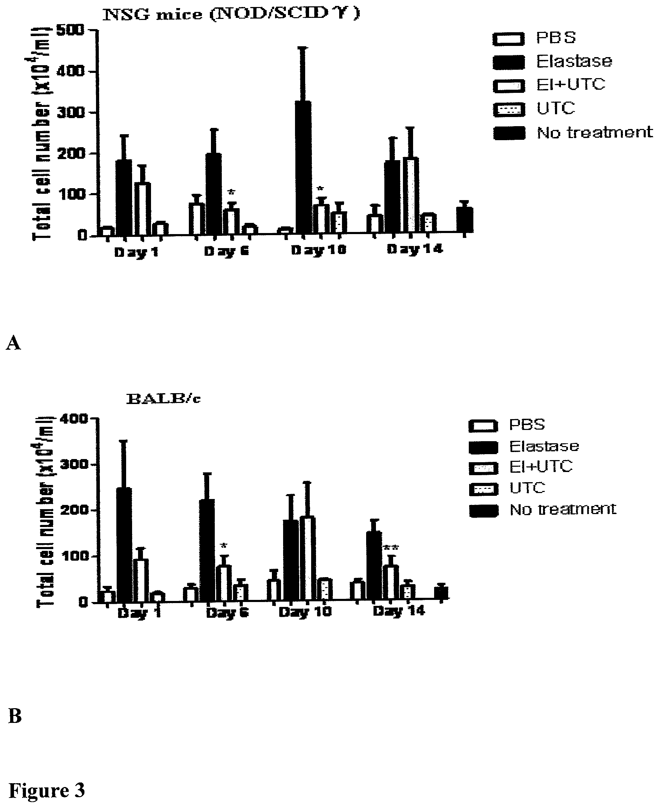

Figure 3 shows the effect of hUTC infusion and/or porcine pancreatic elastase (PPE) treatment on Bronchoalveolar lavage (BAL) fluid composition at 1, 6, 10 and 14 days following human umbilical cord tissue-derived cell (hUTC) administration. Figure 3A shows the results for NSG mice and Figure 3B shows the results for BALB/c mice. Negative controls were sham infected/sensitized with saline and/or vehicle. BAL fluid was examined for the total cell number. In each case, five (5) animals were assessed. Results are expressed as mean ± S.E.M. of cell number. (*, p < 0.05, ***, p<0.001).

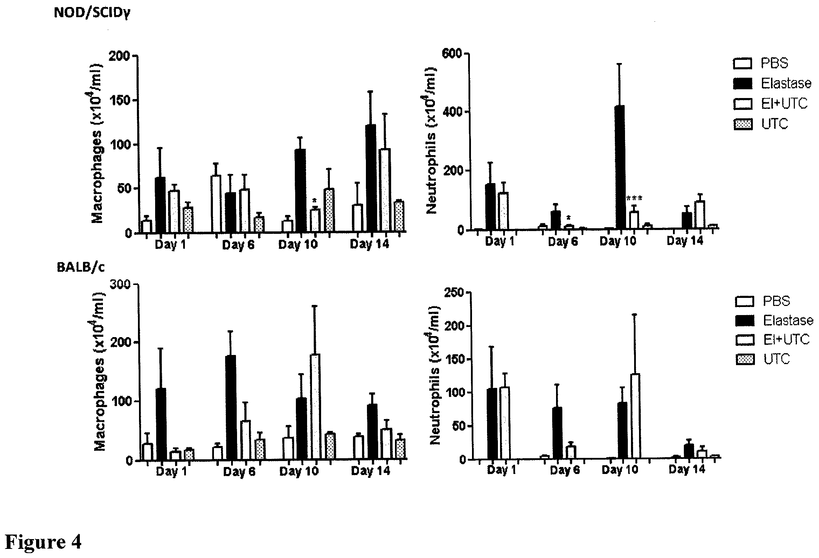

Figure 4 shows the effect of hUTC infusion and/or porcine pancreatic elastase (PPE) treatment on BAL fluid composition at 1, 6, 10 and 14 days following hUTC administration. Negative controls were sham sensitized with saline and/or vehicle. BAL fluid was examined for the presence of neutrophils and macrophages. In each case, five (5) animals were assessed. Results are expressed as mean ± S.E.M. of cell number. (*, p < 0.05, ***, p<0.001).

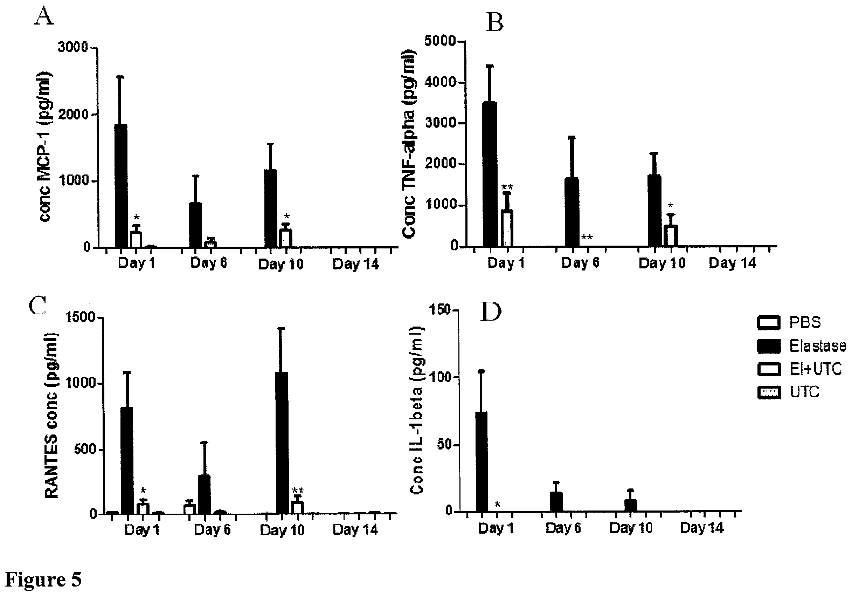

Figure 5 shows the effect of hUTC infusion and/or porcine pancreatic elastase (PPE) treatment in BAL supernatant cytokine composition at 1, 6, 10 and 14 days following hUTC administration to NOD/SCIDy mice. Figure 5A shows the cytokine response for MCP-1. Figure 5B shows the cytokine response for TNF-α. Figure 5C shows the response for TNF-α and Figure 5D shows the response for IL-1β. The responses were determined independently from five mice per group and are expressed as means ± S.E.M. (*, p < 0.05).

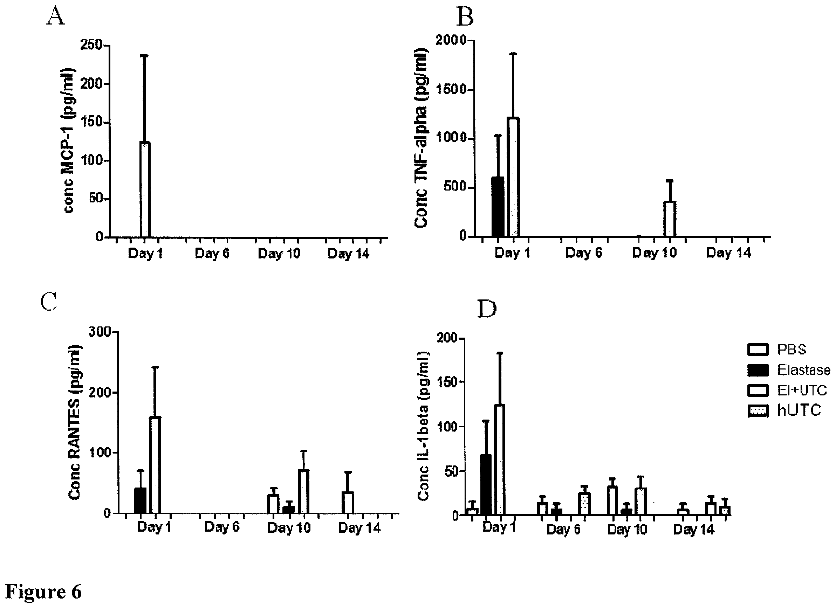

Figure 6 shows the effect of hUTC infusion and/or porcine pancreatic elastase (PPE) treatment in BAL supernatant composition at 1, 6, 10 and 14 days following hUTC administration in BALB/c mice. Cytokine responses are shown for MCP-1 (*see* Figure 6A) TNF-α (*see* Figure 6B), RANTES (*see* Figure 6C) and IL-1β (*see* Figure 6D). The responses were determined independently from five (5) mice per group and are expressed as means ± S.E.M. (*, p < 0.05).

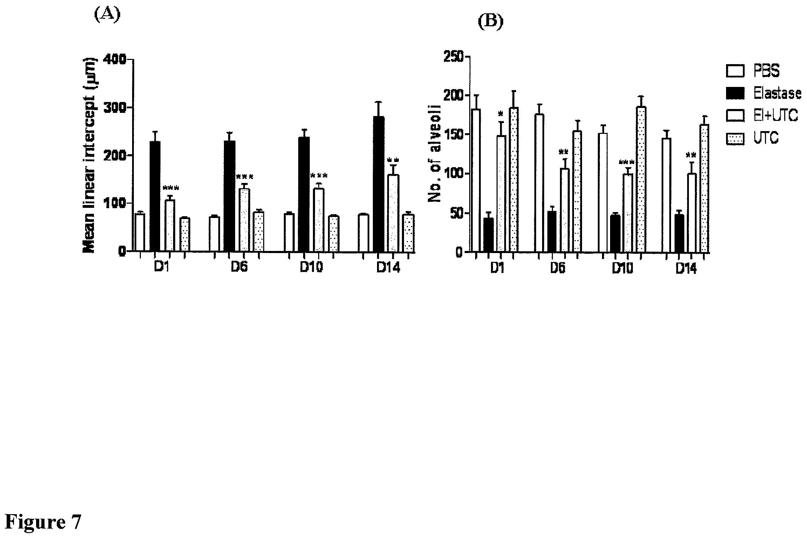

Figures 7 shows the mean linear intercept (A) and the number of alveoli (B) measured at a magnification of x 100 and expressed as mean ± SD from five (5) animals per group and time period. Symbols indicate the results of statistical analysis: *p < 0.01, **p, 0.005, ***p, 0.001 compared to Elastase group.

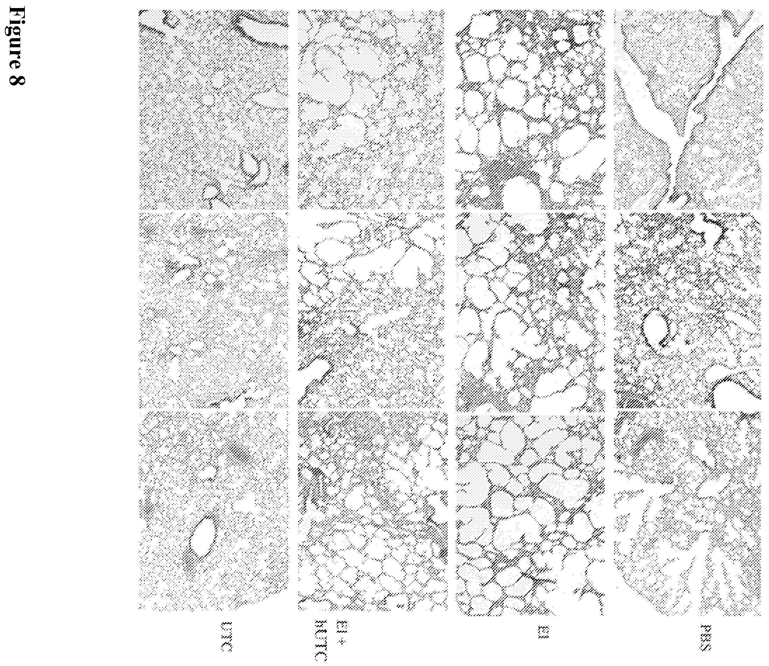

Figure 8 shows the detection of emphysematous damage using haematoxylin and eosin (H&E) staining of fixed lung sections (n = 5 per group) from control mice (PBS) or mice receiving Elastase (E1), Elastase +hUTC therapy (El+hUTC) or hUTC alone. Three (3) representative slides shown for each sample. Original magnification x100.

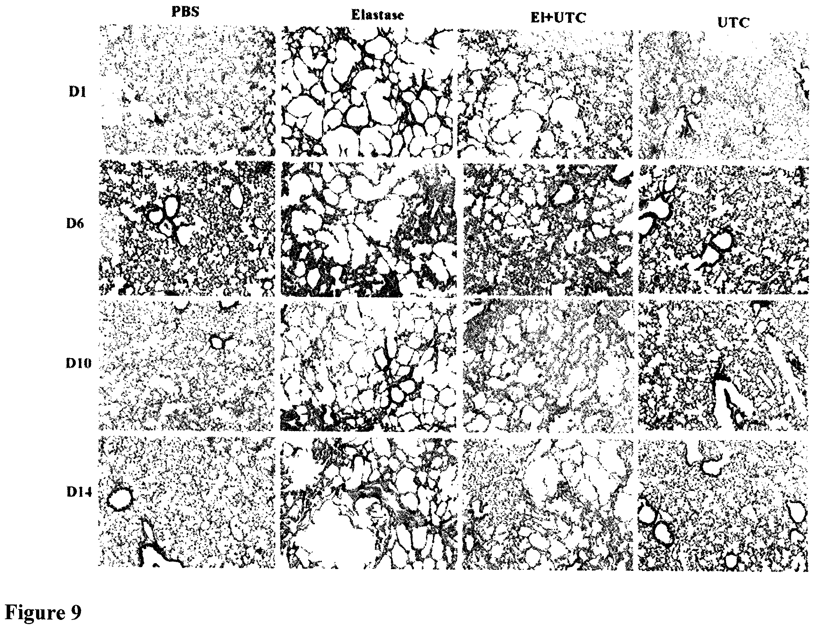

Figure 9 shows that hUTC reduced the extent of elastase-induced emphysema in immuno-compromised (NOD/SCIDy) mice at day 1, 6, 10, and 14. Emphysematous damage was detected using haematoxylin and eosin (H&E) staining of fixed lung sections (n = 5 per group). Original magnification x 100.

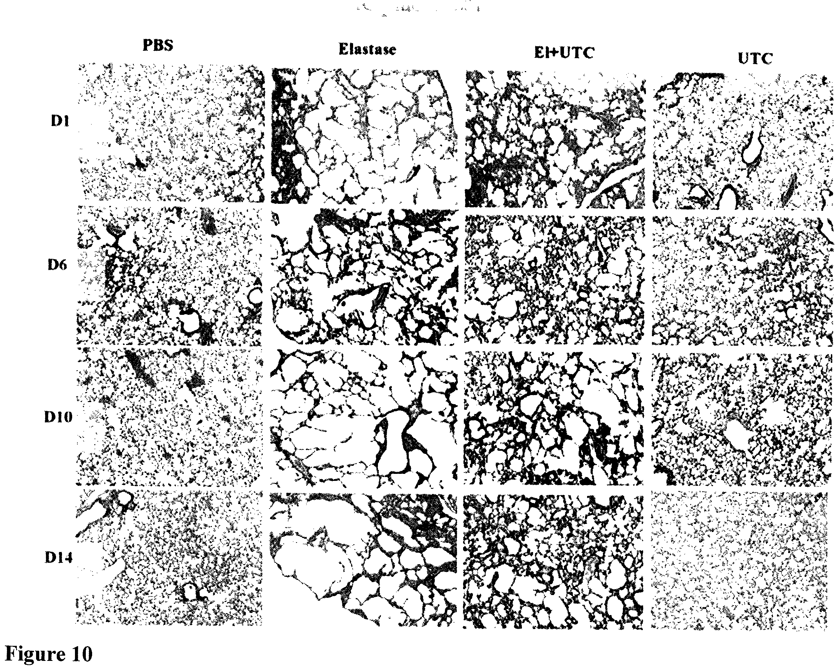

Figure 10 shows that hUTC reduced the extent of elastase-induced emphysema in immuno-competent (BALB/c) mice at day 1, 6, 10, and 14. Emphysematous damage was detected using haematoxylin and eosin (H&E) staining of fixed lung sections (n = 5 per group). Original magnification x 100.

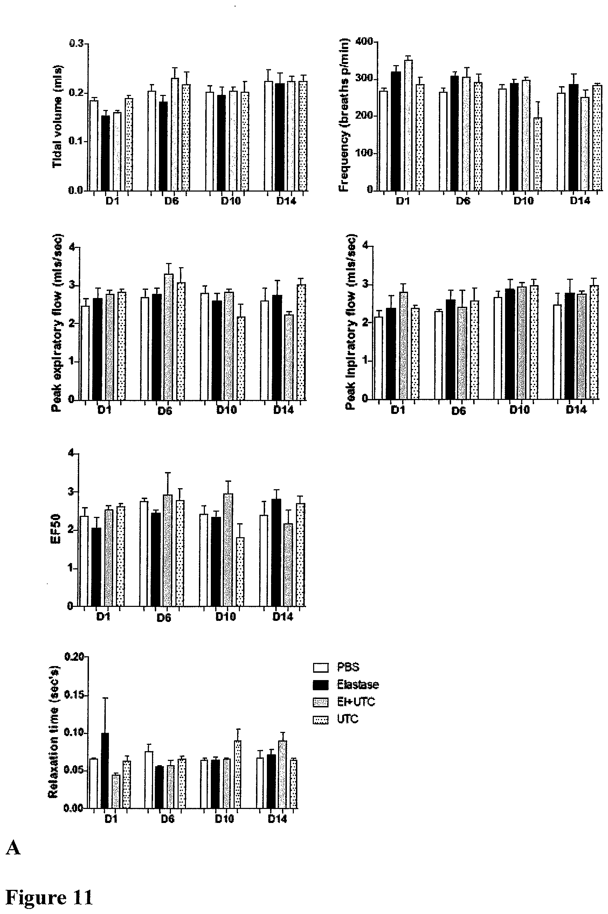

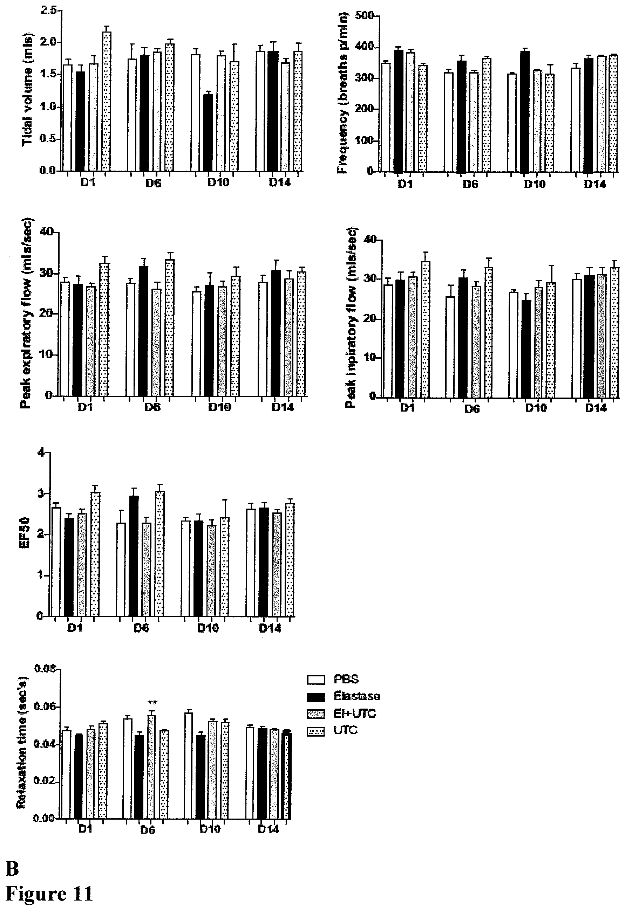

Figure 11 shows the effect of hUTC infusion and/or porcine pancreatic elastase (PPE) treatment on lung function at 1, 6, 10 and 14 days following hUTC administration in NOD/SCIDy mice (*see* Figure 11A) and BALB/c mice (*see* Figure 11B). Negative controls were sham-treated with saline and/or vehicle.

### DETAILED DESCRIPTION

This application is based on the discovery that human umbilical cord tissue-derived cells in an *in vivo* setting modulate (*e.g.* reduce) the production of pro-inflammatory mediators involved in the pathology of a lung disease, disorder, and/or injury such as *e.g.* COPD. In particular, Applicants have found that administration of human umbilical cord tissue-derived cells results in the reduction or even the inhibition of the production of pro-inflammatory mediators of respiratory disease (*e.g.* a lung disease, disorder, and/or injury) - such as *e.g.* TNF-α, RANTES, MCP-1 and IL-1β.

Accordingly, this invention relates to methods of using umbilical cord-tissue derived cells in the modulation (*e.g*. reduction) of production of pro-inflammatory mediators involved in the pathology of COPD in a patient suffering from COPD. In one embodiment, the invention provides for the reduction or even inhibition of pro-inflammatory mediators involved in the pathology of COPD and, therefore, causes a reduction of the disease symptoms. Optimally, the invention provides continual modulation (*e.g*. reduction) of production and/or inhibition of pro-inflammatory mediators involved in the symptoms and/or pathology of COPD particularly compared to conventional drug therapy, oxygen therapy, surgery, and pulmonary rehabilitation.

### I. Definitions

Various terms are used throughout the specification and claims. Such terms are to be given their ordinary meaning in the art unless otherwise indicated. Other specifically defined terms are to be construed in a manner consistent with the definition provided herein.

"Lung tissue" can include, but is not limited to, all lung tissue structures and associated tissues, including, but not limited to, veins, arteries, vessels, capillaries, and cells of the type that are part of, or associated with, such structures; lung and pleaural tissue; and vascular smooth muscle, pericyte, and vascular endothelial lineages and/or phenotypes.

As used herein, "respiratory or lung diseases, disorders and injuries" include, but are not limited to, obstructive lung diseases, restrictive lung diseases, respiratory tract infections (upper and lower), respiratory tumors, pleural cavity diseases, and pulmonary vascular diseases. The damage to lung tissue caused by these diseases, disorders and/or injuries can be characterized as lung damage. Furthermore, damaged lung tissue includes all lung tissue structures and associated tissues, including, veins, arteries, vessels, capillaries, and cells of the type that are part of, or associated with, such structures.

"Obstructive lung diseases" can include chronic obstructive pulmonary diseases (COPD) (*e.g.* chronic bronchitis and emphysema), cystic fibrosis, bronchiectasis, bronchiolitis, and allergic bronchopulmonary aspergillosis. COPD, for example, is caused by noxious particles or gases (most commonly from smoking), which trigger an abnormal inflammatory response in the lung. The inflammatory response in the larger airways is known as chronic bronchitis, which is diagnosed clinically when people regularly cough up sputum. In the alveoli, the inflammatory response causes destruction of the tissue of the lung, a process known as emphysema. It should be realized that these issues are those associated with COPD as it pertains to the instant invention. The etiology of COPD includes, but is not limited to, tobacco smoking, occupational exposures to workplace dusts (*e.g*., in coal mining, gold mining, the cotton textile industry and the chemical industry), air pollution, and genetics.

"Restrictive lung diseases," as used herein, are also known as interstitial lung diseases (ILDs). Many of these are idiopathic. Examples include idiopathic pulmonary fibrosis, idiopathic interstitial pneumonia (of which there are several types), sarcoidosis, eosinophilic pneumonia, lymphangioleiomyomatosis, pulmonary Langerhans' cell histiocytosis, and pulmonary alveolar proteinosis. ILDs affect the interstitium of the lung: alveolar epithelium, pulmonary capillary endothelium, basement membrane, perivascular and perilymphatic tissues. Most types of ILDs involve fibrosis.

Respiratory tumors include both malignant and benign tumors. Malignant tumors include, for example, small cell lung cancer, non-small cell lung cancer (adenocarcinoma, squamous cell carcinoma, and large cell undifferentiated carcinoma), lymphoma, as well as other cancers. Benign tumors are rare but can include pulmonary hamartoma and congenital malformations, for example.

As used herein, "acute lung injury" (ALI) is a diffuse heterogeneous lung injury characterized by hypoxemia, non-cardiogenic pulmonary edema, low lung compliance and widespread capillary leakage. ALI is caused by any stimulus of local or systemic inflammation. Acute respiratory distress syndrome (ARDS) is more severe than ALI. As used herein, ALI and ARDS can be characterized by abrupt onset of hypoxemia with diffuse pulmonary edema in response to either direct injury or indirect injury. As used herein, "direct injury includes," but is not limited to, lung injuries stemming from drowning episodes, pneumonia, inhaled toxic gases, and pulmonary contusions. As used herein, "indirect injury" can be from severe sepsis, transfusion, shock, and pancreatitis, for example. These injuries that lead to ALI and ARDS result in disruption of the alveolar-capillary interface, leakage of protein rich fluid into the interstitium and alveolar space, extensive release of cytokines, and migration of neutrophils.

The lung diseases, disorders, and injuries disclosed herein are known in the art. The characteristics of each, including associated complications, etiologies, and treatments, are known by those of skill in the art. This includes lung diseases, disorders and injuries not specifically discussed herein, as they would apply to obstructive and restrictive lung diseases, disorders and injuries.

The cells used in the present invention are generally referred to as postpartum cells or postpartum-derived cells (PPDCs). The cells are more specifically "umbilicus-derived cells" or "umbilical cord-derived cells" (UDC), or "umbilical cord tissue-derived cells" (UTC). In addition, the cells may be described as being stem or progenitor cells, the latter term being used in the broad sense. The term "derived" is used to indicate that the cells have been obtained from their biological source and grown or otherwise manipulated *in vitro* (*e.g.*, cultured in a growth medium to expand the population and/or to produce a cell line). The *in vitro* manipulations of umbilical stem cells and the unique features of the umbilicus-derived cells of the present invention are described in detail below.

Stem cells are undifferentiated cells defined by the ability of a single cell both to self-renew, and to differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation, and to contribute substantially to most, if not all, tissues following injection into blastocysts.

Stem cells are classified according to their developmental potential as: (1) totipotent; (2) pluripotent; (3) multipotent; (4) oligopotent; and (5) unipotent. Totipotent cells are able to give rise to all embryonic and extraembryonic cell types. Pluripotent cells are able to give rise to all embryonic cell types. Multipotent cells include those able to give rise to a subset of cell lineages, but all within a particular tissue, organ, or physiological system. For example, hematopoietic stem cells (HSC) can produce progeny that include HSC (self-renewal), blood cell-restricted oligopotent progenitors, and all cell types and elements (*e.g*., platelets) that are normal components of the blood. Cells that are oligopotent can give rise to a more restricted subset of cell lineages than multipotent stem cells. Cells that are unipotent are able to give rise to a single cell lineage (*e.g*., spermatogenic stem cells).

Stem cells are also categorized on the basis of the source from which they are obtained. An adult stem cell is generally a multipotent undifferentiated cell found in tissue comprising multiple differentiated cell types. The adult stem cell can renew itself. Under normal circumstances, it can also differentiate to yield the specialized cell types of the tissue from which it originated, and possibly other tissue types. An embryonic stem cell is a pluripotent cell from the inner cell mass of a blastocyst-stage embryo. A fetal stem cell is one that originates from fetal tissues or membranes. A postpartum stem cell is a multipotent or pluripotent cell that originates substantially from extraembryonic tissue available after birth, namely, the umbilical cord. These cells have been found to possess features characteristic of pluripotent stem cells, including rapid proliferation and the potential for differentiation into many cell lineages. Postpartum stem cells may be blood-derived (*e.g*., as are those obtained from umbilical cord blood) or non-blood-derived (*e.g*., as obtained from the non-blood tissues of the umbilical cord).

Various terms are used to describe cells in culture. "Cell culture" refers generally to cells taken from a living organism and grown under "condition in culture" or "cultured." A primary cell culture is a culture of cells, tissues, or organs taken directly from an organism(s) before the first subculture. Cells are expanded in culture when they are placed in a growth medium under conditions that facilitate cell growth and/or division, resulting in a larger population of the cells. When cells are expanded in culture, the rate of cell proliferation is sometimes measured by the amount of time needed for the cells to double in number. This is referred to as "doubling time."

The term "a cell line" generally refers to a population of cells formed by one or more subcultivations of a primary cell culture. Each round of subculturing is referred to as a passage. When cells are subcultured, they are referred to as having been "passaged." A specific population of cells, or a cell line, is sometimes referred to or characterized by the number of times it has been passaged. For example, a cultured cell population that has been passaged ten times may be referred to as a P10 culture. The primary culture, *i.e.,* the first culture following the isolation of cells from tissue, is designated P0. Following the first subculture, the cells are described as a secondary culture (PI or passage 1). After the second subculture, the cells become a tertiary culture (P2 or passage 2), and so on. It will be understood by those of skill in the art that there may be many population doublings during the period of passaging; therefore, the number of population doublings of a culture is greater than the passage number. The expansion of cells (*i.e*., the number of population doublings) during the period between passaging depends on many factors, including, but not limited to, the seeding density, substrate, medium, growth conditions, and time between passaging.

"Differentiation" is the process by which an unspecialized "uncommitted" or less specialized cell acquires the features of a specialized cell, such as a nerve cell or a muscle cell, for example. A "differentiated" cell is one that has taken on a more specialized "committed" position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. "De-differentiation" refers to the process by which a cell reverts to a less specialized (or committed) position within the lineage of a cell. As used herein, the "lineage" of a cell defines the heredity of the cell, *i.e.,* which cells it came from and what cells it can give rise to. The lineage of a cell places the cell within a hereditary scheme of development and differentiation.

In a broad sense, a "progenitor cell" is a cell that has the capacity to create progeny that are more differentiated than itself, and yet retains the capacity to replenish the pool of progenitors. By that definition, stem cells themselves are also progenitor cells, as are the more immediate precursors to terminally differentiated cells. When referring to the cells of the present disclosure, as described in greater detail below, this broad definition of progenitor cell may be used. In a narrower sense, a progenitor cell is often defined as a cell that is intermediate in the differentiation pathway, *i.e.,* it arises from a stem cell and is intermediate in the production of a mature cell type or subset of cell types. This type of progenitor cell is generally not able to self-renew. Accordingly, if this type of cell is referred to herein, it will be referred to as a "non-renewing progenitor cell" or as an "intermediate progenitor or precursor cell."

Several terms are used herein with respect to cell or tissue transplantation or cell replacement therapy. The terms "autologous transfer," "autologous transplantation," "autograft" and the like refer to treatment wherein the transplant donor is also the cell or transplant recipient. The terms "allogeneic transfer," "allogeneic transplantation," "allograft" and the like refer to transplantation wherein the transplant donor is of the same species as the transplant recipient, but is not the recipient. A cell transplant in which the donor cells have been histocompatibly matched with a recipient is sometimes referred to as a syngeneic transfer. The terms xenogeneic transfer, xenogeneic transplantation, xenograft and the like refer to transplantation wherein the transplant donor is of a different species than the transplant recipient.

The terms "pharmaceutically acceptable carrier" or "pharmaceutically acceptable medium" which may be used interchangeably with the terms "biologically compatible carrier" or "biologically compatible medium" generally refers to reagents, cells, compounds, materials, compositions, and/or dosage forms that are not only compatible with the cells and other agents to be administered therapeutically, but also are, suitable for use in contact with the tissues of human beings and animals without excessive toxicity, irritation, allergic response, or other complication commensurate with a reasonable benefit/risk ratio.

A "conditioned medium" is a medium in which a specific cell or population of cells has been cultured, and then removed. When cells are cultured in a medium, they may secrete cellular factors that can provide trophic support to other cells. Such trophic factors include, but are not limited to, hormones, cytokines, extracellular matrix (ECM), proteins, vesicles, antibodies, and granules. The medium containing the cellular factors is the conditioned medium.

Generally, a "trophic factor" is defined as a substance that promotes survival, growth, proliferation, and/or maturation of a cell, or stimulates increased activity of a cell.

As used herein, the term "growth medium" generally refers to a medium sufficient for the culturing of postpartum-derived cells. In particular, one presently preferred medium for the culturing of the cells of the invention in comprises Dulbecco's Modified Essential Media (DMEM). Particularly preferred is DMEM-low glucose (DMEM-LG) (Invitrogen, Carlsbad, Calif.). The DMEM-LG is preferably supplemented with serum, most preferably fetal bovine serum or human serum. Typically, 15% (v/v) fetal bovine serum (e.g. defined fetal bovine serum, Hyclone, Logan Utah) is added, along with antibiotics/antimycotics (preferably 100 Unit/milliliter penicillin, 100 milligrams/milliliter streptomycin, and 0.25 microgram/milliliter amphotericin B; (Invitrogen, Carlsbad, Calif.)), and 0.001% (v/v) 2-mercaptoethanol (Sigma, St. Louis Mo.). In some cases, different growth media are used, or different supplementations are provided, and these are normally indicated in the text as supplementations to growth medium. In certain chemically-defined media the cells may be grown without serum present at all. In such cases, the cells may require certain growth factors, which can be added to the medium to support and sustain the cells. Presently preferred factors to be added for growth in serum-free media include one or more of bFGF, EGF, IGF-I, and PDGF. In more preferred embodiments, two, three, or all four of the factors are added to serum free or chemically defined media. In other embodiments, LIF is added to serum-free medium to support or improve growth of the cells.

The term "standard growth conditions," as used herein refers to culturing of cells at 37 °C, in a standard atmosphere comprising 5% CO₂ and relative humidity maintained at about 100%. While the foregoing conditions are useful for culturing, it is to be understood that such conditions are capable of being varied by the skilled artisan who will appreciate the options available in the art for culturing cells.

As used herein, the term "about" when referring to a measurable value such as an amount, a temporal duration, and the like, is meant to encompass variations of ± 20% or ± 10%, more preferably ± 5%, even more preferably ± 1%, and still more preferably ± 0. 1% from the specified value, as such variations are appropriate to perform the disclosed methods.

The term "effective amount" refers to a concentration or amount of a compound, material, or composition, as described herein, that is effective to achieve a particular biological result. Such results include, but are not limited to, the regeneration, repair, or improvement of skeletal tissue, the improvement of blood flow, and/or the stimulation and/or support of angiogenesis in patients with lung damage from those diseases, disorders, and injuries. Such effective activity may be achieved, for example, by administering the cells and/or compositions to patients with lung damage as described herein. With respect to the administration of UTC to a patient *in vivo,* an effective amount may range from as few as several hundred or fewer to as many as several million or more. In specific embodiments, an effective amount may range from about 10³ to about 10¹¹, more specifically, at least about 10⁴ cells. It will be appreciated that the number of cells to be administered will vary depending on the specifics of pro-inflammatory mediator involved in the pathology of the lung disease, disorder or injury to be modulated, including, but not limited to, the size or total volume/surface area to be treated, and proximity of the site of administration to the location of the region to be treated, among other factors familiar to the medicinal biologist.

The terms "treat", "treating" or "treatment" refer to any success or indicia of success in the attenuation or amelioration of an injury, pathology or condition, including any objective or subjective parameter such as abatement, remission, diminishing of symptoms or making the injury, pathology, or condition more tolerable to the patient, slowing in the rate of degeneration or decline, making the final point of degeneration less debilitating, improving a subject's physical or mental well-being, or prolonging the length of survival. The treatment or amelioration of symptoms can be based on objective or subjective parameters; including the results of a physical examination, or neurological examination.

The terms "effective period", "effective period of time" or "effective conditions" refer generally to a period of time or other controllable conditions (*e.g*., temperature, humidity for *in vitro* methods), necessary or preferred for an agent or pharmaceutical composition to achieve its intended result.

The terms "individual," "patient" or "subject" are used interchangeably herein, and refer to animals, preferably mammals, and more preferably humans, who are treated with the pharmaceutical or therapeutic compositions or in accordance with the methods described herein.

The term "matrix" as used herein generally refers to biodegradable and/or bioresorbable materials that are administered with the cells to a patient. The matrix may act as a temporary scaffold until replaced by newly grown cells, such as, skeletal muscle, pericytes, vascular smooth muscle, or vascular endothelial tissue. In some embodiments, the matrix may provide for the sustained release of trophic factures or other agents used in conjunction with the cells and may provide a structure for developing tissue growth in the patient. In other embodiments, the matrix simply provides a temporary scaffold for the developing tissue. The matrix can be in particulate form (macroparticles greater than 10 microns in diameter or microparticles less than 10 microns in diameter), or it can be in the form of a structurally stable, three-dimensional implant (*e.g*., a scaffold). The matrix can be a slurry, hydrogel or a three-dimensional structure such as a cube, cylinder, tube, block, film, sheet or an appropriate anatomical form.

The term "scaffold" as used herein generally refers to a three dimensional porous structure that provides a template for cell growth. A scaffold is made of biodegradable and/or bioresorbable materials that degrade over time within the body. The length of time taken for the scaffold to degrade may depend upon the molecular weight of the materials. Thus, higher molecular weight material may result in polymer scaffolds, which retain their structural integrity for longer periods of time; while lower molecular weights result in both slower release and shorter scaffold lives. The scaffold may be made by any means known in the art. Examples of polymers which can be used to form the scaffold include natural and synthetic polymers.

The term "isolate" as used herein generally refers to a cell, which has been separated from its natural environment. This term includes gross physical separation from its natural environment, *e.g*., removal from the donor animal. In preferred embodiments, an isolated cell is not present in a tissue, *i.e.,* the cell is separated or dissociated from the neighboring cells with which it is normally in contact. Preferably, cells are administered as a cell suspension. As used herein, the phrase "cell suspension" includes cells which are in contact with a medium and which have been dissociated, *e.g*., by subjecting a piece of tissue to gentle trituration.

The term "modulate" as used herein generally means to adjust or regulate the production, activity, and/or amounts of the pro-inflammatory mediator involved in the pathology (*e.g.* the manifestation of the disease such as *e.g.* the changes in lung tissue) of a lung disease, disorder and/or injury. In one embodiment, the term modulate encompasses reducing the production of the pro-inflammatory mediator. In another embodiment, the term modulate encompasses inhibiting the production of the pro-inflammatory mediator.

In its various embodiments described herein, herein disclosed are methods and pharmaceutical compositions for modulating (*e.g*. reducing or inhibiting) the of production of pro-inflammatory mediators involved in the pathology of lung diseases, disorders, and/or injuries that utilize progenitor cells and cell populations derived from postpartum tissues, umbilicus tissue in particular. These methods and pharmaceutical compositions are designed to modulated (reduce and/or inhibit) the production of such pro-inflammatory mediators. In addition, they may optionally be designed to stimulate and support angiogenesis, to improve blood flow, to regenerate, repair, and improve lung tissue damaged by a lung disease, disorder, and/or injury, and/or to protect the lung tissue from such diseases, disorders, and/or injuries.

The cells, cell populations and preparations comprising cell lysates, conditioned media and the like, used in the pharmaceutical preparations and methods disclosed herein are described in detail in U.S. Patent Nos. 7,524,489, and 7,510,873, and U.S. Pub. App. No. 2005/0058634 and also herein.

### II. Isolation and growth of umbilical cord tissue-derived cells

According to the methods described herein, a mammalian umbilical cord is recovered upon or shortly after termination of either a full-term or pre-term pregnancy, for example, after expulsion or the after birth. The postpartum tissue may be transported from the birth site to a laboratory in a sterile container such as a flask, beaker, culture dish, or bag. The container may have a solution or medium, including but not limited to a salt solution, such as Dulbecco's Modified Eagle's Medium (DMEM) (also known as Dulbecco's Minimal Essential Medium) or phosphate buffered saline (PBS), or any solution used for the transportation of organs used for transplantation, such as University of Wisconsin solution or perfluorochemical solution. One or more antibiotic and/or antimycotic agents, such as but not limited to penicillin, streptomycin, amphotericin B, gentamicin, and nystatin, may be added to the medium or buffer. The postpartum tissue may be rinsed with an anticoagulant solution such as heparin-containing solution. It is preferable to keep the tissue at about 4 °C to about 10 °C prior to extraction of UTC. It is even more preferable that the tissue not be frozen prior to extraction of UTC.

Isolation of the UTC preferably occurs in an aseptic environment. The umbilical cord may be separated from the placenta by means known in the art. Blood and debris are preferably removed from the postpartum tissue prior to isolation of UTC. For example, the postpartum tissue may be washed with buffer solution, including, but not limited to, phosphate buffered saline. The wash buffer also may comprise one or more antimycotic and/or antibiotic agents, including, but not limited to, penicillin, streptomycin, amphotericin B, gentamicin, and nystatin.

Postpartum tissue comprising an umbilical cord, or a fragment or section thereof, is preferably disaggregated by mechanical force (mincing or shear forces). In a presently preferred embodiment, the isolation procedure also utilizes an enzymatic digestion process. Many enzymes are known in the art to be useful for the isolation of individual cells from complex tissue matrices to facilitate growth in culture. Digestion enzymes range from weakly digestive (*e.g.* deoxyribonucleases and the neutral protease, dispase) to strongly digestive (*e.g*. papain and trypsin), and are available commercially. A non-exhaustive list of such enzymes includes mucolytic enzyme activities, metalloproteases, neutral proteases, serine proteases (such as trypsin, chymotrypsin, or elastase), and deoxyribonucleases. Presently preferred are enzyme activities selected from metalloproteases, neutral proteases and mucolytic activities. For example, collagenases are known to be useful for isolating various cells from tissues. Deoxyribonucleases can digest single-stranded DNA and can minimize cell-clumping during isolation. Preferred methods involve enzymatic treatment with collagenase and dispase, or collagenase, dispase, and hyaluronidase. The skilled artisan will appreciate that many such enzyme treatments are known in the art for isolating cells from various tissue sources, and is well-equipped to assess new or additional enzymes or enzyme combinations for their utility in isolating the cells of the invention. Preferred enzyme treatments can be from about 0.5 to 2 hours long or longer. In some embodiments, the tissue is incubated at about 37 °C during the enzyme treatment of the dissociation step. In some embodiments, postpartum tissue is separated into sections comprising various aspects of the tissue, such as neonatal, neonatal/maternal, and maternal aspects of the placenta, for instance. The separated sections then are dissociated by mechanical and/or enzymatic dissociation according to the methods described herein. Cells of neonatal or maternal lineage may be identified by any means known in the art, for example, by karyotype analysis or *in situ* hybridization for a Y chromosome.

The isolated cells may be used to initiate, or seed, cell cultures. Isolated cells are transferred to sterile tissue culture vessels either uncoated or coated with extracellular matrix or ligands such as laminin, collagen (native, denatured or crosslinked), gelatin, fibronectin, and other extracellular matrix proteins. The cells are cultured in any culture medium capable of sustaining growth of the cell such as, but not limited to, DMEM (high or low glucose), advanced DMEM, DMEM/MCDB 201, Eagle's basal medium, Ham's F10 medium (F10), Ham's F-12 medium (F12), Iscove's modified Dulbecco's medium, Mesenchymal Stem Cell Growth Medium (MSCGM), DMEM/F12, RPMI 1640, and serum/media free medium sold under the trade name CELL-GRO-FREE (Mediatch, Inc., Herndon, Va.). The culture medium may be supplemented with one or more components including, for example, fetal bovine serum (FBS), preferably about 2-15% (v/v); equine serum (ES); human serum (HS); beta-mercaptoethanol (BME or 2-ME), preferably about 0.001% (v/v); one or more growth factors, for example, platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF) and erythropoietin (EPO); amino acids, including L-valine; and one or more antibiotic and/or antimycotic agents to control microbial contamination, such as e.g., penicillin G, streptomycin sulfate, amphotericin B, gentamicin, and nystatin, either alone or in combination. The culture medium preferably comprises growth medium (e.g., DMF-M-low glucose, serum, BME and an antibiotic agent).

The cells are seeded in culture vessels at a density to allow cell growth. In a one embodiment, the cells are cultured at about 0 percent to about 5 percent by volume CO₂ in air. In some other embodiments, the cells are cultured at about 2 percent to about 25 percent O₂ in air, preferably about 5 percent to about 20 percent O₂ in air. The cells preferably are cultured at a temperature of about 25 °C to about 40 °C and more preferably are cultured at 37 °C. The cells are preferably cultured in an incubator. The medium in the culture vessel can be static or agitated, for example, using a bioreactor. The UTC are preferably grown under low oxidative stress (e.g., with addition of glutathione, Vitamin C, Catalase, Vitamin E, N-Acetylcysteine). "Low oxidative stress," as used herein, refers to conditions of no or minimal free radical damage to the cultured cells.

Methods for the selection of the most appropriate culture medium, medium preparation, and cell culture techniques are well known in the art and are described in a variety of sources, including Doyle et al., (eds.), 1995, Cell & Tissue Culture: Laboratory Procedures, John Wiley & Sons, Chichester; and Ho and Wang (eds.), 1991, Animal Cell Bioreactors, Butterworth-Heinemann, Bost*on.*

In some embodiments, the UTC are passaged, or removed to a separate culture vessel containing fresh medium of the same or a different type as that used initially, where the population of cells can be mitotically expanded. The cells of the invention may be used at any point between passage 0 and senescence. The cells preferably are passaged between about 3 and about 25 times, more preferably are passaged about 4 to about 12 times, and preferably are passaged 10 or 11 times. Cloning and/or subcloning may be performed to confirm that a clonal population of cells has been isolated.

The different cell types present in postpartum tissue can be fractionated into subpopulations from which the UTC can be isolated. Fractionation or selection may be accomplished using standard techniques for cell separation including, but not limited to, enzymatic treatment to dissociate postpartum tissue into its component cells, followed by cloning and selection of specific cell types, including, but not limited to, selection based on morphological and/or biochemical markers; selective growth of desired cells (positive selection); selective destruction of unwanted cells (negative selection); separation based upon differential cell agglutinability in the mixed population as, for example, with soybean agglutinin; freeze-thaw procedures; differential adherence properties of the cells in the mixed population; filtration; conventional and zonal centrifugation; centrifugal elutriation (counter-streaming centrifugation); unit gravity separation; countercurrent distribution; electrophoresis; and fluorescence activated cell sorting (FACS).

The culture medium is changed as necessary, *e.g*., by carefully aspirating the medium from the dish with a pipette, and replenishing with fresh medium. Incubation is continued until a sufficient number or density of cells accumulates in the dish. Thereafter any original explanted tissue sections that exist may be removed and the remaining cells separated from the dish trypsinization using standard techniques or using a cell scraper. After trypsinization, the cells are collected, removed to fresh medium and incubated as above. In some embodiments, the medium is changed at least once at approximately 24 hours post-trypsinization to remove any floating cells. The cells remaining in culture are considered to be UTC.

The UTC may be cryopreserved. Accordingly, in a preferred embodiment described in greater detail below, the UTC for autologous transfer (for either the mother or child) may be derived from appropriate postpartum tissues following the birth of a child, then cryopreserved so as to be available in the event they are later needed for transplantation.

### III. Characteristics of umbilical cord tissue derived cells

Examples of UTC derived from umbilicus tissue, which are suitable for use in the claimed methods, uses, pharmaceutical compositions and kits, were deposited with the American Type Culture Collection (ATCC) (10801 University Blvd., Manassas, VA 20110) on June 10, 2004, and assigned ATCC Accession Numbers as follows: (1) strain designation UMB 022803 (P7) was assigned Accession No. PTA-6067; and (2) strain designation UMB 022803 (P17) was assigned Accession No. PTA-6068.

The UTC may be characterized, for example, by growth characteristics (*e.g*., population doubling capability, doubling time, passages to senescence), karyotype analysis (*e.g*., normal karyotype; maternal or neonatal lineage), flow cytometry (*e.g*., FACS analysis), immunohistochemistry and/or immunocytochemistry (*e.g*., for detection of epitopes), gene expression profiling (*e.g*., gene chip arrays; polymerase chain reaction (*e.g*., reverse transcriptase PCR, real time PCR, and conventional PCR)), protein arrays, protein secretion (*e.g*., by plasma clotting assay or analysis of PDC-conditioned medium, for example, by Enzyme Linked ImmunoSorbent Assay (ELISA)), mixed lymphocyte reaction (*e.g*., as measure of stimulation of PBMCs), and/or other methods known in the art.

As such, UTC are defined by a combination of one or more of the following characteristics: (1) growth features; (2) production of certain proteins; (3) gene expression, which relative to a human cell that is a fibroblast, a mesenchymal stem cell, or an iliac crest bone marrow cell, is increased for certain genes; (4) characterized by gene expression, which relative to a human cell that is a fibroblast, a mesenchymal stem cell, or an iliac crest bone marrow cell, is reduced for certain genes; (5) secretion or lack of secretion of trophic factors; (6) lack of expression hTERT or telomerase.

In one embodiment, the UTC may be characterized by possessing one or more of the following growth features: they require L-valine for growth in culture; they are capable of growth in atmospheres containing oxygen from about 5% to about 20%; they have the potential for at least about 40 doublings in culture before reaching senescence; and they attach and expand on tissue culture vessels that are uncoated, or that are coated with gelatin, laminin, collagen, polyornithine, vitronectin or fibronectin. In certain embodiments, the UTC may also possess a normal karyotype, which is maintained as the cells are passaged. Methods for karyotyping are available and known to those of skill in the art.

In other embodiments, the UTC may be characterized by production of certain proteins, including (1) production of at least one of tissue factor, vimentin, and alpha-smooth muscle actin; and (2) production of at least one of CD10, CD13, CD44, CD73, CD90, PDGFr-alpha, PD-L2 and HLA-A,B,C cell surface markers, as detected by flow cytometry. In other embodiments, the UTC may be characterized by lack of production of at least one of CD31, CD34, CD45, CD80, CD86, CD117, CD141, CD178, B7-H2, HLA-G, and HLA-DR, DP, DQ cell surface markers, as detected by flow cytometry. In one embodiment, the cells are characterized by lack of production of CD45 and CD117. In some embodiments, the cells produce at least two of tissue factor, vimentin, and alpha-smooth muscle actin. In other embodiments, the cells produce all three of the proteins tissue factor, vimentin, and alpha-smooth muscle actin.

In other embodiments, the UTC may be characterized by gene expression, which relative to a human cell that is a fibroblast, a mesenchymal stem cell, or an iliac crest bone marrow cell, is increased for a gene encoding at least one of interleukin 8; reticulon 1; chemokine (C-X-C motif) ligand 1 (melonoma growth stimulating activity, alpha); chemokine (C-X-C motif) ligand 6 (granulocyte chemotactic protein 2); chemokine (C-X-C motif) ligand 3; tumor necrosis factor, alpha-induced protein 3. In one embodiment, the UTC may be characterized by gene expression, which relative to a human cell that is a fibroblast, a mesenchymal stem cell, or an iliac crest bone marrow cell, is increased for a gene encoding at least one of interleukin 8, reticulon 1, and chemokine (C-X-C motif) ligand 3; tumor necrosis factor.

In yet another embodiment, the UTC may also be characterized by gene expression, which relative to a human cell that is a fibroblast, a mesenchymal stem cell, or an iliac crest bone marrow cell, is reduced for a gene encoding at least one of: short stature homeobox 2; heat shock 27 kDa protein 2; chemokine (C-X-C motif) ligand 12 (stromal cell-derived factor 1); elastin (supravalvular aortic stenosis, Williams-Beuren syndrome); *Homo sapiens* mRNA; cDNA DKFZp586M2022 (from clone DKFZp586M2022); mesenchyme homeo box 2 (growth arrest-specific homeo box); sine oculis homeobox homolog 1 (*Drosophila*); crystallin, alpha B; disheveled associated activator of morphogenesis 2; DKFZP586B2420 protein; similar to neuralin 1; tetranectin (plasminogen binding protein); src homology three (SH3) and cysteine rich domain; cholesterol 25-hydroxylase; runt-related transcription factor 3; interleukin 11 receptor, alpha; procollagen C-endopeptidase enhancer; frizzled homolog 7 (*Drosophila*); hypothetical gene BC008967; collagen, type VIII, alpha 1; tenascin C (hexabrachion); iroquois homeobox protein 5; hephaestin; integrin, beta 8; synaptic vesicle glycoprotein 2; neuroblastoma, suppression of tumorigenicity 1; insulin-like growth factor binding protein 2, 36 kDa; *Homo sapiens* cDNA FLJ12280 fis, clone MAMMA1001744; cytokine receptor-like factor 1; potassium intermediate/small conductance calcium-activated channel, subfamily N, member 4; integrin, beta 7; transcriptional co-activator with PDZ-binding motif (TAZ); sine oculis homeobox homolog 2 (*Drosophila*); KIAA1034 protein; vesicle-associated membrane protein 5 (myobrevin); EGF-containing fibulin-like extracellular matrix protein 1; early growth response 3; distal-less homeo box 5; hypothetical protein FLJ20373; aldo-keto reductase family 1, member C3 (3-alpha hydroxysteroid dehydrogenase, type II); biglycan; transcriptional co-activator with PDZ-binding motif (TAZ); fibronectin 1; proenkephalin; integrin, beta-like 1 (with EGF-like repeat domains); *Homo sapiens* mRNA full length insert cDNA clone EUROIMAGE 1968422; EphA3; KIAA0367 protein; natriuretic peptide receptor C/guanylate cyclase C (atrionatriuretic peptide receptor C); hypothetical protein FLJ14054; *Homo sapiens* mRNA; cDNA DKFZp564B222 (from clone DKFZp564B222); BCL2/adenovirus E1B 19 kDa interacting protein 3-like; AE binding protein 1; and cytochrome c oxidase subunit VIIa polypeptide 1 (muscle).

In other embodiments, the UTC may be characterized by secretion of at least one of MCP-1, IL-6, IL-8, GCP-2, HGF, KGF, FGF, HB-EGF, BDNF, TPO, MIP1β, 1309, MDC, RANTES, and TIMP, as detected when the cells are cultured *in vitro* in culture. In some embodiments, the UTC may be characterized by lack of secretion of at least one of TGF-beta2, ANG2, PDGFbb, MIP1α and VEGF, as detected by *e.g.* ELISA, when the cells are cultured *in vitro* in culture.

In preferred embodiments, the cells do not express hTERT or telomerase. Accordingly, one embodiment of the disclosure is umbilical-derived cells that do not express hTERT or telomerase (hTert) and that have one or more of the characteristics disclosed herein.

In preferred embodiments, the cells comprise two or more of the above-listed characteristics. More preferred are cells comprising, three, four, five or more of the characteristics. Still more preferred are UTC comprising six, seven, eight, nine, ten, eleven, or more of the characteristics. Still more preferred are cells comprising all of above characteristics.

In one embodiment, the UTC are derived from umbilical cord tissue substantially free of blood, are capable of self-renewal and expansion in culture, require L-valine for growth, can grow in at least about 5% oxygen, and comprise at least one of the following characteristics: (1) the potential for at least about 40 doublings in culture; (2) the ability to attach and expand on an uncoated tissue culture vessel or one coated with gelatin, laminin, collagen, polyornithine, vitronectin, or fibronectin; (3) production of vimentin and alpha-smooth muscle actin; (4) production of CD10, CD13, CD44, CD73, and CD90; and (5) expression of a gene, which relative to a human cell that is a fibroblast, a mesenchymal stem cell, or an iliac crest bone marrow cell, is increased for a gene encoding interleukin 8 and reticulon 1. In some embodiments, such UTC does not produce CD45 and CD 117.

In one embodiment, the UTC are isolated from human umbilical cord tissue substantially free of blood, capable of self-renewal and expansion in culture, lack the production of CD117 and do not express hTERT or telomerase. The UTC optionally (i) express oxidized low density lipoprotein receptor 1, reticulon, chemokine receptor ligand 3, and/or granulocyte chemotactic protein; and/or (ii) do not express CD31, CD34 or CD45; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90.

In another embodiment, the UTC are isolated from human umbilical cord tissue substantially free of blood, capable of self-renewal and expansion in culture, lack the production of CD117 or CD45, and do not express hTERT or telomerase. These UTC optionally express oxidized low density lipoprotein receptor 1, reticulon, chemokine receptor ligand 3, and/or granulocyte chemotactic protein; and/or do not express CD31 or CD34; and/or express, relative to a human fibroblast, mesenchymal stem cell, or iliac crest bone marrow cell, increased levels of interleukin 8 or reticulon 1; and/or have the potential to differentiate into cells of at least a lung tissue; and/or express CD10, CD13, CD44, CD73, and CD90.

In another embodiment, the UTC are isolated from human umbilical cord tissue substantially free of blood, capable of self-renewal and expansion in culture, lack the production of CD117 and CD45, and do not express hTERT or telomerase. These UTC optionally (i) express oxidized low density lipoprotein receptor 1, reticulon, chemokine receptor ligand 3, and/or granulocyte chemotactic protein; and/or (ii) do not express CD31 or CD34; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90.

In an alternate embodiment, the UTC are isolated from human umbilical cord tissue substantially free of blood, capable of self-renewal and expansion in culture, lack the production of CD117 and CD45, and do not express hTERT and telomerase. These UTC optionally (i) express oxidized low density lipoprotein receptor 1, reticulon, chemokine receptor ligand 3, and/or granulocyte chemotactic protein; and/or (ii) do not express CD31 or CD34; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90.

In another embodiment, the cells are isolated from human umbilical cord tissue substantially free of blood, capable of self-renewal and expansion in culture, lack the production of CD117, CD34, CD31, and do not express hTERT or telomerase. These UTC optionally (i) express oxidized low density lipoprotein receptor 1, reticulon, chemokine receptor ligand 3, and/or granulocyte chemotactic protein; and/or (ii) do not express CD45; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90.

In yet another embodiment, the UTC are isolated from human umbilical cord tissue substantially free of blood, capable of self-renewal and expansion in culture, lack the production of CD117, CD45, CD34, CD31, and do not express hTERT or telomerase. The UTC optionally (i) express oxidized low density lipoprotein receptor 1, reticulon, chemokine receptor ligand 3, and/or granulocyte chemotactic protein; and/or (ii) express, relative to a human fibroblast, mesenchymal stem cell, or iliac crest bone marrow cell, increased levels of interleukin 8 or reticulon 1; and/or (iii) have the potential to differentiate into cells of at least a lung tissue; and/or (iv) express CD10, CD13, CD44, CD73, and CD90.

In an alternate embodiment, the UTC are isolated from human umbilical cord tissue substantially free of blood, capable of self-renewal and expansion in culture, lack the production of CD117, CD45, CD34, CD31, and do not express hTERT and telomerase. The UTC optionally (i) express oxidized low density lipoprotein receptor 1, reticulon, chemokine receptor ligand 3, and/or granulocyte chemotactic protein; and/or (ii) express, relative to a human fibroblast, mesenchymal stem cell, or iliac crest bone marrow cell, increased levels of interleukin 8 or reticulon 1; and/or (iii) have the potential to differentiate into cells of at least a lung tissue; and/or (iv) express CD10, CD13, CD44, CD73, and CD90.

In yet another embodiment, the UTC are isolated from human umbilical cord tissue substantially free of blood, capable of self-renewal and expansion in culture and have the following characteristics: lack of production of CD117 and CD45; lack expression of hTERT or telomerase; express oxidized low density lipoprotein receptor 1, reticulon, chemokine receptor ligand 3; express, relative to a human fibroblast, mesenchymal stem cell, or iliac crest bone marrow cell, increased levels of interleukin 8 or reticulon 1; have the potential to differentiate into cells of at least a lung tissue; and express CD10, CD13, CD44, CD73, and CD90. In another embodiment, the UTC are isolated from human umbilical cord tissue substantially free of blood, capable of self-renewal and expansion in culture and have the following characteristics: lack of production of CD117 and CD45; lack expression of hTERT or telomerase; express, relative to a human fibroblast, mesenchymal stem cell, or iliac crest bone marrow cell, increased levels of interleukin 8 or reticulon 1; have the potential to differentiate into cells of at least a lung tissue; and express CD10, CD13, CD44, CD73, and CD90. In yet another embodiment, the UTC are isolated from human umbilical cord tissue substantially free of blood, capable of self-renewal and expansion in culture and have the following characteristics: lack of production of CD117 and CD45; lack expression of hTERT or telomerase; express, relative to a human fibroblast, mesenchymal stem cell, or iliac crest bone marrow cell, increased levels of interleukin 8 or reticulon 1; and express CD10, CD13, CD44, CD73, and CD90.

In yet another embodiment, the UTC are isolated from human umbilical cord tissue substantially free of blood, capable of self-renewal and expansion in culture, and have the following characteristics: potential for at least 40 doublings in culture; production of CD10, CD13, CD44, CD73, CD90, PDGFr-alpha, PD-L2 and HLA-A,B,C; lack of production of CD31, CD34, CD45, CD80, CD86, CD117, CD141, CD178, B7-H2, HLA-G, and HLA-DR,DP,DQ, as detected by flow cytometry; increased expression of interleukin 8, reticulon 1, and chemokine (C-X-C motif) ligand 3, relative to a human cell that is a fibroblast, a mesenchymal stem cell, or an iliac crest bone marrow cell; and do not express hTERT or telomerase.

The UTC described above can be used in methods of modulating (*e.g.*, reducing and/or inhibiting) the production of pro-inflammatory mediators of a lung diseases in a patient suffering from the lung disease. They can be also used in pharmaceutical compositions for modulating (reducing and/or inhibiting) the production of pro-inflammatory mediators of lung diseases in a patient suffering from the lung disease, for example, wherein such compositions comprise the cells having these characteristics and a pharmaceutically acceptable carrier, and can be used in kits for making, using, and practicing such methods and pharmaceutical compositions as described and exemplified herein. In addition, the UTC as described above can be used to make preparations such as cell extracts and subcellular fractions that can be used for making, using, and practicing such methods and pharmaceutical compositions as described and exemplified herein.

Certain cells having the potential to differentiate along lines leading to various phenotypes are unstable and thus can spontaneously differentiate. Presently preferred for use with the invention are UTC that do not spontaneously differentiate, for example, along myoblast, skeletal muscle, vascular smooth muscle, pericyte, hemangiogenic, angiogenic, vasculogenic, or vascular endothelial lines. Preferred cells, when grown in growth medium, are substantially stable with respect to the cell markers produced on their surface, and with respect to the expression pattern of various genes, for example as determined using a medical diagnostic test sold under the trade name GENECHIP (Affymetrix, Inc., Santa Clara, Calif.). The cells remain substantially constant, for example in their surface marker characteristics over passaging and through multiple population doublings.

### IV. Populations of umbilical cord tissue-derived cells

Herein also disclosed is use of populations of UTC described above in reducing the production (or even in inhibiting the production) of pro-inflammatory mediators in a patient having a lung disease. In some embodiments, the cell population may be heterogeneous. A heterogeneous cell population may comprise at least about 5%, 10%, 20%, 30%, 40%, 50%, 60%, 70%, 80%, 90%, or 95% UTC. The heterogeneous cell populations may further comprise stem cells or other progenitor cells, such as myoblasts or other muscle progenitor cells, hemangioblasts, or blood vessel precursor cells; or it may further comprise fully differentiated skeletal muscle cells, smooth muscle cells, pericytes, or blood vessel endothelial cells. In some embodiments, the population is substantially homogeneous, *i.e.,* comprises substantially only the UTC (preferably at least about 96%, 97%, 98%, 99% or more UTC). The homogeneous cell populations of the disclosure are comprised of umbilicus-derived cells. Homogeneous populations of umbilicus-derived cells are preferably free of cells of maternal lineage. Homogeneity of a cell population may be achieved by any method known in the art, for example, by cell sorting (*e.g*., flow cytometry) or by clonal expansion in accordance with known methods. Homogeneous UTC populations may comprise a clonal cell line of postpartum-derived cells. Such populations are particularly useful when a cell clone with highly desirable functionality has been isolated.

In one embodiment, a substantially homogeneous population of UTC is used. In one embodiment, this substantially homogenous population comprises UTC, which are isolated from human umbilical cord tissue substantially free of blood, are capable of self-renewal and expansion in culture, lack the production of CD117 and do not express hTERT or telomerase. The UTC optionally (i) express oxidized low density lipoprotein receptor 1, reticulon, chemokine receptor ligand 3, and/or granulocyte chemotactic protein; and/or (ii) do not express CD31, CD34 or CD45; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90. In another embodiment, the population comprises UTC, which are isolated from human umbilical cord tissue substantially free of blood, are capable of self-renewal and expansion in culture, lack the production of CD117 and CD45 and do not express hTERT or telomerase. The UTC optionally (i) express oxidized low density lipoprotein receptor 1, reticulon, chemokine receptor ligand 3, and/or granulocyte chemotactic protein; and/or (ii) do not express CD31 or CD34; and/or (iii) express, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) express CD10, CD13, CD44, CD73, and CD90. In another embodiment, the population comprises UTC, which are isolated from human umbilical cord tissue substantially free of blood, are capable of self-renewal and expansion in culture, lack the production of CD117, CD34 and CD31, and do not express hTERT or telomerase. In yet another embodiment, the population comprises UTC, which are isolated from human umbilical cord tissue substantially free of blood, are capable of self-renewal and expansion in culture and lack the production of CD117, CD45, CD34, CD31 and/or telomerase. In an alternate embodiment, the substantially homogeneous population of umbilicus-derived cells is isolated from human umbilical cord tissue substantially free of blood, is capable of self-renewal and expansion in culture, and has the following characteristics: potential for at least 40 doublings in culture; production of CD10, CD13, CD44, CD73, CD90, PDGFr-alpha, PD-L2 and HLA-A,B,C; lack of production of CD31, CD34, CD45, CD80, CD86, CD117, CD141, CD178, B7-H2, HLA-G, and HLA-DR,DP,DQ; increased expression of interleukin 8, reticulon 1, and chemokine (C-X-C motif) ligand 3, relative to a human cell that is a fibroblast, a mesenchymal stem cell, or an iliac crest bone marrow cell; and does not express hTERT or telomerase.

In another embodiment, a homogeneous population of UTC is used. In one embodiment, this homogenous population comprises UTC isolated from human umbilical cord tissue substantially free of blood, capable of self-renewal and expansion in culture, lack the production of CD117 and do not express hTERT or telomerase. The population optionally (i) expresses oxidized low density lipoprotein receptor 1, reticulon, chemokine receptor ligand 3, and/or granulocyte chemotactic protein; and/or (ii) does not express CD31, CD34 or CD45; and/or (iii) expresses, relative to a human fibroblast, mesenchymal stem cell, or iliac crest bone marrow cell, increased levels of interleukin 8 or reticulon 1; and/or (iv) has the potential to differentiate into cells of at least a lung tissue; and/or (v) expresses CD10, CD13, CD44, CD73, and CD90. In another embodiment, the homogenous UTC population is isolated from human umbilical cord tissue substantially free of blood, capable of self-renewal and expansion in culture, lacks the production of CD117 and CD45, and does not express hTERT or telomerase. The population optionally (i) expresses oxidized low density lipoprotein receptor 1, reticulon, chemokine receptor ligand 3, and/or granulocyte chemotactic protein; and/or (ii) does not express CD31 or CD34; and/or (iii) expresses, relative to a human fibroblast, mesenchymal stem cell, or iliac crest bone marrow cell, increased levels of interleukin 8 or reticulon 1; and/or (iv) have the potential to differentiate into cells of at least a lung tissue; and/or (v) expresses CD10, CD13, CD44, CD73, and CD90. In an alternate embodiment, the homogenous UTC population is isolated from human umbilical cord tissue substantially free of blood, capable of self-renewal and expansion in culture and lacks the production of CD117, CD34, CD31, and/or telomerase. In yet another embodiment, the homogeneous population is isolated from human umbilical cord tissue substantially free of blood, capable of self-renewal and expansion in culture, lacks the production of CD117, CD45, CD34, and CD31, and does not express hTERT or telomerase. In an alternate embodiment, the substantially homogeneous population of umbilicus-derived cells is isolated from human umbilical cord tissue substantially free of blood, is capable of self-renewal and expansion in culture, and has the following characteristics: potential for at least 40 doublings in culture; production of CD10, CD13, CD44, CD73, CD90, PDGFr-alpha, PD-L2 and HLA-A,B,C; lack of production of CD31, CD34, CD45, CD80, CD86, CD117, CD141, CD178, B7-H2, HLA-G, and HLA-DR,DP,DQ; increased expression of interleukin 8, reticulon 1, and chemokine (C-X-C motif) ligand 3, relative to a human cell that is a fibroblast, a mesenchymal stem cell, or an iliac crest bone marrow cell; and does not express hTERT or telomerase.

Also provided herein is the use of populations of cells incubated in the presence of one or more factors, or under conditions, that stimulate stem cell differentiation along a vascular smooth muscle, vascular endothelial, or pericyte pathway. Such factors are known in the art and the skilled artisan will appreciate that determination of suitable conditions for differentiation can be accomplished with routine experimentation. Optimization of such conditions can be accomplished by statistical experimental design and analysis, for example, response surface methodology allows simultaneous optimization of multiple variables in a biological culture. Presently preferred factors include, but are not limited to, growth or trophic factors, chemokines, cytokines, cellular products, demethylating agents, and other stimuli which are now known or later determined to stimulate differentiation, for example, of stem cells along angiogenic, hemangiogenic, vasculogenic, skeletal muscle, vascular smooth muscle, pericyte, or vascular endothelial pathways or lineages.

### V. Genetic modifications of umbilical cord tissue-derived cells

The UTC may also be genetically modified to produce therapeutically useful gene products, such as *e.g.* to produce angiogenic agents to facilitate or support additional blood vessel formation or growth, or to produce factors to recruit endothelial progenitor cells to the area of lung damage. Endothelial progenitor cells facilitate vasculogenesis and blood flow, particularly following an ischemic event (Urbich C and Dimmeler S., Circ. Res., 2004; 95:343-53). Factors that play a role in endothelial cell recruitment include, but are not limited to VEGF, stromal derived factor-1 (SDF-1), erythropoietin (EPO), G-CSF, statins, strogen, PPAR-γ, CXCR4, FGF, and HGF. Genetic modification may be accomplished using any of a variety of vectors including, but not limited to, integrating viral vectors, *e.g*., retrovirus vector or adeno-associated viral vectors; non-integrating replicating vectors, *e.g*., papilloma virus vectors, SV40 vectors, adenoviral vectors, or replication-defective viral vectors. Other methods of introducing DNA into cells include the use of liposomes, electroporation, a particle gun, or by direct DNA injection.

Hosts cells are preferably transformed or transfected with DNA controlled by or in operative association with, one or more appropriate expression control elements such as promoter or enhancer sequences, transcription terminators, polyadenylation sites, among others, and a selectable marker. Any promoter may be used to drive the expression of the inserted gene. For example, viral promoters include, but are not limited to, the CMV promoter/enhancer, SV40, papillomavirus, Epstein-Barr virus or elastin gene promoter. In some embodiments, the control elements used to control expression of the gene of interest can allow for the regulated expression of the gene so that the product is synthesized only when needed *in vivo.* If transient expression is desired, constitutive promoters are preferably used in a non-integrating and/or replication-defective vector. Alternatively, inducible promoters could be used to drive the expression of the inserted gene when necessary. Inducible promoters include, but are not limited to, those associated with metallothionein and heat shock proteins.

Following the introduction of the foreign DNA, engineered cells may be allowed to grow in enriched media and then switched to selective media. The selectable marker in the foreign DNA confers resistance to the selection and allows cells to stably integrate the foreign DNA as, for example, on a plasmid, into their chromosomes and grow to form foci which, in turn, can be cloned and expanded into cell lines. This method can be advantageously used to engineer cell lines that express the gene product.

The cells of the invention may be genetically engineered to "knock out" or "knock down" expression of factors that promote inflammation or rejection at the implant site. Negative modulatory techniques for the reduction of target gene expression levels or target gene product activity levels are discussed below. "Negative modulation," as used herein, refers to a reduction in the level and/or activity of target gene product relative to the level and/or activity of the target gene product in the absence of the modulatory treatment. The expression of a gene native to a skeletal muscle cell, vascular smooth muscle cell, pericyte, vascular endothelial cell, or progenitor cells thereof can be reduced or knocked out using a number of techniques including, for example, inhibition of expression by inactivating the gene using the homologous recombination technique. Typically, an exon encoding an important region of the protein (or an exon 5' to that region) is interrupted by a positive selectable marker, *e.g*., neo, preventing the production of normal mRNA from the target gene and resulting in inactivation of the gene. A gene may also be inactivated by creating a deletion in part of a gene, or by deleting the entire gene. By using a construct with two regions of homology to the target gene that are far apart in the genome, the sequences intervening the two regions can be deleted (Mombaerts et al., Proc. Nat. Acad. Sci. U.S.A., 1991; 88:3084-87). Antisense, DNAzymes, ribozymes, small interfering RNA (siRNA) and other such molecules that inhibit expression of the target gene can also be used to reduce the level of target gene activity. For example, antisense RNA molecules that inhibit the expression of major histocompatibility gene complexes (HLA) have been shown to be most versatile with respect to immune responses. Still further, triple helix molecules can be utilized in reducing the level of target gene activity.

### VI. Cell lysates and cell soluble fractions prepared from umbilical cord-tissue derived cells.

Herein also disclosed are cell lysates and cell soluble fractions prepared from a UTC, or heterogeneous or homogeneous cell populations comprising a UTC, as well as a UTC or populations thereof that have been genetically modified or that have been stimulated to differentiate along a skeletal muscle, vascular smooth muscle, pericyte, or vascular endothelium pathway. Such lysates and fractions thereof have many utilities. Use of the UTC lysate soluble fraction (*i.e*., substantially free of membranes) *in vivo,* for example, allows the beneficial intracellular milieu to be used allogeneically in a patient without introducing an appreciable amount of the cell surface proteins most likely to trigger rejection, or other adverse immunological responses. Methods of lysing cells are well-known in the art and include various means of mechanical disruption, enzymatic disruption, or chemical disruption, or combinations thereof. Such cell lysates may be prepared from cells directly in their growth medium and thus contain secreted growth factors and the like, or they may be prepared from cells washed free of medium in, for example, PBS or other solution. Washed cells may be resuspended at concentrations greater than the original population density.

In one embodiment, whole cell lysates are prepared, *e.g.*, by disrupting cells without subsequent separation of cell fractions. In another embodiment, a cell membrane fraction is separated from a soluble fraction of the cells by routine methods known in the art, e.g., centrifugation, filtration, or similar methods.

Cell lysates or cell soluble fractions prepared from populations of postpartum-derived cells may be used as is, further concentrated, by *e.g*. ultrafiltration or lyophilization, or even dried, partially purified, combined with pharmaceutically-acceptable carriers or diluents as are known in the art, or combined with other compounds such as biologicals, *e.g*. pharmaceutically useful protein compositions. Cell lysates or fractions thereof may be used *in vitro* or *in vivo,* alone or *e.g*., with autologous or syngeneic live cells. The lysates, if introduced *in vivo,* may be introduced locally at a site of treatment, or remotely to provide, *e.g.* needed cellular growth factors to a patient. Use of cell lysates *in vivo* is known in the art, and one of skill in the art will know the necessary steps to use lysates.

### VII. Pharmaceutical compositions and matrixes comprising umbilical cord-tissue derived cells

In another aspect, herein disclosed are pharmaceutical compositions that utilize the UTC, UTC populations, components, and products of the UTC in various methods for the modulation of pro-inflammatory mediators involved in the pathology of a lung disease, disorders, and/or injury. Certain embodiments encompass pharmaceutical compositions comprising live cells (UTC alone or admixed with other cell types). Other embodiments encompass pharmaceutical compositions comprising UTC cellular components (*e.g*., cell lysates, soluble cell fractions, conditioned medium, ECM, or components of any of the foregoing) or products (*e.g*., trophic and other biological factors produced naturally by the UTC or through genetic modification, conditioned medium from UTC culture). The UTC components and products that can be used in the present invention are described in U.S. Patent Nos. 7,524,489, and 7,510,873, and U.S. Pub. App. No. 2005/0058634. In either case, the pharmaceutical composition may further comprise other active agents, such as anti-inflammatory agents, anti-apoptotic agents, antioxidants, growth factors, myotrophic factors, or myoregenerative or myoprotective drugs as known in the art.

In one embodiment, the pharmaceutical compositions comprise hUTC and a pharmaceutically acceptable carrier. Pharmaceutically acceptable carriers include liquids, semi-solid (*e.g*., gels) and solid materials (*e.g*., cell scaffolds and matrices, tubes sheets and other such materials as known in the art and described in greater detail herein). These semi-solid and solid materials may be designed to resist degradation within the body (non-biodegradable) or they may be designed to degrade within the body (biodegradable, bioerodable). A biodegradable material may further be bioresorbable or bioabsorbable, *i.e*., it may be dissolved and absorbed into bodily fluids (water-soluble implants are one example), or degraded and ultimately eliminated from the body, either by conversion into other materials or breakdown and elimination through natural pathways. The biodegradation rate can vary according to the desired release rate once implanted in the body.

Pharmaceutical compositions comprising UTC live cells are typically formulated as liquids, semisolids (*e.g*., gels) or solids (*e.g*., matrices, scaffolds and the like, as appropriate for vascular or lung tissue engineering). Liquid compositions are formulated for administration by any acceptable route known in the art to achieve delivery of live cells to the target vascular or lung tissues. Typically, these include injection or infusion, either in a diffuse fashion, or targeted to the site of lung injury, damage, or distress, by a route of administration including, but not limited to, intramuscular, intravenous, or intra-arterial delivery via syringes with needles and/or catheters with or without pump devices.

Pharmaceutical compositions comprising live cells in a semi-solid or solid carrier are typically formulated for surgical implantation at the site of lung injury, damage, or distress. It will be appreciated that liquid compositions also may be administered by surgical procedures. In particular embodiments, semi-solid or solid pharmaceutical compositions may comprise semi-permeable gels, lattices, cellular scaffolds and the like, which may be non-biodegradable or biodegradable. For example, in certain embodiments, it may be desirable or appropriate to sequester the exogenous cells from their surroundings, yet enable the cells to secrete and deliver biological molecules (*e.g*. myotrophic factors, angiotrophic factors, or endothelial progenitor cell recruitment factors) to surrounding lung tissue or vascular cells. In these embodiments, cells may be formulated as autonomous implants comprising a living UTC or cell population comprising a UTC surrounded by a non-degradable, selectively permeable barrier that physically separates the transplanted cells from host tissue. Such implants are sometimes referred to as "immunoprotective," as they have the capacity to prevent immune cells and macromolecules from killing the transplanted cells in the absence of pharmacologically induced immunosuppression.

In other embodiments, the pharmaceutical compositions may utilize different varieties of degradable gels and networks. For example, degradable materials particularly suitable for sustained release formulations include biocompatible polymers, such as poly(lactic acid), poly (lactic acid-co-glycolic acid), methylcellulose, hyaluronic acid, collagen, and the like.

In other embodiments, it may be desirable or appropriate to deliver the cells on or in a biodegradable, preferably bioresorbable or bioabsorbable, scaffold or matrix. These typically three-dimensional biomaterials contain the living cells attached to the scaffold, dispersed within the scaffold, or incorporated in an extracellular matrix entrapped in the scaffold. Once implanted into the target region of the body, these implants become integrated with the host tissue, wherein the transplanted cells gradually become established (See, *e.g.,* Tresco, P A, et al., Adv. Drug Delivery Rev., 2000; 42:3-27; *see also* Hutmacher, D.W., J. Biomater. Sci. Polymer Edn., 2001; 12:107-174).

The biocompatible matrix may be comprised of natural, modified natural or synthetic biodegradable polymers, including homopolymers, copolymers and block polymers, and combinations thereof. It is noted that a polymer is generally named based on the monomer from which it is synthesized.

Examples of suitable biodegradable polymers or polymer classes include fibrin, collagen, elastin, gelatin, vitronectin, fibronectin, laminin, thrombin, poly(aminoacid), oxidized cellulose, tropoelastin, silk, ribonucleic acids, deoxyribonucleic acids, proteins, polynucleotides, reconstituted basement membrane matrices, starches, dextrans, alginates, hyaluron, chitin, chitosan, agarose, polysaccharides, hyaluronic acid, poly(lactic acid), poly(glycolic acid), polyethylene glycol, decellularized tissue, self-assembling peptides, polypeptides, glycosaminoglycans, their derivatives and mixtures thereof. For both glycolic acid and lactic acid, an intermediate cyclic dimer is typically prepared and purified prior to polymerization. These intermediate dimers are called glycolide and lactide, respectively. Other useful biodegradable polymers or polymer classes include, without limitation, aliphatic polyesters, poly(alkylene oxalates), tyrosine derived polycarbonates, polyiminocarbonates, polyorthoesters, polyoxaesters, polyamidoesters, polyoxaesters containing amine groups, poly(propylene fumarate), polydioxanones, polycarbonates, polyoxalates, poly(alpha-hydoxyacids), poly(esters), polyurethane, poly(ester urethane), poly(ether urethane), polyanhydrides, polyacetates, polycaprolactones, poly(orthoesters), polyamino acids, polyamides and blends and copolymers thereof. Additional useful biodegradable polymers include, without limitation stereopolymers of L- and D-lactic acid, copolymers of bis(para-carboxyphenoxy) propane and sebacic acid, sebacic acid copolymers, copolymers of caprolactone, poly(lactic acid)/poly(glycolic acid)/polyethyleneglycol copolymers, copolymers of polyurethane and poly(lactic acid), copolymers of alpha-amino acids, copolymers of alpha-amino acids and caproic acid, copolymers of alpha-benzyl glutamate and polyethylene glycol, copolymers of succinate and poly(glycols), polyphosphazene, poly(hydroxyalkanoates) and mixtures thereof. Binary and ternary systems also are contemplated.

In general, a suitable biodegradable polymer for use as the matrix is desirably configured so that it: has mechanical properties that are suitable for the intended application; remains sufficiently intact until tissue has in-grown and healed; does not invoke an inflammatory or toxic response; is metabolized in the body after fulfilling its purpose; is easily processed into the desired final product to be formed; demonstrates acceptable shelf-life; and is easily sterilized.

In one aspect of the invention, the biocompatible polymer used to form the matrix is in the form of a hydrogel. In general, hydrogels are cross-linked polymeric materials that can absorb more than 20% of their weight in water while maintaining a distinct three-dimensional structure. This definition includes dry cross-linked polymers that will swell in aqueous environments, as well as water-swollen materials. A host of hydrophilic polymers can be cross-linked to produce hydrogels, whether the polymer is of biological origin, semisynthetic, or wholly synthetic. The hydrogel may be produced from a synthetic polymeric material. Such synthetic polymers can be tailored to a range of properties and predictable lot-to-lot uniformity, and represent a reliable source of material that generally is free from concerns of immunogenicity. The matrices may include hydrogels formed from self assembling peptides, as those discussed in U.S. Pat. Nos. 5,670,483 and 5,955,343, U.S. Pat. Pub. App. No. 2002/0160471, and PCT Pub. App. No. WO 02/062969, the disclosures of which relate to hydrogel forming self-assembling peptides.

Properties that make hydrogels valuable in drug delivery applications include the equilibrium swelling degree, sorption kinetics, solute permeability, and their *in vivo* performance characteristics. Permeability to compounds depends in part upon the swelling degree or water content and the rate of biodegradation. Since the mechanical strength of a gel declines in direct proportion to the swelling degree, it is also well within the contemplation of the present invention that the hydrogel can be attached to a substrate so that the composite system enhances mechanical strength. In some embodiments, the hydrogel can be impregnated within a porous substrate, to gain the mechanical strength of the substrate, along with the useful delivery properties of the hydrogel.

Non-limiting examples of scaffold or matrix (sometimes referred to collectively as "framework") that may be used in the present invention include textile structures such as weaves, knits, braids, meshes, non-wovens, and warped knits; porous foams, semi-porous foams, perforated films or sheets, microparticles, beads, and spheres and composite structures being a combination of the above structures. Non-woven mats may, for example, be formed using fibers comprised of a synthetic absorbable copolymer of glycolic and lactic acids (PGA/PLA), sold under the tradename VICRYL sutures (Ethicon, Inc., Somerville, N.J.). Foams, composed of, for example, poly(epsilon-caprolactone)/poly(glycolic acid) (PCL/PGA) copolymer, formed by processes such as freeze-drying, or lyophilization, as discussed in U.S. Pat. No. 6,355,699, also may be utilized. Hydrogels such as self-assembling peptides (*e.g*., RAD16) may also be used. *In* situ-forming degradable networks are also suitable for use in the invention (*See*, *e.g*., Anseth, K S et al., J. Controlled Release, 2002; 78:199-209; Wang, D. et al., Biomaterials, 2003; 24:3969-3980; U.S. Pub. App. No. 2002/0022676). These *in situ* forming materials are formulated as fluids suitable for injection, then may be induced to form a hydrogel by a variety of means such as change in temperature, pH, and exposure to light in situ or *in vivo.*

In another embodiment, the framework is a felt, which can be composed of a multifilament yarn made from a bioabsorbable material, *e.g*., PGA, PLA, PCL copolymers or blends, or hyaluronic acid. The yarn is made into a felt using standard textile processing techniques consisting of crimping, cutting, carding and needling. In another embodiment, cells are seeded onto foam scaffolds that may be composite structures.

In many of the above-mentioned embodiments, the framework may be molded into a useful shape, such as that of a blood vessel. Furthermore, it will be appreciated that UTC may be cultured on pre-formed, non-degradable surgical or implantable devices, e.g., in a manner corresponding to that used for preparing fibroblast-containing GDC endovascular coils, for instance (Marx, W. F. et al., Am. J. Neuroradiol., 2001; 22:323-333).

The matrix, scaffold, or device may be treated prior to inoculation of cells in order to enhance cell attachment. For example, prior to inoculation, nylon matrices can be treated with 0.1 molar acetic acid and incubated in polylysine, PBS, and/or collagen to coat the nylon. Polystyrene can be similarly treated using sulfuric acid. The external surfaces of a framework may also be modified to improve the attachment or growth of cells and differentiation of tissue, such as by plasma coating the framework or addition of one or more proteins (*e.g*., collagens, elastic fibers, reticular fibers), glycoproteins, glycosaminoglycans (*e.g*., heparin sulfate, chondroitin-4-sulfate, chondroitin-6-sulfate, dermatan sulfate, keratin sulfate), genetic materials such as cytokines and growth factors, a cellular matrix, and/or other materials, including, but not limited to, gelatin, alginates, agar, agarose, and plant gums, among other factors affecting cell survival and differentiation.

UTC-containing frameworks are prepared according to methods known in the art. For example, cells can be grown freely in a culture vessel to sub-confluency or confluency, lifted from the culture and inoculated onto the framework. Growth factors may be added to the culture medium prior to, during, or subsequent to inoculation of the cells to trigger differentiation and tissue formation, if desired. Alternatively, the frameworks themselves may be modified so that the growth of cells thereon is enhanced, or so that the risk of rejection of the implant is reduced. Thus, one or more biologically active compounds, including, but not limited to, anti-inflammatory compounds, immunosuppressants, or growth factors, may be added to the framework for local release.

A UTC, parts of a UTC, or cell populations comprising a UTC, or components of or products produced by a UTC, may be used in a variety of ways to support and facilitate the repair, regeneration, and improvement of lung cells and tissues, to improve blood flow, and to stimulate and/or support angiogenesis, especially in lung disease patients. Such utilities encompass *in vitro*, *ex vivo* and *in vivo* methods.

Specific embodiments are directed to the direct repair, regeneration or replacement of, or the support of the repair, regeneration, or replacement of blood vessels for the treatment of lung injury or damage.

The UTC may be administered alone (*e.g*., as substantially homogeneous populations) or as admixtures with other cells. As described above, the UTC may be administered as formulated in a pharmaceutical preparation with a matrix or scaffold, or with conventional pharmaceutically acceptable carriers. Where the UTC are administered with other cells, they may be administered simultaneously or sequentially with the other cells (either before or after the other cells). Cells that may be administered in conjunction with the UTC include, but are not limited to, myocytes, lung tissue cells, skeletal muscle progenitor cells, vascular smooth muscle cells, vascular smooth muscle progenitor cells, pericytes, vascular endothelial cells, or vascular endothelium progenitor cells, and/or other multipotent or pluripotent stem cells. The cells of different types may be admixed with the UTC immediately or shortly prior to administration, or they may be co-cultured together for a period of time prior to administration.

The UTC may be administered with other beneficial drugs or biological molecules, or other active agents, such as anti-inflammatory agents, anti-apoptotic agents, antioxidants, growth factors, angiogenic factors, or myoregenerative or myoprotective drugs as known in the art. When the UTC are administered with other agents, they may be administered together in a single pharmaceutical composition, or in separate pharmaceutical compositions, simultaneously or sequentially with the other agents (either before or after administration of the other agents). The other agents may be a part of a treatment regimen that begins either before transplantation and continuing throughout the course of recovery, or may be initiated at the time of transplantation, or even after transplantation, as a physician of skill in the art deems appropriate.

In one embodiment, the UTC are administered as undifferentiated cells, *i.e*., as cultured in growth medium. Alternatively, the UTC may be administered following exposure in culture to conditions that stimulate differentiation toward a desired lung tissue phenotype, for example vascular smooth muscle, pericyte, or vascular endothelium phenotypes.

The cells of the invention may be surgically implanted, injected, delivered (*e.g*., by way of a catheter, syringe, shunt, stent, microcatheter, or pump), or otherwise administered directly or indirectly to the site of lung injury, damage, or distress. Routes of administration of the cells of the invention or compositions thereof include, but are not limited to, intravenous, intramuscular, subcutaneous, intranasal, intrathecal, intracisternal, or via syringes with needles or catheters with or without pump devices.

When cells are administered in semi-solid or solid devices, surgical implantation into a precise location in the body is typically a suitable means of administration. Liquid or fluid pharmaceutical compositions, however, may be administered through the blood, or directly into affected lung tissue (*e.g*., throughout a diffusely affected area, such as would be the case for diffuse ALI or ARDS). The migration of the UTC can be guided by chemical signals, growth factors, or calpains.

The umbilical cord tissue-derived cells or compositions and/or matrices comprising the umbilical cord tissue-derived cells may be delivered to the site via a micro catheter, intracatheterization, or via a mini-pump. The vehicle excipient or carrier can be any of those known to be pharmaceutically acceptable for administration to a patient, particularly locally at the site at which cellular differentiation is to be induced. Examples include liquid media, for example, Dulbeccos Modified Eagle's Medium (DMEM), sterile saline, sterile phosphate buffered saline, Leibovitz's medium (L15, Invitrogen, Carlsbad, Calif.), dextrose in sterile water, and any other physiologically acceptable liquid.

Other embodiments encompass methods of modulating pro-inflammatory mediators involved in the pathology of a lung injury or damage by administering therapeutic compositions comprising a pharmaceutically acceptable carrier and UTC cellular components (*e.g*., cell lysates or components thereof) or products (*e.g*., trophic and other biological factors produced naturally by the UTC or through genetic modification, conditioned medium from UTC culture), or UTC growth medium or products purified from growth medium. In some embodiments, the biological factors are FGF and HGF. These methods may further comprise administering other active agents, such as growth factors, angiogenic factors, or myoregenerative or myoprotective drugs as known in the art.

Dosage forms and regimes for administering the UTC or any of the other therapeutic or pharmaceutical compositions described herein are developed in accordance with good medical practice, taking into account the condition of the individual patient, e.g., nature and extent of the injury or damage from the lung damaging event, age, sex, body weight and general medical condition, and other factors known to medical practitioners. Thus, the effective amount of a pharmaceutical composition to be administered to a patient is determined by these considerations as known in the art.

The UTC has been shown not to stimulate allogeneic PBMCs in a mixed lymphocyte reaction. Accordingly, allogeneic, or even xenogeneic, transplantation of a UTC may be tolerated in some instances. In some embodiments, the UTC itself provides an immunosuppressant effect, thereby preventing host rejection of the transplanted UTC. In such instances, pharmacological immunosuppression during cell therapy may not be necessary.

However, in other instances it may be desirable or appropriate to pharmacologically immunosuppress a patient prior to initiating cell therapy. This may be accomplished through the use of systemic or local immunosuppressive agents, or it may be accomplished by delivering the cells in an encapsulated device, as described above. These and other means for reducing or eliminating an immune response to the transplanted cells are known in the art. As an alternative, the UTC may be genetically modified to reduce their immunogenicity, as mentioned above.

Survival of the transplanted UTC in a living patient can be determined through the use of a variety of scanning techniques, *e.g*., computerized axial tomography (CAT or CT) scan, magnetic resonance imaging (MRI) or positron emission tomography (PET) scans. Determination of transplant survival can also be done post mortem by removing the lung tissue or vascular tissue, and examining it visually or through a microscope. Alternatively, cells can be treated with stains that are specific for lung tissue cells, for example vascular smooth muscle cells, pericytes, or vascular endothelial cells. Transplanted cells can also be identified by prior incorporation of tracer dyes such as rhodamine- or fluorescein-labeled microspheres, fast blue, ferric microparticles, bisbenzamide or genetically introduced reporter gene products, such as beta-galactosidase or beta-glucuronidase.

In another aspect, herein disclosed are kits that utilize the UTC, UTC populations, components, and products of the UTC in various methods for stimulating and/or supporting angiogenesis, for improving blood flow, for regenerating, repairing, and improving lung tissue injured or damaged by a lung-damaging event, as described above. Where used for treatment of damage or injury caused by a lung disease, disorders and/or injuries or other scheduled treatment, the kits may include one or more cell populations, including at least the UTC and a pharmaceutically acceptable carrier (liquid, semi-solid or solid). The kits also optionally may include a means of administering the cells, for example by injection. The kits further may include instructions for use of the cells. Kits prepared for field hospital use, such as for military use, may include full-procedure supplies including tissue scaffolds, surgical sutures, and the like, where the cells are to be used in conjunction with repair of acute injuries. Kits for assays and *in vitro* methods as described herein may contain one or more of: (1) a UTC or components or products of the UTC; (2) reagents for practicing the *in vitro* method; (3) other cells or cell populations, as appropriate; and (4) instructions for conducting the *in vitro* method.

Additionally as used in the following examples and elsewhere in the specification, the UTC useful in the invention may be isolated and characterized according to the disclosures of U.S. Patent Nos. 7,524,489, and 7,510,873, and U.S. Pub. App. No. 2005/0058634, which relate to the description, isolation, and characterization of hUTC.

### EXAMPLE 1

### Pulmonary Protective Efficacy In a Mouse Model of Hyperoxia-Induced Acute Lung Injury

This example illustrates the effectiveness of a human UTC (isolation and characterization of hUTC may be found at Examples 6 to 16) to enhance lung repair and regeneration in a model of hyperoxia induced lung injury.

### Umbilical Cell Culture and Isolation

Umbilicus-derived cells (UDC, hUTC) were prepared as described in U.S. Patent Nos. 7,524,489, and 7,510,873 and U.S. Pub. App. No. 2005/0058634. The cells were cultured to the desired passage and then cryogenically preserved.

### Animal Model

Female C57BL/6 mice (seven weeks of age) were obtained from Ace Animals (Boyertown, PA). Immediately prior to injection, hUTC were thawed at 37°C (water bath) and washed two times in phosphate buffered saline (PBS) and resuspended in 1 mL of PBS. Cells were counted using a hemocytometer. Cell viability was determined by trypan blue dye exclusion. Cells were reconstituted at a concentration of 1 x 10⁶ cells in 200 µl PBS.

The study outline is summarized in Table 1-1 below. On Day 0, cells (1 x 10⁶ hUTC in 200 µl PBS) or PBS vehicle were slowly administered to mice by intravenous tail vein injection using a 1 mL syringe and a 26-gauge needle and animals were then exposed to either room air or 90% O₂. Exposure to 90% O₂ was accomplished by placing the animals into a BioSpherix chamber (BioSpherix, LTD, Lacona, NY) that has been primed and equilibrated to 90% O₂ for 1 hour. Supportive care (heat support and NutriCal) was provided daily for these animals. Animal observations, mortality, survival, and percent oxygen concentrations for each tank were recorded two times a day. On day four post-treatment, animals were euthanized using 50 mg/mL Nembutal (pentobarbital).

**Table 1-1. Experimental design.**

| **Treatment group** | **Atmospheric treatment** | **Treatment** | **Number of animals** |
|---|---|---|---|
| 1 | Room Air | PBS | 12 |
| 2 | 90% O₂ | PBS | 12 |
| 3 | 90% O₂ | 1e⁶ hUTC | 12 |

### Bronchoalveolar lavage fluid (BALF) Total Protein Analysis

To determine the total protein in each sample, cell free BALF was analyzed using a BCA Protein Assay (Pierce). Analysis was completed using the Softmax 4.0 program and data was graphed using Graph Pad Prism Software.

### BALF and Lung Homogenate Cytokines/Chemokine Analysis

To prepare BALF, six (6) animals per treatment group were euthanized and lungs were lavaged once with 1.0 mL sterile PBS (Invitrogen) and the tubes were placed on wet ice. The BALF was centrifuged at 1000 rpm for 5 minutes and the supernatant fluid was removed and used for further analysis.

To prepare lung homogenates, six (6) animals per group were euthanized, subjected to whole body perfusion with PBS and the left lungs were dissected and placed on ice in Lysing Matrix D tubes and then centrifuged in a FastPrep® instrument at a speed of 4.0 for 40 seconds.

Cytokine/chemokine levels in both BALF and lung homogenate supernatant were determined using a mouse 22-multiplex bead kit (Millipore) following the manufacturer's protocol and analyzed using the BioRad Bioplex machine. The results were graphed and analyzed using GraphPad Prism Software.

### Human Cell Detection

Total RNA was isolated from mouse tissues by Asuragen, Inc., according to the company's standard operating procedures. The purity and quantity of total RNA samples were determined by absorbance readings at 260 and 280 nm using a NanoDrop ND-1000 UV spectrophotometer. RNA integrity was evaluated using an Agilent Bioanalyzer.

Human-specific assays for GAPDH mRNA (Hs99999905_m1_GAPDH) were used to estimate the number of hUTC within mouse lung tissue. Samples for quantitative RT-PCR (qRT-PCR) analysis using single-tube TaqMan® Assays (Applied Biosystems) were processed by Asuragen, Inc., according to the company's standard operating procedures. Dilutions of total RNA was reverse transcribed using the TaqMan® High Capacity cDNA Synthesis Kit (Applied Biosystems) according to the manufacturer's instructions and in a total reaction volume of 20 microliters per dilution. 50 ng input cDNA was then analyzed by PCR. All amplifications were performed in triplicate on a validated ABI 7500 real-time thermocycler. Following incubation at 95°C for 10 minutes, samples were amplified in 40 cycles of 95°C for 15 seconds, then 60°C for 1 minute. Total number of hUTC within the mouse lungs was estimated based on a standard curve generated by analyzing known amounts of purified hUTC total RNA.

### BALF Total Protein

Exposure to 90% O₂ for 4 days resulted in an increase in the total protein content of BALF compared to room air control animals (p < 0.01, Figure 1, Table 1-2). Furthermore, there was a statistically significant decrease in the total BALF protein in the 90% O₂ hUTC treatment group as compared to the 90% O₂ PBS treatment group (p < 0.05).

| **Table 1-2.** *BALF total protein concentration:* Total protein was measured using Pierce BCA Protein Assay. | | |
|---|---|---|
| **Treatment Group** | **Animal number** | **Total BALF protein Concentration (µg/dl)** |
| 1 | 1 | 401.89 |
| 1 | 2 | 1006.68 |
| 1 | 3 | 660.67 |
| 1 | 4 | 494.49 |
| 1 | 5 | 1432.64 |
| 1 | 6 | 76.23 |
| | Mean: | 678.77 |
| | Stdev: | 437.77 |
| 2 | 1 | 1701.60 |
| 2 | 2 | 1438.46 |
| 2 | 3 | 1197.13 |
| 2 | 4 | 2823.95 |
| 2 | 5 | 4482.76 |
| 2 | 6 | 3174.32 |
| | Mean: | 2469.70 |
| | Stdev: | 1260.74 |
| 3 | 1 | 984.87 |
| 3 | 2 | 691.20 |
| 3 | 3 | 1172.41 |
| 3 | 4 | 893.28 |
| 3 | 5 | 695.56 |
| 3 | 6 | 1359.95 |
| | Mean: | 966.21 |
| | Stdev: | 265.37 |

### BALF and Lung Homogenate Cytokine Analysis

Tables 1-3 to 1-6 show the chemokine/cytokine analysis of Lung Homogenate (Tables 1-3 and 1-4) and BALF (Bronchoalveolar lavage fluid) (Tables 1-5 and 1-6). The data is shown in data is also shown as graphs (Figure 2A and 2B). A statistically significant decrease in BALF keratinocyte factor (KC), gamma interferon-inducible cytokine (IP-10), interleukin 1α (IL-1α) and lung homogenate monocyte chemotactic factor-1 (MCP-1) was observed in animals treated with hUTC and exposed to 90% O₂ compared to animals treated with PBS vehicle and exposed to 90% O₂ (p < 0.02). (Figures 2A and 2B, Tables 1-3 and 1-4). (nd = not detected).

| **Table 1-3:** Lung Homogenate Cytokine Analysis | | | | | | | | | | | | |
|---|---|---|---|---|---|---|---|---|---|---|---|---|
| **Treatment group** | **Animal number** | **MIP1-α** | **GMCSF** | **MCP-1** | **KC** | **RANTES** | **IFg** | **IL-1β** | **IL-1α** | **GCSF** | **IP-10** | **IL-2** |
| 1 | 1 | 0 | 0 | 0 | 53 | 36 | 52 | 22 | 0 | 6 | 840 | 0 |
| 1 | 2 | 0 | 0 | 0 | 77 | 18 | 76 | 17 | 10 | 6 | 720 | 0 |
| 1 | 3 | 0 | 0 | 0 | 45 | 24 | 59 | 14 | 29 | 4 | 524 | 9 |
| 1 | 4 | 0 | 0 | 0 | 67 | 28 | 58 | 31 | 34 | 7 | 621 | 9 |
| 1 | 5 | 0 | 0 | 0 | 101 | 14 | 83 | 20 | 0 | 6 | 824 | 25 |
| 1 | 6 | 0 | 0 | 0 | 66 | 32 | 63 | 14 | 198 | 7 | 755 | 16 |
| **Mean:** | | **0** | **0** | **0** | **68** | **25** | **65** | **20** | **45** | **6** | **714** | **10** |
| **StdDev:** | | **0** | **0** | **0** | **20** | **9** | **12** | **6** | **76** | **1** | **122** | **10** |
| | | | | | | | | | | | | |
| 2 | 1 | 0 | 0 | 139 | 149 | 9 | 70 | 13 | 37 | 23 | 1315 | 8 |
| 2 | 2 | 0 | 0 | 526 | 555 | 4 | 51 | 4 | 18 | 171 | 1146 | 5 |
| 2 | 3 | 0 | 0 | 111 | 103 | 9 | 62 | 24 | 56 | 8 | 712 | 7 |
| 2 | 4 | 0 | 0 | 143 | 322 | 0 | 34 | 8 | 9 | 59 | 503 | 0 |
| 2 | 5 | 0 | 0 | 143 | 334 | 7 | 68 | 11 | 28 | 20 | 1783 | 8 |
| 2 | 6 | 0 | 0 | 295 | 303 | 4 | 39 | 9 | 11 | 75 | 619 | 4 |
| **Mean:** | | **0** | **0** | **226** | **294** | **5** | **54** | **11** | **27** | **59** | **1013** | **5** |
| **StdDev:** | | **0** | **0** | **161** | **160** | **4** | **15** | **7** | **18** | **60** | **492** | **3** |
| | | | | | | | | | | | | |
| 3 | 1 | 0 | 0 | 0 | 115 | 7 | 50 | 16 | 30 | 16 | 1121 | 8 |
| 3 | 2 | 0 | 0 | 0 | 163 | 11 | 50 | 9 | 38 | 20 | 1105 | 8 |
| 3 | 3 | 0 | 0 | 0 | 157 | 13 | 56 | 7 | 25 | 24 | 1465 | 7 |
| 3 | 4 | 0 | 0 | 0 | 161 | 16 | 59 | 8 | 24 | 12 | 1647 | 7 |
| 3 | 5 | 0 | 0 | 0 | 337 | 7 | 41 | 16 | 26 | 49 | 711 | 8 |
| 3 | 6 | 0 | 0 | 88 | 318 | 17 | 40 | 9 | 15 | 45 | 1839 | 8 |
| **Mean:** | | **0** | **0** | **15** | **209** | **12** | **49** | **11** | **27** | **27** | **1315** | **8** |
| **StdDev:** | | **0** | **0** | **36** | **94** | **4** | **8** | **4** | **8** | **15** | **413** | **1** |
| **t-test** | | **nd** | **nd** | **0.02** | **0.26** | **0.08** | **0.57** | **0.86** | **1.00** | **0.28** | **0.45** | **0.10** |

| **Table 1-4:** Lung Homogenate Cytokine Analysis | | | | | | | | | | | | |
|---|---|---|---|---|---|---|---|---|---|---|---|---|
| **Treatment group** | **Animal number** | **IL-4** | **IL-5** | **IL-6** | **IL-7** | **IL-10** | **IL-12p70** | **TNF-α** | **IL-9** | **IL-13** | **IL-15** | **IL-17** |
| 1 | 1 | 1 | 11 | 1 | 0 | 27 | 9 | 0 | 82 | 8 | 0 | 0 |
| 1 | 2 | 1 | 7 | 8 | 0 | 46 | 18 | 0 | 113 | 14 | 27 | 0 |
| 1 | 3 | 1 | 12 | 5 | 0 | 31 | 15 | 0 | 82 | 10 | 0 | 0 |
| 1 | 4 | 1 | 16 | 9 | 16 | 51 | 18 | 0 | 135 | 11 | 22 | 0 |
| 1 | 5 | 1 | 15 | 34 | 0 | 42 | 23 | 0 | 105 | 22 | 24 | 4 |
| 1 | 6 | 1 | 17 | 14 | 0 | 37 | 24 | 0 | 100 | 15 | 28 | 0 |
| **Mean:** | | **1** | **13** | **12** | **3** | **39** | **18** | **0** | **103** | **13** | **17** | **1** |
| **StdDev:** | | **0** | **4** | **12** | **6** | **9** | **6** | **0** | **20** | **5** | **13** | **1** |
| | | | | | | | | | | | | |
| 2 | 1 | 1 | 30 | 69 | 0 | 39 | 12 | 0 | 96 | 18 | 0 | 0 |
| 2 | 2 | 1 | 13 | 448 | 0 | 20 | 0 | 0 | 62 | 7 | 0 | 0 |
| 2 | 3 | 1 | 14 | 33 | 0 | 37 | 4 | 0 | 82 | 8 | 0 | 0 |
| 2 | 4 | 1 | 11 | 91 | 0 | 26 | 4 | 0 | 63 | 6 | 0 | 0 |
| 2 | 5 | 1 | 11 | 47 | 0 | 30 | 4 | 0 | 106 | 8 | 28 | 0 |
| 2 | 6 | 1 | 11 | 121 | 0 | 27 | 5 | 0 | 54 | 0 | 0 | 0 |
| **Mean:** | | **1** | **15** | **135** | **0** | **30** | **5** | **0** | **77** | **8** | **5** | **0** |
| **StdDev:** | | **0** | **7** | **156** | **0** | **7** | **4** | **0** | **21** | **6** | **11** | **0** |
| | | | | | | | | | | | | |
| 3 | 1 | 1 | 15 | 31 | 0 | 34 | 15 | 0 | 79 | 15 | 0 | 0 |
| 3 | 2 | 1 | 16 | 36 | 0 | 28 | 6 | 0 | 65 | 9 | 0 | 0 |
| 3 | 3 | 1 | 16 | 53 | 0 | 36 | 9 | 0 | 68 | 9 | 0 | 0 |
| 3 | 4 | 1 | 14 | 26 | 0 | 24 | 5 | 0 | 90 | 12 | 21 | 0 |
| 3 | 5 | 1 | 13 | 64 | 0 | 25 | 13 | 0 | 60 | 6 | 0 | 0 |
| 3 | 6 | 2 | 14 | 84 | 0 | 24 | 11 | 0 | 73 | 6 | 0 | 0 |
| **Mean:** | | **1** | **15** | **49** | **0** | **29** | **10** | **0** | **72** | **10** | **3** | **0** |
| **StdDev:** | | **0** | **1** | **22** | **0** | **5** | **4** | **0** | **11** | **4** | **8** | **0** |
| **t-test** | | **0.18** | **0.96** | **0.25** | **nd** | **0.63** | **0.01** | **nd** | **0.68** | **0.31** | **0.86** | **nd** |

| T**able 1-5:** BALF (Bronchoalveolar lavage fluid) Homogenate Cytokine Analysis | | | | | | | | | | | | |
|---|---|---|---|---|---|---|---|---|---|---|---|---|
| **Treatment group** | **Animal number** | **MIP1-α** | **GMCSF** | **MCP-1** | **KC** | **RANTES** | **IFg** | **IL-1β** | **IL-1α** | **GCSF** | **IP-10** | **IL-2** |
| 1 | 1 | 0 | 0 | 0 | 8 | 0 | 162 | 0 | 8 | 0 | 113 | 0 |
| 1 | 2 | 0 | 0 | 0 | 8 | 0 | 174 | 0 | 15 | 0 | 195 | 0 |
| 1 | 3 | 0 | 0 | 0 | 7 | 0 | 131 | 0 | 0 | 0 | 95 | 0 |
| 1 | 4 | 0 | 0 | 0 | 8 | 0 | 195 | 4 | 9 | 0 | 176 | 0 |
| 1 | 5 | 0 | 0 | 0 | 12 | 0 | 197 | 0 | 14 | 0 | 216 | 0 |
| 1 | 6 | 0 | 0 | 0 | 0 | 0 | 79 | 0 | 6 | 0 | 53 | 0 |
| **Mean:** | | **0** | **0** | **0** | **7** | **0** | **156** | **1** | **9** | **0** | **141** | **0** |
| **StdDev:** | | **0** | **0** | **0** | **4** | **0** | **45** | **2** | **6** | **0** | **64** | **0** |
| | | | | | | | | | | | | |
| 2 | 1 | 0 | 0 | 0 | 276 | 0 | 101 | 0 | 0 | 59 | 351 | 0 |
| 2 | 2 | 0 | 0 | 0 | 425 | 0 | 127 | 0 | 0 | 65 | 381 | 0 |
| 2 | 3 | 0 | 0 | 0 | 390 | 0 | 183 | 0 | 7 | 64 | 435 | 0 |
| 2 | 4 | 0 | 0 | 0 | 533 | 0 | 107 | 0 | 0 | 284 | 453 | 0 |
| 2 | 5 | 0 | 0 | 0 | 322 | 0 | 140 | 0 | 0 | 70 | 568 | 0 |
| 2 | 6 | 0 | 0 | 0 | 501 | 0 | 104 | 0 | 0 | 234 | 554 | 0 |
| **Mean:** | | **0** | **0** | **0** | **408** | **0** | **127** | **0** | **1** | **129** | **457** | **0** |
| **StdDev:** | | **0** | **0** | **0** | **100** | **0** | **31** | **0** | **3** | **102** | **89** | **0** |
| | | | | | | | | | | | | |
| 3 | 1 | 0 | 0 | 0 | 175 | 0 | 151 | 0 | 6 | 50 | 346 | 0 |
| 3 | 2 | 0 | 0 | 0 | 256 | 0 | 108 | 0 | 5 | 45 | 201 | 0 |
| 3 | 3 | 0 | 0 | 0 | 331 | 0 | 169 | 0 | 7 | 51 | 403 | 0 |
| 3 | 4 | 0 | 0 | 0 | 222 | 0 | 134 | 0 | 12 | 37 | 246 | 0 |
| 3 | 5 | 0 | 0 | 0 | 175 | 0 | 140 | 0 | 14 | 33 | 347 | 0 |
| 3 | 6 | 0 | 0 | 0 | 252 | 0 | 156 | 0 | 9 | 44 | 292 | 0 |
| **Mean:** | | **0** | **0** | **0** | **235** | **0** | **143** | **0** | **9** | **43** | **306** | **0** |
| **StdDev:** | | **0** | **0** | **0** | **59** | **0** | **21** | **0** | **3** | **7** | **74** | **0** |
| **t-test** | | **nd** | **nd** | **nd** | **0.01** | **nd** | **0.26** | **nd** | **0.01** | **0.10** | **0.02** | **nd** |

| Table 1-6: BALF (Bronchoalveolar lavage fluid) Homogenate Cytokine Analysis | | | | | | | | | | | | |
|---|---|---|---|---|---|---|---|---|---|---|---|---|
| **Treatment group** | **Animal number** | **IL-4** | **IL-5** | **IL-6** | **IL-7** | **IL-10** | **IL-12p70** | **TNF-α** | **IL-9** | **IL-13** | **IL-15** | **IL-17** |
| 1 | 1 | 0 | 0 | 1 | 0 | 39 | 0 | 0 | 63 | 6 | 0 | 0 |
| 1 | 2 | 0 | 0 | 1 | 0 | 19 | 0 | 0 | 170 | 4 | 0 | 0 |
| 1 | 3 | 0 | 0 | 0 | 0 | 26 | 0 | 0 | 82 | 0 | 0 | 0 |
| 1 | 4 | 0 | 0 | 1 | 0 | 35 | 4 | 0 | 175 | 5 | 0 | 0 |
| 1 | 5 | 0 | 0 | 1 | 0 | 39 | 0 | 0 | 221 | 6 | 0 | 0 |
| 1 | 6 | 0 | 0 | 1 | 0 | 11 | 0 | 0 | 82 | 4 | 0 | 0 |
| **Mean:** | | **0** | **0** | **1** | **0** | **28** | **1** | **0** | **132** | **4** | **0** | **0** |
| **StdDev:** | | **0** | **0** | **0** | **0** | **11** | **1** | **0** | **65** | **2** | **0** | **0** |
| | | | | | | | | | | | | |
| 2 | 1 | 0 | 4 | 24 | 0 | 14 | 0 | 0 | 41 | 4 | 0 | 0 |
| 2 | 2 | 0 | 0 | 36 | 0 | 24 | 0 | 0 | 46 | 5 | 0 | 0 |
| 2 | 3 | 1 | 0 | 27 | 0 | 35 | 0 | 0 | 102 | 7 | 0 | 0 |
| 2 | 4 | 0 | 4 | 384 | 0 | 14 | 0 | 0 | 49 | 0 | 0 | 0 |
| 2 | 5 | 0 | 0 | 84 | 0 | 23 | 0 | 0 | 84 | 11 | 0 | 0 |
| 2 | 6 | 0 | 4 | 347 | 0 | 13 | 0 | 0 | 121 | 7 | 0 | 0 |
| **Mean:** | | **0** | **2** | **150** | **0** | **21** | **0** | **0** | **74** | **6** | **0** | **0** |
| **StdDev:** | | **0** | **2** | **169** | **0** | **9** | **0** | **0** | **34** | **4** | **0** | **0** |
| | | | | | | | | | | | | |
| 3 | 1 | 0 | 0 | 67 | 0 | 32 | 0 | 0 | 60 | 0 | 0 | 0 |
| 3 | 2 | 0 | 0 | 33 | 0 | 23 | 0 | 0 | 41 | 4 | 0 | 0 |
| 3 | 3 | 0 | 0 | 37 | 0 | 35 | 4 | 0 | 110 | 6 | 0 | 0 |
| 3 | 4 | 0 | 0 | 61 | 0 | 24 | 0 | 0 | 69 | 4 | 0 | 0 |
| 3 | 5 | 0 | 4 | 150 | 0 | 25 | 0 | 0 | 76 | 4 | 0 | 0 |
| 3 | 6 | 0 | 0 | 35 | 0 | 28 | 0 | 0 | 77 | 4 | 0 | 0 |
| **Mean:** | | **0** | **1** | **64** | **0** | **28** | **1** | **0** | **72** | **4** | **0** | **0** |
| **StdDev:** | | **0** | **2** | **45** | **0** | **5** | **2** | **0** | **23** | **2** | **0** | **0** |
| **t-test** | | **0.36** | **0.40** | **0.29** | **nd** | **0.08** | **0.36** | **nd** | **0.88** | **0.27** | **nd** | **nd** |

### Human Cell Engraftment

On day four post-treatment, animals were sacrificed, lungs were harvested and total RNA was isolated for human cell detection. Results showed the presence of hUTC within the lungs of hUTC treated animals, but absent from the lungs of PBS treated animals (Table 1-3).

Table 1-7 shows the results of human cell detection. The presence of hUTC within mouse lungs at day four-post treatment was determined by measuring human specific GAPDH mRNA transcripts using real-time PCR. Cycle threshold (CT) values less than 34 indicate that hUTC are present within the mouse lung tissue. No hUTC mRNA transcripts detected within mouse lung tissue (Absent). hUTC mRNA transcripts detected within mouse lung tissue (Present).

| **Table 1-7.** *Human cell detection.* | | |
|---|---|---|
| **Treatment group** | **Average CT value** | **HUTC within mouse lung** |
| 1 | 36.1 | Absent |
| 1 | 36.5 | Absent |
| 2 | 34.9 | Absent |
| 2 | 34.4 | Absent |
| 3 | 26.2 | Present |
| 3 | 29.6 | Present |
| 3 | 26.6 | Present |
| 3 | 26.9 | Present |
| 3 | 26.1 | Present |
| 3 | 26.5 | Present |

The effect of prophylactic intravenous administration of hUTC on the development of hyperoxia induced acute lung injury in mice was evaluated. The reduced level of total protein in the BALF, following hUTC administration in mice exposed to 90% O₂, suggests that hUTC were able to reduce hyperoxia induced vascular leak/edema in the lung. In addition, the data showed that hUTC caused a reduction in the levels of three important chemokines suggesting reduced inflammation in the lung. These data provide evidence that hUTC might be an important therapeutic agent for the treatment of lung disease.

### EXAMPLE 2

### Evaluation of the Protective Efficacy of Human Umbilical Tissue Derived Cells (hUTC) in a Novel Humanized Murine Model of Elastase-Induced Emphysema

This example evaluated the efficacy of human umbilical tissue-derived cells (hUTC) in both a novel and classical model of COPD (emphysema). These models were based on delivery of elastase to the airways leading to emphysematous destruction. The classical model used BALB/c mice; the novel model used NOD/SCID/Cytokine receptor gamma chain null mice (NOD/SCIDy) (hereafter "NSG"), which have been developed as a test bed for testing human cell therapies.

### Study Design

Mice were anesthetized by inhalation of isofluorane and given six intranasal administrations of porcine pancreatic elastase (Sigma-Aldrich, St. Louis, MO) over the course of fourteen days (1x 30µg every 3 times a week). Control mice received intranasal administration of saline alone. Two hours after the first elastase treatment, 0.5 x 10⁶ human umbilical tissue cells (hUTC) were administered via tail vein injection. hUTC were administered as a single dose in a total volume of 100 µl. Vehicle alone was administered in a similar fashion as in the hUTC treatment groups.

The study encompassed biochemical/protein analysis (Part 1) and (2) histology and lung function testing (plethysmography) (Part 2).

For this study, mice were injected porcine pancreatic elastase (PPE) intranasally (i.n.) and hUTC intravenously (i.v.) at day 0. At day 2, 5, 7, 9 and 11, the mice received further injections of PPE. At day 1, 6, 10 and 14 mice were harvested for further analysis. 320, 6 to 8 weeks old, female mice were used the strain/species: NOD/SCIDy (160) or wild type (BALB/C) *Mus muscularis* (160). Table 2-1 summarizes the experimental design.

| **Table 2-1: Summary of Experimental Design** | | |
|---|---|---|
| Group Number | Number of Animals | Test Material |
| 1 | 20 (n=5 @ 4 time points) | Wild type: Saline i.n. + vehicle |
| 2 | 20 | Wild type: elastase i.n. + vehicle |
| 3 | 20 | Wild type: elastase i.n. + hUTC |
| 4 | 16 (n=4 @ 4 time points | Wild type: Saline i.n. + hUTC |
| 5 | 4 (Harvest all on day 14) | No treatment |
| 6 | 20 | NOD/SCIDγ: Saline i.n. + vehicle |
| 7 | 20 | NOD/SCIDγ: elastase i.n. + vehicle |
| 8 | 20 | NOD/SCIDγ: elastase i.n.+ hUTC |
| 9 | 16 | NOD/SCIDγ: saline i.n. + hUTC |
| 10 | 4 | No treatment |

### Umbilical Cell Culture and Isolation

Umbilicus-derived cells ("UDC" or "hUTC") were prepared as described in U.S. Patent Nos. 7,524,489, and 7,510,873 and U.S. Pub. App. No. 2005/0058634. The cells were cultured to the desired passage and then cryogenically preserved.

### Dose preparation

Immediately prior to injection, hUTC were thawed at 37 °C (water bath). Cells were counted using a hemocytometer. Cell viability was determined by trypan blue dye exclusion. Cells needed to have a viability of 80% or greater at the time of injection. If viability was less than 80%, then cells were discarded. Cells were adjusted to the appropriate concentration with vehicle, to 100 ul. Cells suspended in vehicle were administered via tail vein injection using a syringe pump, a suitable small volume syringe, and a 28-gauge needle. Cells were administered slowly over 8 to 9 minutes to deliver cells at approximately 0.33 mL/min/kg with recording of delivery times. Cell administration occurred within 80 minutes of preparation. Cells were kept on wet ice prior to administration and the times of administration were recorded.

### Procedures

As discussed above, animals in Part 1 of the study were dedicated to biochemical/protein analysis while animals in Part 2 were dedicated to histology and lung function testing (plethysmography).

For the biochemical/protein analysis (Part 1), on day 1, 6, 10, and 14 after vehicle or hUTC injections, four or five animals from each group were sacrificed. Bronchoalveolar lavage fluid (BALF) was obtained from each animal and stored at -70 °C until time of analyses of cytokines present in BALF. Lungs, liver, and spleen were flash frozen and stored in RNA*later*® (Life Technologies, Grand Island, NY) at -70 °C for subsequent RNA extraction. Aliquots of liver and spleen were stored in RNA*later*® for additional transcriptional analysis.

### Analysis of trophic factors in BALF

Cellularity and cytokine/chemokine levels (murine MCP-1, IL-1β, TNF-α, RANTES) in both BALF and lung homogenate supernatant were determined using a mouse multiplex bead array kit (Becton Dickinson) following the manufacturer's protocol and analyzed using bead array flow cytometry. Murine targets selected as focus is on reduction of pathologic mechanisms. Candidate human effector targets included human HGF, human IL-1RA, and VEGF. Human factors were analyzed in pooled material at one time point only for feasibility. Total protein in BALF was quantified using a BCA™ Protein Assay (Pierce, Rockford, Ill.) to allow normalization within sample groups.

### Analysis of trophic factors in lung homogenate

A sample of cells from lung homogenate was assessed by quantitative RT-PCR for murine MCP-1, IL-1β, TNF-α, RANTES and human HGF, human IL-1RA and VEGF to assess markers of pathology (MCP-1, TNF-α, IL-1β and RANTES) or indicators for therapeutic activity (human HGF, IL-1RA or VEGF). RT-PCR for human IL-1RA and VEGF in lung tissue homogenates showed no detection of either cytokine at any time point.

Part 2 replicated Part 1 but exploited readouts not compatible with the procedures in Part 1. On day 1, 6, 10 and 14 after vehicle or hUTC injections, restrained plethysmography was carried out on animals from each group. Furthermore, four or five mice were sacrificed at each time point and lungs sampled as described below.

### Plethysmography

Plethysmography was performed on mice at each time point and at the end of experiment. Briefly, lung function was measured by restrained methods to determine respiratory frequency, tidal volume, relaxation time, peak inspiratory and expiratory flow, EF50, and change in lung volume. This indicates the impact of cell therapy on lung function at different times

### Histopathology

Lungs were obtained from each treatment group at each time point. Lungs were fixed with 10% formaldehyde neutral buffer solution, dehydrated in a graded ethanol series, embedded in paraffin, and sliced at 4 µm. The paraffin sections were stained with hematoxylin-eosin (H&E) for histopathology analysis. The histology sections were evaluated for injury. Alveolar surface area was determined using histolopatholy images analyzed using Image J software available online from the National Institute of Health. For each lung section, the surface area of alveoli within five random fields was measured and the mean surface area was calculated. It is well known that a reduction in the size of the alveolar space in an injured lung correlates with improved alveolar elasticity and compliance, and this indicates the impact of cell therapy on these parameters.

### Results

### Reconstitution of cells for injection

Of the 10 vials of hUTC used in this experiment, none were discarded on quality criteria. Viability was >80% in each case. The recovery details of the cells are shown in Tables 2-2 and 2-3 below. The recovery was high and slightly exceeded 100% of expected value (probably due to recovering slightly greater volume than expected from vials).

| **Table 2-2 Recovery Details** | | | | | |
|---|---|---|---|---|---|
| | Via Lot FV23L10B | No. of cells on vial (x10⁶) | Date | Time thawed | Time Counted |
| 1 | 267 | 5 | 12-APR | 11.43 | 11.55 |
| 2 | 252 | 5 | 12-APR | 11.43 | 11.55 |
| 3 | 288 | 5 | 12-APR | 11.43 | 11.55 |
| 4 | 265 | 5 | 12-APR | 11.43 | 11.55 |
| 5 | 257 | 5 | 12-APR | 11.43 | 11.55 |
| TOTAL | | 25 | | | |

| **Table 2-3 Recovery Details** | | | | | |
|---|---|---|---|---|---|
| | Via Lot FV23L10B | No. of cells on vial (x10⁶) | Date | Time thawed | Time Counted |
| 1 | 249 | 5 | 19-APR | 10.15 | 10.2 |
| 2 | 264 | 5 | 19-APR | 10.15 | 10.2 |
| TOTAL | | 10 | | | |
| 3 | 260 | 5 | 19-APR | 11.57 | 12.03 |
| 4 | 278 | 5 | 19-APR | 11.57 | 12.03 |
| 5 | 263 | 5 | 19-APR | 11.57 | 12.03 |
| TOTAL | | 15 | | | |

### Animal Reports & Observations

Some mouse tails showed white patching during intravenous injection. This was unrelated to cell therapy, but misplacement of administration needle during delivery. When this occurred, the needle was removed and inserted correctly into the vein and the administration of cells/vehicle was continued. No animals died as a result of either elastase treatment or cell delivery. No detectable adverse effects were observed in any animals as a result of hUTC delivery.

### Analysis of Bronchoalveolar Lavage Fluid (BALF)

At each time point, mice were sacrificed by lethal injection of sodium pentobarbital, and BALF was collected. Total leukocytes (Figure 3) and differential cell counts (Figure 4) were performed on the cell pellet. The resulting supernatant was retrieved for further analysis of cytokine activity (Figures 5 & 6) and total protein levels (Tables 2.4 to 2.7).

### Total leukocytes in BALF.

Control mice showed minimal cell infiltration in bronchoalveolar lavage, whereas elastase administration resulted in significant infiltration of cell. Total cellular infiltration was decreased in elastase-treated mice that received hUTC in both mouse strains.

### Differential cell staining of BALF.

Cytospins were carried out on BALF and stained using a modified Giemsa staining protocol. The samples were analyzed for the presence of lymphocytes, macrophages, and neutrophils. One hundred cells per cytospin were counted and counts were corrected for BALF volume. The results were expressed as means ± standard errors. Comparisons among three or more groups were made by ANOVA. Differences were considered significant at p<0.05.

*Summary of differential staining analysis.* In NSG mice, hUTC therapy resulted in significantly decreased macrophages at day 10 in elastase-treated mice when compared to elastase-treated mice that did not receive hUTC. Similarly, a marked reduction in neutrophils was observed at day 6 and 10 in elastase-treated mice that received hUTC. In contrast, no significant decrease in either macrophages or neutrophils was observed in elastase-treated wild-type mice that received hUTC at any time point.

### Cytokine Analysis.

BALF and lung tissue homogenate were extracted and analyzed by multiplex bead array for inflammatory mediators MCP-1, TNF-α, RANTES and IL-1β as these play well-defined roles in the mucus hypersecretion, destruction of airway parenchyma, fibrosis, tissue damage and inflammation that is associated with COPD.

hUTC modulated cytokine responses in bronchoalveolar lavage fluid (BALF) supernatant in elastase-treated NSG (NOD/SCIDy) mice (Figure 5). Non-elastase treated control mice exhibited little or no MCP-1, TNF-α, RANTES and IL-1β whereas elastase-treated mice exhibited typical increases in all target cytokines at each time point. However, a significant reduction in inflammatory mediators (* p<0.05) was observed in BALF in those mice receiving hUTC (*see* Figure 5) for RANTES day 1/10; IL-1β day 1; TNF-α day (d) 1, 6, 10; MCP-1 day 1, day 10.

hUTC and cytokine responses in bronchoalveolar lavage fluid (BALF) supernatant were determined for elastase-treated wild-type BALB/c strain mice (Figure 6). The effect of hUTC infusion and/or porcine pancreatic elastase (PPE) treatment on BAL supernatant composition at 1, 6, 10, and 14 days following hUTC administration is shown. In brief, responses were more variable in this strain and consistent or significant differences were not observed.

### Total protein concentration.

The total protein concentration in recovered BALF and lung tissue homogenate was determined using the Bradford assay (Bio-Rad, Hercules, CA), which was standardized using bovine serum albumin (BSA) *(see* Tables 2-4 to 2.7 below).

**Table 2-4 Protein concentrations in BALF of NSG mice**

| **Total protein (µg/ml) ± S.E.M.** | | | | | |
|---|---|---|---|---|---|
| | ***n*** | **d1** | **d6** | **d10** | **d14** |
| **PBS** | 5 | 1.051 ± 0.173 | 1.040 ± 0.167 | 1.505 ± 0.121 | 0.600 ± 0.140 |
| **Elastase** | 5 | 1.512 ± 0.284 | 0.852 ± 0.0453 | 1.929 ± 0.213 | 0.542 ± 0.188 |
| **Elastase +** hUTC | 5 | 1.571 ± 0.353 | 1.639 ± 0.236 | 1.232 ± 0.259 | 1.037 ± 0.180 |
| hUTC | 4 | 0.880 ± 0.1028 | 1.201 ± 0.144 | 1.129 ± 0.265 | 0.692 ± 0.013 |

**Table 2-5 Protein concentrations in BALF of BALB/c mice**

| **Total protein (µg/ml) ± S.E.M.** | | | | | |
|---|---|---|---|---|---|
| | ***n*** | **d1** | **d6** | **d10** | **d14** |
| **PBS** | 5 | 9.302 ± 1.401 | 7.446 ± 0.189 | 4.632 ± 0.239 | 3.993 ± 0.156 |
| **Elastase** | 5 | 9.872 ± 0.317 | 7.058 ± 0.0444 | 3.604 ± 0.530 | 3.226 ± 0.765 |
| **Elastase + hUTC** | 5 | 8.844 ± 0.989 | 6.340 ± 0.361 | 3.617 ± 0.677 | 3.514 ± 0.309 |
| **hUTC** | 4 | 5.388 ± 0.560 | 4.581 ± 0.123 | 3.755 ± 0.306 | 2.165 ± 0.198 |

**Table 2-6 Protein concentrations in lung tissue homogenate of NSG mice**

| **Total protein (µg/ml)** ± **S.E.M.** | | | | | |
|---|---|---|---|---|---|
| | ***n*** | **d1** | **d6** | **d10** | **d14** |
| **PBS** | 5 | 0.558 ± 0.157 | 2.845 ± 0.749 | 2.272 ± 0.346 | 2.171 ± 0.756 |
| **Elastase** | 5 | 0.309 ± 0.031 | 1.623 ± 0.458 | 2.360 ± 0.522 | 2.298 ± 0.425 |
| **Elastase + hUTC** | 5 | 0.602 ± 0.094 | 1.730 ± 0.448 | 3.465 ± 0.549 | 1.592 ± 0.525 |
| **hUTC** | 4 | 0.872 ± 0.180 | 0.403 ± 0.147 | 2.491 ± 0.647 | 1.214 ± 0.481 |

**Table 2-7 Protein concentrations in lung tissue homogenate of BALB/c mice**

| **Total protein (µg/ml) ± S.E.M.** | | | | | |
|---|---|---|---|---|---|
| | ***n*** | **d1** | **d6** | **d10** | **d14** |
| **PBS** | 5 | 1.277 ± 0.403 | 0.955 ± 0.281 0.852 ± | 0.428 ± 0.089 | 0.283 ± 0.071 |
| **Elastase** | 5 | 1.781 ± 0.424 | 0.0453 | 0.897 ± 0.248 | 0.250 ± 0.037 |
| **Elastase + hUTC** | 5 | 1.057 ± 0.256 | 1.639 ± 0.236 | 0.624 ± 0.070 | 0.198 ± 0.068 |
| **hUTC** | 4 | 1.399 ± 0.318 | 1.201 ± 0.144 | 0.461 ± 0.111 | 0.192 ± 0.069 |

No significant difference in total protein was detected between treatment groups in either strain.

### Histology

*Quantitative analysis.* Lungs from non-lavaged mice were removed and fixed in 10% (v/v) formalin/PBS, embedded in paraffin, sectioned and stained with haematoxylin/eosin (H&E) (*see* Figure 7). Air space enlargement was quantified. The linear intercepts of alveoli were measured, and the mean linear intercept (Lm) was used as a morphometric parameter of emphysema. From each lung sample, three (3) representative nonoverlapping fields were selected. A randomly distributed grid was overlaid onto the image of H&E-stained section. The following quantitative measures were calculated: Mean linear intercept (Lm); and number of alveoli. Lm represents the average size of alveoli. Bronchi and blood vessels were excluded from the measurements.

### Histopathology

Lungs from non-lavaged mice were removed and fixed in 10% (v/v) formalin/PBS, embedded in paraffin, sectioned and stained with haematoxylin/eosin (H&E). Figure 8 shows representative photomicrographs from each treatment group at day 1. Elastase-treated mice that subsequently received hUTC showed significantly reduced pathology when compared to elastase-treated mice that received saline at the same time point. The airspace enlargement because of elastase treatment was attenuated by hUTC administration (Figure 8 and associated legend p17). Figure 9 shows that hUTC reduced the extent of elastase-induced emphysema in immune-compromised (NOD/SCIDy) mice at day 1, 6, 10, and 14. Figure 10 shows that hUTC reduced the extent of elastase-induced emphysema in immuno-competent (BALB/c) mice at day 1, 6, 10, and 14.

### Lung function

Plethysmography was performed on each mouse at each time point and at the end of experiment as described in the previous study. Lung function was assessed by restrained methods to determine respiratory frequency, tidal volume, relaxation time, peak inspiratory and expiratory flow, EF50, and change in lung volume. No significant differences were detected between groups (statistical analysis by one-way ANOVA). All parameters are presented in the Figures 11A and 11B.

### Analysis of trophic factors in lung homogenate

A sample of cells from the lung homogenate was assessed by quantitative RT-PCR to measure mRNA for human HGF, IL-1RA and VEGF to assess indicators of therapeutic activity. No human HGF, VEGF, or IL-1RA was detected in lung homogenate using the methods described here.

### Conclusion

This study evaluated the efficacy of hUTC in standard or novel murine models of emphysema, a type of chronic pulmonary disease. Elastase was instilled into the lungs of both mouse strains, producing changes characteristic of human and experimental emphysema: larger, but fewer alveoli and reduced alveolar surface area due to the destruction of the alveolar walls; increased production of inflammatory mediators; influx of inflammatory cell types into the bronchoalveolar lavage fluid. Treatment with hUTC inhibited these effects in the models, preventing or remediating emphysema (*i.e.* COPD) and suggesting the possibility of a similar effect in humans.

These data have a number of implications. First, hUTC are effective in the prevention of key emphysematous features of COPD in a novel human-in-mouse model. hUTC inhibited the production of pro-inflammatory mediators (TNF-α, RANTES, MCP-1 and IL-Iβ) typically associated with pathology in the NSG model. More importantly hUTC significantly reduced the extent of elastase-induced emphysema in murine lungs, and hUTC treated COPD mice (both models) showed significantly shorter mean linear intercept (Lm) between alveoli (*i.e*. less destruction) than untreated emphysematous controls. Likewise, hUTC treatment preserved an increased number of alveoli and reduced the inflammatory cell infiltrate in BAL fluid in the humanized NSG model of COPD. Thus, hUTC effectively disrupt the key features of pathology.

The NSG model is also useful test bed for examining human cell therapeutics and provides an opportunity to perform future work supporting hUTC as an effective cell therapy. This model also allows opportunities for more fundamental examination of mechanisms of therapeutic action and indeed questions around timing and frequency of administration.

Overall, the differences in pathology and reduction in inflammatory mediators after a single hUTC administration are strongly supportive of hUTC efficacy in the novel model.

Thus, the key findings of this experiment were as follows:

hUTC inhibit the production of pro-inflammatory mediators (TNF-α, RANTES, MCP-1 and IL-1β) in a "humanized" (NSG) COPD model and do not modulate these cytokine responses in the immunocompetent murine model.

hUTC significantly reduced the extent of elastase-induced emphysema in murine lungs in both models.

hUTC treated COPD mice (both models) showed significantly shorter mean linear intercept (Lm) between alveoli (*i.e.* less destruction) than untreated emphysematous controls. Likewise, hUTC treatment preserved an increased number of alveoli.

hUTC reduced the cell infiltrate in BAL fluid in the humanized (NSG) COPD model.

### EXAMPLE 3

### Isolation of Cells

**Umbilical cell isolation.** Umbilical cords were obtained from National Disease Research Interchange (NDRI, Philadelphia, PA). The tissues were obtained following normal deliveries. The cell isolation protocols were performed aseptically in a laminar flow hood. To remove blood and debris, the cord was washed in phosphate buffered saline (PBS; Invitrogen, Carlsbad, CA) in the presence of penicillin at 100 Units/milliliter and streptomycin at 100 milligrams/milliliter, and amphotericin B at 0.25 micrograms/milliliter (Invitrogen Carlsbad, CA). The tissues were then mechanically dissociated in 150 cm² tissue culture plates in the presence of 50 milliliters of medium (DMEM-low glucose or DMEM-high glucose; Invitrogen), until the tissue was minced into a fine pulp. The chopped tissues were transferred to 50 milliliter conical tubes (approximately 5 grams of tissue per tube).

The tissue was then digested in either DMEM-low glucose medium or DMEM-high glucose medium, each containing penicillin at 100 Units/milliliter, streptomycin at 100 milligrams/milliliter, amphotericin B at 0.25 micrograms/milliliter, and the digestion enzymes. In some experiments an enzyme mixture of collagenase and dispase was used ("C:D") (collagenase (Sigma, St Louis, MO), 500 Units/milliliter; and dispase (Invitrogen), 50 Units/milliliter, in DMEM-low glucose medium). In other experiments a mixture of collagenase, dispase and hyaluronidase ("C:D:H") was used (C:D:H = collagenase, 500 Units/milliliter; dispase, 50 Units/milliliter; and hyaluronidase (Sigma), 5 Units/milliliter, in DMEM-low glucose). The conical tubes containing the tissue, medium and digestion enzymes were incubated at 37°C in an orbital shaker (Environ, Brooklyn, NY) at 225 rpm for 2 hours.

After digestion, the tissues were centrifuged at 150 x g for 5 minutes, the supernatant was aspirated. The pellet was resuspended in 20 milliliters of growth medium (DMEM:Low glucose (Invitrogen), 15 percent (v/v) fetal bovine serum (FBS; defined fetal bovine serum; Lot #AND 18475; Hyclone, Logan, UT), 0.001% (v/v) 2-mercaptoethanol (Sigma), penicillin at 100 Units per milliliter, streptomycin at 100 micrograms per milliliter, and amphotericin B at 0.25 micrograms per milliliter; (each from Invitrogen, Carlsbad, CA)). The cell suspension was filtered through a 70-micron nylon BD FALCON Cell Strainer (BD Biosciences, San Jose, CA). An additional 5 milliliters rinse comprising growth medium was passed through the strainer. The cell suspension was then passed through a 40-micrometer nylon cell strainer (BD Biosciences, San Jose, CA) and chased with a rinse of an additional 5 milliliters of growth medium.

The filtrate was resuspended in growth medium (total volume 50 milliliters) and centrifuged at 150 x g for 5 minutes. The supernatant was aspirated and the cells were resuspended in 50 milliliters of fresh growth medium. This process was repeated twice more.

After the final centrifugation, supernatant was aspirated and the cell pellet was resuspended in 5 milliliters of fresh growth medium. The number of viable cells was determined using trypan blue staining. Cells were then cultured under standard conditions.

The cells isolated from umbilical cord tissues were seeded at 5,000 cells/cm² onto gelatin-coated T-75 flasks (Corning Inc., Corning, NY) in growth medium. After two days, spent medium and unadhered cells were aspirated from the flasks. Adherent cells were washed with PBS three times to remove debris and blood-derived cells. Cells were then replenished with growth medium and allowed to grow to confluence (about 10 days from passage 0) to passage 1. On subsequent passages (from passage 1 to 2 etc.), cells reached sub-confluence (75-85 percent confluence) in 4-5 days. For these subsequent passages, cells were seeded at 5,000 cells/cm². Cells were grown in a humidified incubator with 5 percent carbon dioxide at 37°C.

In some experiments, cells were isolated from postpartum tissues in DMEM-low glucose medium after digestion with LIBERASE (2.5 milligrams per milliliter, Blendzyme 3; Roche Applied Sciences, Indianapolis, IN) and hyaluronidase (5 Units/milliliter, Sigma). Digestion of the tissue and isolation of the cells was as described for other protease digestions above, however, the LIBERASE/hyaluronidase mixture was used instead of the C:D or C:D:H enzyme mixture. Tissue digestion with LIBERASE resulted in the isolation of cell populations from postpartum tissues that expanded readily.

Procedures were compared for isolating cells from the umbilical cord using differing enzyme combinations. Enzymes compared for digestion included: collagenase; dispase; hyaluronidase; collagenase:dispase mixture (C:D); collagenase:hyaluronidase mixture (C:H); dispase:hyaluronidase mixture (D:H); and collagenase:dispase:hyaluronidase mixture (C:D:H). Differences in cell isolation utilizing these different enzyme digestion conditions were observed (Table 3-1).

Other attempts were made to isolate pools of cells from umbilical cord by different approaches. In one instance umbilical cord was sliced and washed with growth medium to dislodge the blood clots and gelatinous material. The mixture of blood, gelatinous material and growth medium was collected and centrifuged at 150 x g. The pellet was resuspended and seeded onto gelatin-coated flasks in growth medium. From these experiments, a cell population was isolated that readily expanded.

Cells have also been isolated from cord blood samples obtained from NDRI. The isolation protocol used was that of International Patent Application PCT/US2002/029971 by Ho et al. Samples (50 milliliter and 10.5 milliliters, respectively) of umbilical cord blood (NDRI, Philadelphia PA) were mixed with lysis buffer (filter-sterilized 155 millimolar ammonium chloride, 10 millimolar potassium bicarbonate, 0.1 millimolar EDTA buffered to pH 7.2 (all components from Sigma, St. Louis, MO)). Cells were lysed at a ratio of 1:20 cord blood to lysis buffer. The resulting cell suspension was vortexed for 5 seconds, and incubated for 2 minutes at ambient temperature. The lysate was centrifuged (10 minutes at 200 x g). The cell pellet was resuspended in Complete Minimal Essential Medium (Gibco, Carlsbad CA) containing 10 percent fetal bovine serum (Hyclone, Logan UT), 4 millimolar glutamine (Mediatech Herndon, VA) penicillin at 100 Units per milliliter and streptomycin at 100 micrograms per milliliter (Gibco, Carlsbad, CA). The resuspended cells were centrifuged (10 minutes at 200 x g), the supernatant was aspirated, and the cell pellet was washed in complete medium. Cells were seeded directly into either T75 flasks (Corning, NY), T75 laminin-coated flasks, or T175 fibronectin-coated flasks (both Becton Dickinson, Bedford, MA).

To determine whether cell populations could be isolated under different conditions and expanded under a variety of conditions immediately after isolation, cells were digested in growth medium with or without 0.001 percent (v/v) 2-mercaptoethanol (Sigma, St. Louis, MO), using the enzyme combination of C:D:H, according to the procedures provided above. All cells were grown in the presence of penicillin at 100 Units per milliliter and streptomycin at 100 micrograms per milliliter. Under all tested conditions, cells attached and expanded well between passage 0 and 1 (Table 2-2). Cells in conditions 5-8 and 13-16 were demonstrated to proliferate well up to 4 passages after seeding at which point they were cryopreserved.

The combination of C:D:H, provided the best cell yield following isolation, and generated cells that expanded for many more generations in culture than the other conditions (Table 3-1). An expandable cell population was not attained using collagenase or hyaluronidase alone. No attempt was made to determine if this result is specific to the collagenase that was tested.

| **Table 3-1:** Isolation of cells from umbilical cord tissue using varying enzyme combinations | | |
|---|---|---|
| **Enzyme Digest** | **Cells Isolated** | **Cell Expansion** |
| Collagenase | X | X |
| Dispase | + (>10 h) | + |
| Hyaluronidase | X | X |
| Collagenase:Dispase | ++ (< 3 h) | ++ |
| Collagenase:Hyaluronidase | ++ (< 3 h) | + |
| Dispase:Hyaluronidase | + (>10 h) | + |
| Collagenase:Dispase:Hyaluronidase | +++ (< 3 h) | +++ |
| Key: + = good, ++ = very good, +++ = excellent, X = no success | | |

Cells attached and expanded well between passage 0 and 1 under all conditions tested for enzyme digestion and growth (Table 3-2). Cells in experimental conditions 5-8 and 13-16 proliferated well up to 4 passages after seeding, at which point they were cryopreserved. All cells were cryopreserved for further analysis.

| **Table 3-2:** Isolation and culture expansion of postpartum cells under varying conditions: | | | | | | |
|---|---|---|---|---|---|---|
| **Condition** | **Medium** | **15% FBS** | **BME** | **Gelatin** | **20% O₂** | **Growth Factors** |
| 1 | DMEM-Lg | Y | Y | Y | Y | N |
| 2 | DMEM-Lg | Y | Y | Y | N (5%) | N |
| 3 | DMEM-Lg | Y | Y | N | Y | N |
| 4 | DMEM-Lg | Y | Y | N | N (5%) | N |
| 5 | DMEM-Lg | N (2%) | Y | N (Laminin) | Y | EGF/FGF (20 ng/ml) |
| 6 | DMEM-Lg | N (2%) | Y | N (Laminin) | N (5%) | EGF/FGF (20 ng/ml) |
| 7 | DMEM-Lg | N (2%) | Y | N (Fibronectin) | Y | PDGF/VEGF |
| 8 | DMEM-Lg | N (2%) | Y | N (Fibronectin) | N (5%) | PDGF/VEGF |
| 9 | DMEM-Lg | Y | N | Y | Y | N |
| 10 | DMEM-Lg | Y | N | Y | N (5%) | N |
| 11 | DMEM-Lg | Y | N | N | Y | N |
| 12 | DMEM-Lg | Y | N | N | N (5%) | N |
| 13 | DMEM-Lg | N (2%) | N | N (Laminin) | Y | EGF/FGF (20 ng/ml) |
| 14 | DMEM-Lg | N (2%) | N | N (Laminin) | N (5%) | EGF/FGF (20 ng/ml) |
| 15 | DMEM-Lg | N (2%) | N | N (Fibronectin) | Y | PDGF/VEGF |
| 16 | DMEM-Lg | N (2%) | N | N (Fibronectin) | N (5%) | PDGF/VEGF |

Nucleated cells attached and grew rapidly. These cells were analyzed by flow cytometry and were similar to cells obtained by enzyme digestion.

The preparations contained red blood cells and platelets. No nucleated cells attached and divided during the first 3 weeks. The medium was changed 3 weeks after seeding and no cells were observed to attach and grow.

Populations of cells could be isolated from umbilical tissue efficiently using the enzyme combination collagenase (a metalloprotease), dispase (neutral protease) and hyaluronidase (mucolytic enzyme which breaks down hyaluronic acid). LIBERASE, which is a blend of collagenase and a neutral protease, may also be used. Blendzyme 3, which is collagenase (4 Wunsch units/gram) and thermolysin (1714 casein Units/gram), was also used together with hyaluronidase to isolate cells. These cells expanded readily over many passages when cultured in growth expansion medium on gelatin coated plastic.

Cells were also isolated from residual blood in the cords, but not cord blood. The presence of cells in blood clots washed from the tissue, which adhere and grow under the conditions used, may be due to cells being released during the dissection process.

### EXAMPLE 4

### Growth Characteristics of Cells

The cell expansion potential of umbilicus-derived cells was compared to other populations of isolated stem cells. The process of cell expansion to senescence is referred to as Hayflick's limit (Hayflick L., J. Am. Geriatr. Soc., 1974; 22(1):1-12; Hayflick L., Gerontologist, 1974; 14(1):37-45).

Tissue culture plastic flasks were coated by adding 20 milliliters 2% (w/v) gelatin (Type B: 225 Bloom; Sigma, St Louis, MO) to a T75 flask (Corning Inc., Corning, NY) for 20 minutes at room temperature. After removing the gelatin solution, 10 milliliters phosphate-buffered saline (PBS) (Invitrogen, Carlsbad, CA) was added and then aspirated.

For comparison of growth expansion potential the following cell populations were utilized; i) mesenchymal stem cells (MSC; Cambrex, Walkersville, MD); ii) adipose-derived cells (U.S. Patent No. 6,555,374; U.S. Pub. App. No. 2004/0058412); iii) normal dermal skin fibroblasts (cc-2509 lot # 9F0844; Cambrex, Walkersville, MD); and iv) umbilicus-derived cells. Cells were initially seeded at 5,000 cells/cm² on gelatin-coated T75 flasks in growth medium. For subsequent passages, cell cultures were treated as follows. After trypsinization, viable cells were counted after trypan blue staining. Cell suspension (50 microliters) was combined with trypan blue (50 microliters, Sigma, St. Louis MO). Viable cell numbers were estimated using a hemocytometer.

Following counting, cells were seeded at 5,000 cells/cm² onto gelatin-coated T 75 flasks in 25 milliliters of fresh growth medium. Cells were grown in a standard atmosphere (5 percent carbon dioxide (v/v)) at 37 °C. The growth medium was changed twice per week. When cells reached about 85 percent confluence they were passaged; this process was repeated until the cells reached senescence.

At each passage, cells were trypsinized and counted. The viable cell yield, population doublings [In (cells final/cells initial)/ln2], and doubling time (time in culture/population doubling) were calculated. For the purposes of determining optimal cell expansion, the total cell yield per passage was determined by multiplying the total yield for the previous passage by the expansion factor for each passage (*i.e*., expansion factor = cells final/cells initial).

The expansion potential of cells banked at passage 10 was also tested. A different set of conditions was used. Normal dermal skin fibroblasts (cc-2509 lot # 9F0844; Cambrex, Walkersville, MD) and umbilicus-derived cells were tested. These cell populations had been banked at passage 10 previously, having been cultured at 5,000 cells/cm² at each passage to that point. The effect of cell density on the cell populations following cell thaw at passage 10 was determined. Cells were thawed under standard conditions and counted using trypan blue staining. Thawed cells were then seeded at 1,000 cells/cm² in growth medium. Cells were grown under standard atmospheric conditions at 37 °C. Growth medium was changed twice a week. Cells were passaged as they reached about 85% confluence. The cells were subsequently passaged until senescence, *i.e*., until they could not be expanded any further. Cells were trypsinized and counted at each passage. The cell yield, population doubling (In (cells final/cells initial)/ln2) and doubling time (time in culture)/population doubling) were calculated. The total cell yield per passage was determined by multiplying total yield for the previous passage by the expansion factor for each passage (*i.e*., expansion factor = cells final/cells initial).

The expansion potential of freshly isolated umbilicus-derived cell cultures under low cell seeding conditions was tested in another experiment. Umbilicus-derived cells were isolated as described in a previous example. The cells were seeded at 1,000 cells/cm² and passaged as described above until senescence. The cells were grown under standard atmospheric conditions at 37 °C. Growth Medium was changed twice per week. The cells were passaged as they reached about 85% confluence. At each passage, cells were trypsinized and counted by trypan blue staining. The cell yield, population doubling (In (cell final/cell initial)/ln 2) and doubling time (time in culture/population doubling) were calculated for each passage. The total cell yield per passage was determined by multiplying the total yield for the previous passage by the expansion factor for each passage (*i.e*., expansion factor = cell final/cell initial). Cells were grown on gelatin and non-gelatin coated flasks.

It has been demonstrated that low O₂ cell culture conditions can improve cell expansion in certain circumstances (U.S. Pub. App. No. 2004/0005704). In order to determine if cell expansion of umbilicus-derived cells could be improved by altering cell culture conditions, cultures of umbilicus-derived cells were grown in low oxygen conditions. The cells were seeded at 5,000 cells/cm² in growth medium on gelatin coated flasks. The cells were initially cultured under standard atmospheric conditions through passage 5, at which point they were transferred to low oxygen (5% O₂) culture conditions.

In other experiments cells were expanded on non-coated, collagen-coated, fibronectin-coated, laminin-coated and matrigel-coated plates. Cultures have been demonstrated to expand well on these different matrices.

Umbilicus-derived cells expanded for more than 40 passages generating cell yields of > 1E17 cells in 60 days. In contrast, MSCs and fibroblasts senesced after < 25 days and <60 days, respectively. Although both adipose-derived and omental cells expanded for almost 60 days they generated total cell yields of 4.5E12 and 4.24E13 respectively. Thus, when seeded at 5,000 cells/cm² under the experimental conditions utilized, umbilicus-derived cells expanded much better than the other cell types grown under the same conditions (Table 4-1).

| **Table 4-1:** Growth characteristics for different cell populations grown to senescence | | | |
|---|---|---|---|
| **Cell Type** | **Senescence** | **Total Population Doublings** | **Yield (Total Cells)** |
| MSC | 24 d | 8 | 4.72 E7 |
| Adipose-derived cell | 57 d | 24 | 4.50 E12 |
| Fibroblasts | 53 d | 26 | 2.82 E13 |
| Umbilical | 65 d | 42 | 6.15 E17 |

Umbilicus-derived and fibroblast cells expanded for greater than 10 passages generating cell yields of > 1E11 cells in 60 days (Table 4-2). Under these conditions both the fibroblasts and the umbilicus-derived cell populations senesced after 80 days, completing >50 and > 40 population doublings respectively.

| **Table 4-2:** Growth characteristics for different cell populations using low density growth expansion from passage 10 through senescence | | | |
|---|---|---|---|
| **Cell Type (Passage No.)** | **Senescence** | **Total Population Doublings** | **Yield (Total Cells)** |
| Fibroblast (P10) | 80 days | 43.68 | 2.59 E11 |
| Umbilical (P10) | 80 days | 53.6 | 1.25 E14 |

Cells expanded well under the reduced oxygen conditions; however, culturing under low oxygen conditions does not appear to have a significant effect on cell expansion for postpartum-derived cells. These results are preliminary in the sense that any ultimate conclusions to be made regarding the effect of reduced oxygen would best be drawn from experiments on growing cells in low oxygen from initial isolation. Standard atmospheric conditions have already proven successful for growing sufficient numbers of cells, and low oxygen culture is not required for the growth of postpartum-derived cells.

The current cell expansion conditions of growing isolated umbilicus-derived cells at densities of about 5,000 cells/cm², in growth medium on gelatin-coated or uncoated flasks, under standard atmospheric oxygen, are sufficient to generate large numbers of cells at passage 11. Furthermore, the data suggests that the cells can be readily expanded using lower density culture conditions (*e.g*. 1,000 cells/cm²). Umbilicus-derived cell expansion in low oxygen conditions also facilitates cell expansion, although no incremental improvement in cell expansion potential has yet been observed when utilizing these conditions for growth. Presently, culturing umbilicus-derived cells under standard atmospheric conditions is preferred for generating large pools of cells. However, when the culture conditions are altered, umbilicus-derived cell expansion can likewise be altered. This strategy may be used to enhance the proliferative and differentiative capacity of these cell populations.

Under the conditions utilized, while the expansion potential of MSC and adipose-derived cells is limited, umbilicus-derived cells expand readily to large numbers.

### EXAMPLE 5

### Growth of Cells in Medium Containing D-Valine

It has been reported that medium containing D-valine instead of the normal L-valine isoform can be used to selectively inhibit the growth of fibroblast-like cells in culture (Hongpaisan, J. Cell Biol. Int., 2000; 24:1-7; Sordillo et al., Cell Biol. Int. Rep., 1988; 12:355-64). Experiments were performed to determine whether umbilicus-derived cells could grow in medium containing D-valine.

Umbilicus-derived cells (P5) and fibroblasts (P9) were seeded at 5,000 cells/cm² in gelatin-coated T75 flasks (Corning, Corning, NY). After 24 hours the medium was removed and the cells were washed with phosphate buffered saline (PBS) (Gibco, Carlsbad, CA) to remove residual medium. The medium was replaced with a modified growth medium (DMEM with D-valine (special order Gibco), 15% (v/v) dialyzed fetal bovine serum (Hyclone, Logan, UT), 0.001% (v/v) betamercaptoethanol (Sigma), penicillin at 50 Units/milliliter and streptomycin at 50 milligrams/milliliter (Gibco)).

Umbilicus-derived cells and fibroblast cells seeded in the D-valine-containing medium did not proliferate, unlike cells seeded in growth medium containing dialyzed serum. Fibroblasts cells changed morphologically, increasing in size and changing shape. All of the cells died and eventually detached from the flask surface after four weeks. Thus, it may be concluded that umbilical cord tissue-derived cells require L-valine for cell growth and to maintain long-term viability. L-valine is preferably not removed from the growth medium for umbilical cord tissue-derived cells.

### EXAMPLE 6

### Karyotype Analysis of Cells

Cell lines used in cell therapy are preferably homogeneous and free from any contaminating cell type. Human cells used in cell therapy should have a normal number (46) of chromosomes with normal structure. To identify umbilicus-derived cell lines that are homogeneous and free from cells of non-umbilical tissue origin, karyotypes of cell samples were analyzed.

UTC from postpartum tissue of a male neonate were cultured in Growth Media. Postpartum tissue from a male neonate (X,Y) was selected to allow distinction between neonatal-derived cells and maternal derived cells (X,X). Cells were seeded at 5,000 cells per square centimeter in growth medium in a T25 flask (Corning, Corning, NY) and expanded to 80% confluence. A T25 flask containing cells was filled to the neck with Growth Media. Samples were delivered to a clinical cytogenetics lab by courier (estimated lab to lab transport time is one hour). Chromosome analysis was performed by the Center for Human & Molecular Genetics at the New Jersey Medical School, Newark, NJ. Cells were analyzed during metaphase when the chromosomes are best visualized. Of twenty cells in metaphase counted, five were analyzed for normal homogeneous karyotype number (two). A cell sample was characterized as homogeneous if two karyotypes were observed. A cell sample was characterized as heterogeneous if more than two karyotypes were observed. Additional metaphase cells were counted and analyzed when a heterogeneous karyotype number (four) was identified.

All cell samples sent for chromosome analysis were interpreted by the cytogenetics laboratory staff as exhibiting a normal appearance. Three of sixteen cell clines analyzed exhibited a heterogenous phenotype (XX and XY) indicating the presence of cells derived from both neonatal and maternal origins (Table 6-1). Each of the cell samples was characterized as homogeneous (Table 6-1).

| **Table 6-1.** Karyotype results of UTC. | | | | | |
|---|---|---|---|---|---|
| **Tissue** | **Passage** | **Metaphase cells counted** | **Metaphase cells analyzed** | **Number of karyotypes** | **ISCN Karyotype** |
| Umbilical | 23 | 20 | 5 | 2 | 46, XX |
| Umbilical | 6 | 20 | 5 | 2 | 46, XY |
| Umbilical | 3 | 20 | 5 | 2 | 46, XX |

Chromosome analysis identified umbilicus-derived UTC whose karyotypes appear normal as interpreted by a clinical cytogenetic laboratory. Karyotype analysis also identified cell lines free from maternal cells, as determined by homogeneous karyotype.

### EXAMPLE 7

### Flow Cytometric Evaluation of Cell Surface Markers

Characterization of cell surface proteins or "markers" by flow cytometry can be used to determine a cell line's identity. The consistency of expression can be determined from multiple donors, and in cells exposed to different processing and culturing conditions. Postpartum cell lines isolated from the umbilicus were characterized by flow cytometry, providing a profile for the identification of these cell lines.

Cells were cultured in growth medium, in plasma-treated T75, T150, and T225 tissue culture flasks (Corning, Corning, NY) until confluent. The growth surfaces of the flasks were coated with gelatin by incubating 2% (w/v) gelatin (Sigma, St. Louis, MO) for 20 minutes at room temperature.

Adherent cells in flasks were washed in phosphate buffered saline (PBS); (Gibco, Carlsbad, MO) and detached with trypsin/EDTA (Gibco). Cells were harvested, centrifuged, and resuspended in 3% (v/v) FBS in PBS at a cell concentration of 1x10⁷ per milliliter. In accordance with the manufacture's specifications, antibody to the cell surface marker of interest (see below) was added to 100 microliters of cell suspension and the mixture was incubated in the dark for 30 minutes at 4 °C. After incubation, cells were washed with PBS and centrifuged to remove unbound antibody. Cells were resuspended in 500 microliters PBS and analyzed by flow cytometry. Flow cytometry analysis was performed with a FACScalibur instrument (Becton Dickinson, San Jose, CA). The following antibodies to cell surface markers were used (*see* Table 7-1).

| **Table 7-1: Antibodies used in characterizing cell surface markers of UDCs.** | | |
|---|---|---|
| **Antibody** | **Manufacture** | **Catalog Number** |
| CD10 | BD Pharmingen (San Diego, CA) | 555375 |
| CD13 | BD Pharmingen | 555394 |
| CD31 | BD Pharmingen | 555446 |
| CD34 | BD Pharmingen | 555821 |
| CD44 | BD Pharmingen | 555478 |
| CD45RA | BD Pharmingen | 555489 |
| CD73 | BD Pharmingen | 550257 |
| CD90 | BD Pharmingen | 555596 |
| CD117 | BD Pharmingen | 340529 |
| CD141 | BD Pharmingen | 559781 |
| PDGFr-alpha | BD Pharmingen | 556002 |
| HLA-A, B, C | BD Pharmingen | 555553 |
| HLA-DR, DP, DQ | BD Pharmingen | 555558 |
| IgG-FITC | Sigma (St. Louis, MO) | F-6522 |
| IgG- PE | Sigma | P-4685 |

Umbilicus-derived cells were analyzed at passages 8, 15, and 20. To compare differences among donors, umbilical from different donors were compared to each other. Umbilicus-derived cells cultured on gelatin-coated flasks were compared to umbilicus cultured on uncoated flasks.

Four treatments used for isolation and preparation of cells were compared. Cells derived from postpartum tissue by treatment with 1) collagenase; 2) collagenase/dispase; 3) collagenase/hyaluronidase; and 4) collagenase/hyaluronidase/dispase were compared.

Umbilical cord-derived cells at passage 8, 15, and 20 analyzed by flow cytometry all expressed CD10, CD13, CD44, CD73, CD 90, PDGFr-alpha and HLA-A, B, C, indicated by increased fluorescence relative to the IgG control. These cells were negative for CD31, CD34, CD45, CD117, CD141, and HLA-DR, DP, DQ, indicated by fluorescence values consistent with the IgG control.

Umbilical cord-derived cells isolated from separate donors analyzed by flow cytometry each showed positive for the production of CD10, CD13, CD44, CD73, CD 90, PDGFr-alpha and HLA-A, B, C, reflected in the increased values of fluorescence relative to the IgG control. These cells were negative for the production of CD31, CD34, CD45, CD117, CD141, and HLA-DR, DP, DQ with fluorescence values consistent with the IgG control.

The umbilical cord-derived cells expanded on gelatin and uncoated flasks analyzed by flow cytometry were all positive for the production of CD10, CD13, CD44, CD73, CD 90, PDGFr-alpha and HLA-A, B, C, with increased values of fluorescence relative to the IgG control. These cells were negative for the production of CD31, CD34, CD45, CD117, CD141, and HLA-DR, DP, DQ, with fluorescence values consistent with the IgG control.

Analysis of umbilical cord-derived by flow cytometry has established of an identity of these cell lines. Umbilical cord-derived cells are positive for CD10, CD13, CD44, CD73, CD90, PDGFr-alpha, HLA-A,B,C and negative for CD31, CD34, CD45, CD117, CD141 and HLA-DR, DP, DQ. This identity was consistent between variations in variables including the donor, passage, culture vessel surface coating, digestion enzymes, and placental layer. Some variation in individual fluorescence value histogram curve means and ranges were observed, but all positive curves under all conditions tested were normal and expressed fluorescence values greater than the IgG control, thus confirming that the cells comprise a homogeneous population which has positive expression of the markers.

### EXAMPLE 8

### Analysis of Cells by Oligonucleotide Array

Oligonucleotide arrays were used to compare gene expression profiles of umbilicus-derived and placenta-derived cells with fibroblasts, human mesenchymal stem cells, and another cell line derived from human bone marrow. This analysis provided a characterization of the postpartum-derived cells and identified unique molecular markers for these cells.

*Postpartum tissue-derived cells.* Human umbilical cords and placenta were obtained from National Disease Research Interchange (NDRI, Philadelphia, PA) from normal full term deliveries with patient consent. The tissues were received and cells were isolated as described in Example 6 after digestion with a C:D:H mixtures. Cells were cultured in growth medium on gelatin-coated plastic tissue culture flasks. The cultures were incubated at 37° C with 5% CO₂.

*Fibroblasts.* Human dermal fibroblasts were purchased from Cambrex Incorporated (Walkersville, MD; Lot number 9F0844) and ATCC CRL-1501 (CCD39SK). Both lines were cultured in DMEM/F12 medium (Invitrogen, Carlsbad, CA) with 10% (v/v) fetal bovine serum (Hyclone) and penicillin/streptomycin (Invitrogen)). The cells were grown on standard tissue-treated plastic.

*Human Mesenchymal Stem Cells (hMSC).* hMSCs were purchased from Cambrex Incorporated (Walkersville, MD; Lot numbers 2F1655, 2F1656 and 2F1657) and cultured according to the manufacturer's specifications in MSCGM Media (Cambrex). The cells were grown on standard tissue cultured plastic at 37 °C with 5% CO₂.

*Human Iliac Crest Bone Marrow Cells (ICBM).* Human iliac crest bone marrow was received from NDRI with patient consent. The marrow was processed according to the method outlined by Ho, et al. (WO 03/025149). The marrow was mixed with lysis buffer (155 mM NH₄Cl, 10 mM KHCO₃, and 0.1 mM EDTA, pH 7.2) at a ratio of 1 part bone marrow to 20 parts lysis buffer. The cell suspension was vortexed, incubated for 2 minutes at ambient temperature, and centrifuged for 10 minutes at 500 x g. The supernatant was discarded and the cell pellet was resuspended in Minimal Essential Medium-alpha (Invitrogen) supplemented with 10% (v/v) fetal bovine serum and 4 mM glutamine. The cells were centrifuged again and the cell pellet was resuspended in fresh medium. The viable mononuclear cells were counted using trypan-blue exclusion (Sigma, St. Louis, MO). The mononuclear cells were seeded in plastic tissue culture flasks at 5 x 10⁴ cells/cm². The cells were incubated at 37 °C with 5% CO₂ at either standard atmospheric O₂ or at 5% O₂. Cells were cultured for 5 days without a media change. Media and non-adherent cells were removed after 5 days of culturing. The adherent cells were maintained in culture.

Actively growing cultures of cells were removed from the flasks with a cell scraper in cold phosphate buffered saline (PBS). The cells were centrifuged for 5 minutes at 300 x g. The supernatant was removed and the cells were resuspended in fresh PBS and centrifuged again. The supernatant was removed and the cell pellet was immediately frozen and stored at - 80° C. Cellular mRNA was extracted and transcribed into cDNA. The cDNA was then transcribed into cRNA and biotin-labeled. The biotin-labeled cRNA was hybridized with Affymetrix GENECHIP HG-U133A oligonucleotide arrays (Affymetrix, Santa Clara, CA). The hybridizations and data collection were performed according to the manufacturer's specifications. Data analyses was performed using "Significance Analysis of Microarrays" (SAM) version 1.21 computer software (Tusher, V.G. et al., 2001, Proc. Natl. Acad. Sci. USA 98: 5116-5121). Licenses for the analysis software are available through the Office of Technology Licensing, Stanford University, and more information is available on the World Wide Web at Professor Tibshirani's web site in the Dep't of Statistics, Stanford University (*www-stat.stanford.edu*/*∼tibs*/*SAM*/)*.*

Fourteen different populations of cells were analyzed in this study. The cells along with passage information, culture substrate, and culture media are listed in Table 8-1. The cells lines are listed by their identification code along with passage at the time of analysis, cell growth substrate, and growth media.

| **Table 8-1.** Cells analyzed by the microarray study. | | | |
|---|---|---|---|
| **Cell Population** | **Passage** | **Substrate** | **Media** |
| Umbilical (022803) | 2 | Gelatin | DMEM, 15% FBS, βME |
| Umbilical (042103) | 3 | Gelatin | DMEM, 15% FBS, βME |
| Umbilical (071003) | 4 | Gelatin | DMEM, 15% FBS, βME |
| Placenta (042203) | 12 | Gelatin | DMEM, 15% FBS, βME |
| Placenta (042903) | 4 | Gelatin | DMEM, 15% FBS, βME |
| Placenta (071003) | 3 | Gelatin | DMEM, 15% FBS, βME |
| ICBM (070203) (5% O₂) | 3 | Plastic | MEM 10% FBS |
| ICBM (062703) (std O₂) | 5 | Plastic | MEM 10% FBS |
| ICBM (062703 )(5% O₂) | 5 | Plastic | MEM 10% FBS |
| hMSC (Lot 2F1655) | 3 | Plastic | MSCGM |
| hMSC (Lot 2F1656) | 3 | Plastic | MSCGM |
| hMSC (Lot 2F1657) | 3 | Plastic | MSCGM |
| hFibroblast (9F0844) | 9 | Plastic | DMEM-F12, 10% FBS |
| hFibroblast (CCD39SK) | 4 | Plastic | DMEM-F12, 10% FBS |

The data were evaluated by principle component analysis with SAM software as described above. The analysis revealed 290 genes that were expressed in different relative amounts in the cells tested. This analysis provided relative comparisons between the populations.

Table 8-2 shows the Euclidean distances that were calculated for the comparison of the cell pairs. The Euclidean distances were based on the comparison of the cells based on the 290 genes that were differentially expressed among the cell types. The Euclidean distance is inversely proportional to similarity between the expression of the 290 genes. The Euclidean distance was calculated for the cell types using the 290 genes that were expressed differentially between the cell types. Similarity between the cells is inversely proportional to the Euclidean distance.

| **Table 8-2. The Euclidean Distances for the Cell Pairs.** | |
|---|---|
| ***CELL PAIR*** | **Euclidean Distance** |
| **ICBM-HMSC** | 24.71 |
| **PLACENTA-UMBILICAL** | 25.52 |
| **ICBM-FIBROBLAST** | 36.44 |
| **ICBM-PLACENTA** | 37.09 |
| **FIBROBLAST-MSC** | 39.63 |
| **ICBM-UMBILICAL** | 40.15 |
| **FIBROBLAST-UMBILICAL** | 41.59 |
| **MSC-PLACENTA** | 42.84 |
| **MSC-UMBILICAL** | 46.86 |
| **ICBM-PLACENTA** | 48.41 |

Tables 8-3, 8-4, and 8-5 show the expression of genes increased in placenta-derived cells (Table 8-3), increased in umbilical cord-derived cells (Table 8-4), and reduced in umbilical cord and placenta-derived cells (Table 8-5).

| **Table 8-3. Genes which are specifically increased in expression in the placenta-derived cells as compared to the other cell lines assayed. (Genes Increased in Placenta-Derived Cells)** | | |
|---|---|---|
| **Probe Set ID** | **Gene Name** | **NCBI Accession No.** |
| 209732_at | C-type (calcium dependent, carbohydrate-recognition domain) lectin, superfamily member 2 (activation-induced) | AF070642 |
| 206067_s_at | Wilms tumor 1 | NM_024426 |
| 207016_s_at | aldehyde dehydrogenase 1 family, member A2 | AB015228 |
| 206367_at | Renin | NM_000537 |
| 210004_at | oxidized low density lipoprotein (lectin-like) receptor 1 | AF035776 |
| 214993_at | *Homo sapiens*, clone IMAGE:4179671, mRNA, partial cds | AF070642 |
| 202178_at | protein kinase C, zeta | NM_002744 |
| 209780_at | hypothetical protein DKFZp564F013 | AL136883 |
| 204135_at | downregulated in ovarian cancer 1 | NM_014890 |
| 213542_at | *Homo sapiens* mRNA; cDNA DKFZp547K1113 (from clone DKFZp547K1113) | AI246730 |

| **Table 8-4. Genes which are specifically increased in expression in umbilical cord -derived cells as compared to the other cell lines assayed. (Genes Increased in Umbilicus-Derived Cells)** | | |
|---|---|---|
| **Probe Set ID** | **Gene Name** | **NCBI Accession No.** |
| 202859_x_at | Interleukin 8 | NM_000584 |
| 211506_s_at | Interleukin 8 | AF043337 |
| 210222_s_at | reticulon 1 | BC000314 |
| 204470_at | chemokine (C-X-C motif) ligand 1 (melanoma growth stimulating activity | NM_001511 |
| 206336_at | chemokine (C-X-C motif) ligand 6 (granulocyte chemotactic protein 2) | NM_002993 |
| 207850_at | Chemokine (C-X-C motif) ligand 3 | NM_002090 |
| 203485_at | reticulon 1 | NM_021136 |
| 202644_s_at | tumor necrosis factor, alpha-induced protein 3 | NM_006290 |

| **Table 8-5. Genes which were decreased in expression in the umbilical cord and placenta cells as compared to the other cell lines assayed. (Genes Decreased in Umbilicus- and Placenta-Derived Cells)** | | |
|---|---|---|
| **Probe Se**t **ID** | **Gene name** | **NCBI Accession No.** |
| 210135_s_at | short stature homeobox 2 | AF022654.1 |
| 205824_at | heat shock 27kDa protein 2 | NM_001541.1 |
| 209687_at | chemokine (C-X-C motif) ligand 12 (stromal cell-derived factor 1) | U19495.1 |
| 203666_at | chemokine (C-X-C motif) ligand 12 (stromal cell-derived factor 1) | NM_000609.1 |
| 212670_at | elastin (supravalvular aortic stenosis, Williams-Beuren syndrome) | AA479278 |
| 213381_at | *Homo sapiens* mRNA; cDNA DKFZp586M2022 (from clone DKFZp586M2022) | N91149 |
| 206201_s_at | mesenchyme homeobox 2 (growth arrest-specific homeobox) | NM_005924.1 |
| 205817_at | Sine oculis homeobox homolog 1 (*Drosophila*) | NM_005982.1 |
| 209283_at | crystallin, alpha B | AF007162.1 |
| 212793_at | dishevelled associated activator of morphogenesis 2 | BF513244 |
| 213488_at | DKFZP586B2420 protein | AL050143.1 |
| 209763_at | similar to neuralin 1 | AL049176 |
| 205200_at | Tetranectin (plasminogen binding protein) | NM_003278.1 |
| 205743_at | src homology three (SH3) and cysteine rich domain | NM_003149.1 |
| 200921_s_at | B-cell translocation gene 1, anti-proliferative | NM_001731.1 |
| 206932_at | cholesterol 25-hydroxylase | NM_003956.1 |
| 204198_s_at | runt-related transcription factor 3 | AA541630 |
| 219747_at | hypothetical protein FLJ23191 | NM_024574.1 |
| 204773_at | Interleukin 11 receptor, alpha | NM_004512.1 |
| 202465_at | Procollagen C-endopeptidase enhancer | NM_002593.2 |
| 203706_s_at | Frizzled homolog 7 (*Drosophila*) | NM_003507.1 |
| 212736_at | hypothetical gene BC008967 | BE299456 |
| 214587_at | Collagen, type VIII, alpha 1 | BE877796 |
| 201645_at | Tenascin C (hexabrachion) | NM_002160.1 |
| 210239_at | iroquois homeobox protein 5 | U90304.1 |
| 203903_s_at | Hephaestin | NM_014799.1 |
| 205816_at | integrin, beta 8 | NM_002214.1 |
| 203069_at | synaptic vesicle glycoprotein 2 | NM_014849.1 |
| 213909_at | *Homo sapiens* cDNA FLJ12280 fis, clone MAMMA1001744 | AU147799 |
| 206315_at | cytokine receptor-like factor 1 | NM_004750.1 |
| 204401_at | potassium intermediate/small conductance calcium-activated channel, subfamily N, member 4 | NM_002250.1 |
| 216331_at | integrin, alpha 7 | AK022548.1 |
| 209663_s_at | integrin, alpha 7 | AF072132.1 |
| 213125_at | DKFZP586L151 protein | AW007573 |
| 202133_at | transcriptional co-activator with PDZ-binding motif (TAZ) | AA081084 |
| 206511_s_ at | Sine oculis homeobox homolog 2 (*Drosophila*) | NM_016932.1 |
| 213435_at | KIAA1034 protein | AB028957.1 |
| 206115_at | early growth response 3 | NM_004430.1 |
| 213707_s_at | distal-less homeobox 5 | NM_005221.3 |
| 218181_s_at | hypothetical protein FLJ20373 | NM_017792.1 |
| 209160_at | aldo-keto reductase family 1, member C3 (3-alpha hydroxysteroid dehydrogenase, type II) | AB018580.1 |
| 213905_x_at | Biglycan | AA845258 |
| 201261_x_at | Biglycan | BC002416.1 |
| 202132_at | transcriptional co-activator with PDZ-binding motif (TAZ) | AA081084 |
| 214701_s_at | fibronectin 1 | AJ276395.1 |
| 213791_at | Proenkephalin | NM_ 006211.1 |
| 205422_s_at | Integrin, beta-like 1 (with EGF-like repeat domains) | NM_004791.1 |
| 214927_at | *Homo sapiens* mRNA full length insert cDNA clone EUROIMAGE 1968422 | AL359052.1 |
| 206070_s_at | EphA3 | AF213459.1 |
| 212805_at | KIAA0367 protein | AB002365.1 |
| 219789_at | natriuretic peptide receptor C/guanylate cyclase C (atrionatriuretic peptide receptor C) | AI628360 |
| 219054_at | hypothetical protein FLJ14054 | NM_024563.1 |
| 213429_at | *Homo sapiens* mRNA; cDNA DKFZp564B222 (from clone DKFZp564B222) | AW025579 |
| 204929_s_at | vesicle-associated membrane protein 5 (myobrevin) | NM_006634.1 |
| 201843_s_at | EGF-containing fibulin-like extracellular matrix protein 1 | NM_004105.2 |
| 221478_at | BCL2/adenovirus E1B 19kDa interacting protein 3-like | AL132665.1 |
| 201792_at | AE binding protein 1 | NM_001129.2 |
| 204570_at | cytochrome c oxidase subunit VIIa polypeptide 1 (muscle) | NM_001864.1 |
| 201621_at | neuroblastoma, suppression of tumorigenicity 1 | NM_005380.1 |
| 202718_at | Insulin-like growth factor binding protein 2, 36kDa | NM_000597.1 |

Tables 8-6, 8-7, and 8-8 show the expression of genes increased in human fibroblasts (Table 8-6), ICBM cells (Table 8-7), and MSCs (Table 8-8).

| **Table 8-6. Genes which were increased in expression in fibroblasts as compared to the other cell lines assayed. (Genes increased in fibroblasts)** |
|---|
| dual specificity phosphatase 2 |
| KIAA0527 protein |
| *Homo sapiens* cDNA: FLJ23224 fis, clone ADSU02206 |
| dynein, cytoplasmic, intermediate polypeptide 1 |
| ankyrin 3, node of Ranvier (ankyrin G) |
| inhibin, beta A (activin A, activin AB alpha polypeptide) |
| ectonucleotide pyrophosphatase/phosphodiesterase 4 (putative function) |
| KIAA1053 protein |
| microtubule-associated protein 1A |
| zinc finger protein 41 |
| HSPC019 protein |
| *Homo sapiens* cDNA: FLJ23564 fis, clone LNG10773 |
| *Homo sapiens* mRNA; cDNA DKFZp564A072 (from clone DKFZp564A072) |
| LIM protein (similar to rat protein kinase C-binding enigma) |
| inhibitor of kappa light polypeptide gene enhancer in B-cells, kinase complex-associated protein |
| hypothetical protein FLJ22004 |
| Human (clone CTG-A4) mRNA sequence |
| ESTs, Moderately similar to cytokine receptor-like factor 2; cytokine receptor CRL2 precursor [*Homo sapiens*] |
| transforming growth factor, beta 2 |
| hypothetical protein MGC29643 |
| antigen identified by monoclonal antibody MRC OX-2 |
| putative X-linked retinopathy protein |

| **Table 8-7. Genes which were increased in expression in the ICBM-derived cells as compared to the other cell lines assayed.** |
|---|
| **Genes Increased In ICBM Cells** |
| •cardiac ankyrin repeat protein |
| •MHC class I region ORF |
| •integrin, alpha 10 |
| •hypothetical protein FLJ22362 |
| •UDP-N-acetyl-alpha-D-galactosamine:polypeptide N-acetylgalactosaminyltransferase 3 (GalNAc-T3) |
| •interferon-induced protein 44 |
| •SRY (sex determining region Y)-box 9 (campomelic dysplasia, autosomal sex-reversal) |
| •keratin associated protein 1-1 |
| •hippocalcin-like 1 |
| •jagged 1 (Alagille syndrome) |
| •proteoglycan 1, secretory granule |

| **Table 8-8. Genes which were increased in expression in the MSC cells as compared to the other cell lines assayed. (Genes Increased in MSC Cells)** |
|---|
| •interleukin 26 |
| •maltase-glucoamylase (alpha-glucosidase) |
| •nuclear receptor subfamily 4, group A, member 2 |
| •v-fos FBJ murine osteosarcoma viral oncogene homolog |
| •hypothetical protein DC42 |
| •nuclear receptor subfamily 4, group A, member 2 |
| •FBJ murine osteosarcoma viral oncogene homolog B |
| •WNT1 inducible signaling pathway protein 1 |
| •MCF.2 cell line derived transforming sequence |
| •potassium channel, subfamily K, member 15 |
| •cartilage paired-class homeoprotein 1 |
| •*Homo sapiens* cDNA FLJ12232 fis, clone MAMMA1001206 |
| •*Homo sapiens* cDNA FLJ34668 fis, clone LIVER2000775 |
| •jun B proto-oncogene |
| •B-cell CLL/lymphoma 6 (zinc finger protein 51) |
| •zinc finger protein 36, C3H type, homolog (mouse) |

The present example was performed to provide a molecular characterization of the cells derived from umbilical cord and placenta. This analysis included cells derived from three different umbilical cords and three different placentas. The study also included two different lines of dermal fibroblasts, three lines of mesenchymal stem cells, and three lines of iliac crest bone marrow cells. The mRNA that was expressed by these cells was analyzed on a GENECHIP oligonucleotide array that contained oligonucleotide probes for 22,000 genes.

The analysis revealed that transcripts for 290 genes were present in different amounts in these five different cell types. These genes include ten genes that are specifically increased in the placenta-derived cells and seven genes specifically increased in the umbilical cord-derived cells. Fifty-four genes were found to have specifically lower expression levels in placenta-derived and umbilical cord tissue-derived cells.

The expression of selected genes has been confirmed by PCR, as shown in Example 9. Postpartum-derived cells generally, and umbilical derived cells, in particular, have distinct gene expression profiles, for example, as compared to other human cells, such as the bone marrow-derived cells and fibroblasts tested here.

### EXAMPLE 9

### Cell Markers

Gene expression profiles of cells derived from the human umbilical cord and human placenta were compared with those of cells derived from other sources using an Affymetrix GENECHIP. Six "signature" genes were identified: oxidized LDL receptor 1, interleukin-8 (IL-8), renin, reticulon, chemokine receptor ligand 3 (CXC ligand 3), and granulocyte chemotactic protein 2 (GCP-2). These "signature" genes were expressed at relatively high levels in umbilicus-derived cells.

The procedures described in this example were conducted to verify the microarray data and compare data for gene and protein expression, as well as to establish a series of reliable assays for detection of unique identifiers for umbilicus-derived cells.

Umbilicus-derived cells (four isolates), placenta-derived cells (three isolates, including one isolate predominately neonatal as identified by karyotyping analysis) and Normal Human Dermal Fibroblasts (NHDF; neonatal and adult) were grown in growth medium in gelatin-coated T75 flasks. Mesenchymal Stem Cells (MSCs) were grown in Mesenchymal Stem Cell Growth Medium Bullet kit (MSCGM; Cambrex, Walkersville, MD).

For IL-8 experiments, cells were thawed from liquid nitrogen and plated in gelatin-coated flasks at 5,000 cells/cm², grown for 48 hours in growth medium and then grown further for 8 hours in 10 milliliters of serum starvation medium [DMEM -low glucose (Gibco, Carlsbad, CA), penicillin (50 Units/milliliter), streptomycin (50 micrograms/milliliter)(Gibco) and 0.1% (w/v) Bovine Serum Albumin (BSA; Sigma, St. Louis, MO)]. RNA was then extracted and the supernatants were centrifuged at 150 x g for 5 minutes to remove cellular debris. Supernatants were frozen at -80°C until ELISA analysis.

Umbilical cord tissue-derived cells, placental tissue-derived cells as well as human fibroblasts derived from human neonatal foreskin, were cultured in growth medium in gelatin-coated T75 flasks. The cells were frozen at passage 11 in liquid nitrogen. The cells were thawed and transferred to 15 milliliter centrifuge tubes. After centrifugation at 150 x g for 5 minutes, the supernatant was discarded. The cells were resuspended in 4 milliliters culture medium and counted. Cells were grown in a 75 cm² flask containing 15 milliliters of growth medium at 375,000 cell/flask for 24 hours. The medium was changed to a serum starvation medium for 8 hours. Serum starvation medium was collected at the end of incubation, centrifuged at 14,000 x g for 5 minutes (and stored at -20°C).

To estimate the number of cells in each flask, 2 milliliters of trypsin/EDTA (Gibco, Carlsbad, CA) were added each flask. After cells detached from the flask, trypsin activity was neutralized with 8 milliliters of growth medium. The cells were transferred to a 15 milliliter centrifuge tube and centrifuged at 150 x g for 5 minutes. The supernatant was removed and 1 milliliter growth medium was added to each tube to resuspend the cells. The cell number was determined with a hemocytometer.

The amount of IL-8 secreted by the cells into serum starvation medium was analyzed using ELISA assays (R&D Systems, Minneapolis, MN). All assays were conducted according to the instructions provided by the manufacturer.

RNA was extracted from confluent umbilical cord-derived cells and fibroblasts, or for IL-8 expression, from cells treated as described above. Cells were lysed with 350 microliters buffer RLT containing beta-mercaptoethanol (Sigma, St. Louis, MO) according to the manufacturer's instructions (RNeasy® Mini Kit; Qiagen, Valencia, CA). RNA was extracted according to the manufacturer's instructions (RNeasy Mini Kit; Qiagen, Valencia, CA) and subjected to DNase treatment (2.7 Units/sample) (Sigma St. Louis, MO). RNA was eluted with 50 microliters DEPC-treated water and stored at -80°C. RNA was also extracted from human umbilical cord. Tissue (30 milligrams) was suspended in 700 microliters of buffer RLT containing beta-mercaptoethanol. Samples were mechanically homogenized and the RNA extraction proceeded according to manufacturer's specification. RNA was extracted with 50 microliters of DEPC-treated water and stored at -80 °C.

RNA was reverse-transcribed using random hexamers with the TaqMan® reverse transcription reagents (Applied Biosystems, Foster City, CA) at 25 °C for 10 minutes, 37 °C for 60 minutes, and 95 °C for 10 minutes. Samples were stored at -20 °C.

Genes identified by cDNA microarray as uniquely regulated in umbilical cord cells and placental cells (signature genes - including oxidized LDL receptor, interleukin-8, renin, and reticulon), were further investigated using real-time and conventional PCR.

PCR was performed on cDNA samples using gene expression products sold under the tradename ASSAYS-ON-DEMAND (Applied Biosystems) gene expression products. Oxidized LDL receptor (Hs00234028); renin (Hs00166915); reticulon (Hs00382515); CXC ligand 3 (Hs00171061); GCP-2 (Hs00605742); IL-8 (Hs00174103); and GAPDH were mixed with cDNA and TaqMan® Universal PCR master mix according to the manufacturer's instructions (Applied Biosystems) using a 7000 sequence detection system with ABI Prism 7000 SDS software (Applied Biosystems). Thermal cycle conditions were initially 50 °C for 2 minutes and 95 °C for 10 minutes, followed by 40 cycles of 95 °C for 15 seconds and 60 °C for 1 minute. PCR data were analyzed according to manufacturer's specifications (User Bulletin #2 from Applied Biosystems for ABI Prism 7700 Sequence Detection System).

Conventional PCR was performed using an ABI PRISM 7700 (Perkin Elmer Applied Biosystems, Boston, MA) to confirm the results from real-time PCR. PCR was performed using 2 microliters of cDNA solution (1× Taq polymerase (tradename AMPLITAQ GOLD) universal mix PCR reaction buffer (Applied Biosystems) and initial denaturation at 94°C for 5 minutes. Amplification was optimized for each primer set. For IL-8, CXC ligand 3, and reticulon (94 °C for 15 seconds, 55 °C for 15 seconds and 72 °C for 30 seconds for 30 cycles); for renin (94 °C for 15 seconds, 53 °C for 15 seconds and 72 °C for 30 seconds for 38 cycles); for oxidized LDL receptor and GAPDH (94 °C for 15 seconds, 55 °C for 15 seconds and 72 °C for 30 seconds for 33 cycles). Primers used for amplification are listed in Table 9-1. Primer concentration in the final PCR reaction was 1 micromolar except for GAPDH which was 0.5 micromolar. GAPDH primers were the same as for real-time PCR, except that the manufacturer's TaqMan® probe was not added to the final PCR reaction. Samples were separated on 2% (w/v) agarose gel and stained with ethidium bromide (Sigma, St. Louis, MO). Images were captured on 667 film (Universal Twinpack, VWR International, South Plainfield, NJ) using a fixed focal-length POLAROID camera (VWR International, South Plainfield, NJ).

**Table 9-1: Primers used**

| Primer name | Primers | | |
|---|---|---|---|
| **Oxidized LDL receptor** | S: | 5' -GAGAAATCCAAAGAGCAAATGG-3' | (SEQ ID NO:1) |
| | A: | 5'-AGAATGGAAAACTGGAATAGG-3' | (SEQ ID NO:2) |
| **Renin** | S: | 5'-TCTTCGATGCTTCGGATTCC-3' | (SEQ ID NO:3) |
| | A: | 5'-GAATTCTCGGAATCTCTGTTG-3' | (SEQ ID NO:4) |
| **Reticulon** | S: | 5'-TTACAAGCAGTGCAGAAAACC-3' | (SEQ ID NO:5) |
| | A: | 5'-AGTAAACATTGAAACCACAGCC-3' | (SEQ ID NO:6) |
| **Interleukin-8** | S: | 5' -TCTGCAGCTCTGTGTGAAGG-3' | (SEQ ID NO:7) |
| | A: | 5'-CTTCAAAAACTTCTCCACAACC-3' | (SEQ ID NO:8) |
| **Chemokine (CXC) ligand 3** | S: | 5'-CCCACGCCACGCTCTCC-3' | (SEQ ID NO:9) |
| | A: | 5'-TCCTGTCAGTTGGTGCTCC -3' | (SEQ ID NO:10) |

Umbilical cord-derived cells and placental tissue-derived cells were fixed with cold 4% (w/v) paraformaldehyde (Sigma-Aldrich, St. Louis, MO) for 10 minutes at room temperature. One isolate each of umbilical cord-derived cells at passage 0 (P0) (directly after isolation) and passage 11 (P11) (two isolates of Umbilical cord-derived cells) and fibroblasts (P11) were used. Immunocytochemistry was performed using antibodies directed against the following epitopes: vimentin (1:500, Sigma, St. Louis, MO), desmin (1:150; Sigma - raised against rabbit; or 1:300; Chemicon, Temecula, CA - raised against mouse,), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, CA). In addition, the following markers were tested on passage 11 umbilical cord-derived cells: anti-human GROalpha - PE (1:100; Becton Dickinson, Franklin Lakes, NJ), anti-human GCP-2 (1:100; Santa Cruz Biotech, Santa Cruz, CA), anti-human oxidized LDL receptor 1 (ox-LDL R1; 1:100; Santa Cruz Biotech), and anti-human NOGA-A (1:100; Santa Cruz, Biotech).

Cultures were washed with phosphate-buffered saline (PBS) and exposed to a protein blocking solution containing PBS, 4% (v/v) goat serum (Chemicon, Temecula, CA), and 0.3% (v/v) Triton (Triton X-100; Sigma, St. Louis, MO) for 30 minutes to access intracellular antigens. Where the epitope of interest was located on the cell surface (CD34, ox-LDL R1), Triton X-100 was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the process. Primary antibodies, diluted in blocking solution, were then applied to the cultures for a period of 1 hour at room temperature. The primary antibody solutions were removed and the cultures were washed with PBS prior to application of secondary antibody solutions (1 hour at room temperature) containing block along with goat anti-mouse IgG - Texas Red (1:250; Molecular Probes, Eugene, OR) and/or goat anti-rabbit IgG - Alexa 488 (1:250; Molecular Probes) or donkey anti-goat IgG - FITC (1:150, Santa Cruz Biotech). Cultures were then washed and 10 micromolar DAPI (Molecular Probes) applied for 10 minutes to visualize cell nuclei.

Following immunostaining, fluorescence was visualized using an appropriate fluorescence filter on an Olympus inverted epi-fluorescent microscope (Olympus, Melville, NY). In all cases, positive staining represented fluorescence signal above control staining where the entire procedure outlined above was followed with the exception of application of a primary antibody solution (no 1° control). Representative images were captured using a digital color videocamera and Image-Pro software (Media Cybernetics, Carlsbad, CA). For triple-stained samples, each image was taken using only one emission filter at a time. Layered montages were then prepared using Adobe Photoshop software (Adobe, San Jose, CA).

Adherent cells in flasks were washed in phosphate buffered saline (PBS) (Gibco, Carlsbad, CA) and detached with Trypsin/EDTA (Gibco, Carlsbad, CA). Cells were harvested, centrifuged, and re-suspended 3% (v/v) FBS in PBS at a cell concentration of 1x10⁷/milliliter. One hundred microliter aliquots were delivered to conical tubes. Cells stained for intracellular antigens were permeabilized with Perm/Wash buffer (BD Pharmingen, San Diego, CA). Antibody was added to aliquots as per manufacturer's specifications, and the cells were incubated for in the dark for 30 minutes at 4 °C. After incubation, cells were washed with PBS and centrifuged to remove excess antibody. Cells requiring a secondary antibody were resuspended in 100 microliter of 3% FBS. Secondary antibody was added as per manufacturer's specification, and the cells were incubated in the dark for 30 minutes at 4 °C. After incubation, cells were washed with PBS and centrifuged to remove excess secondary antibody. The washed cells were resuspended in 0.5 milliliter PBS and analyzed by flow cytometry. The following antibodies were used: oxidized LDL receptor 1 (sc-5813; Santa Cruz, Biotech), GROa (555042; BD Pharmingen, Bedford, MA), Mouse IgG1 kappa, (P-4685 and M-5284; Sigma), and Donkey against Goat IgG (sc-3743; Santa Cruz, Biotech.). Flow cytometry analysis was performed with FACScalibur (Becton Dickinson San Jose, CA).

Results of real-time PCR for selected "signature" genes performed on cDNA from cells derived from human umbilical cord, human placental tissue, adult and neonatal fibroblasts, and Mesenchymal Stem Cells (MSCs) indicate that reticulon expression was higher in umbilicus-derived cells as compared to other cells. The data obtained from real-time PCR were analyzed by the ΔΔCT method and expressed on a logarithmic scale. No significant differences in the expression levels of CXC ligand 3 and GCP-2 were found between the postpartum cells and the controls. The results of real-time PCR were confirmed by conventional PCR. Sequencing of PCR products further validated these observations. No significant difference in the expression level of CXC ligand 3 was found between the postpartum cells and the controls using conventional PCR CXC ligand 3 primers listed in Table 9-1.

The expression of the cytokine, IL-8 in umbilical cord cells was elevated in both growth medium-cultured and serum-starved umbilical cord-derived cells. All real-time PCR data were validated with conventional PCR and by sequencing PCR products.

After growth in serum-free media, the conditioned media were examined for the presence of IL-8. Table 9-2 shows the results of the ELISA assay for interleukin-8 (IL-8) performed on placenta- and umbilical cord-derived cells as well as human skin fibroblasts. Values are presented here are picogram/million cells, n=2, sem. ND: Not Detected.

The greatest amounts of IL-8 were detected in media in which umbilical cells had been grown (Table 9-2). No IL-8 was detected in medium in which human dermal fibroblasts had been grown.

| **Table 9-2:** IL-8 protein expression measured by ELISA | |
|---|---|
| **Cell type** | **IL-8** |
| Human fibroblasts | ND |
| Placenta Isolate 1 | ND |
| UMBC Isolate 1 | 2058.42±144.67 |
| Placenta Isolate 2 | ND |
| UMBC Isolate 2 | 2368.86±22.73 |
| Placenta Isolate3 (normal O₂) | 17.27±8.63 |
| Placenta Isolate 3 (low O₂, W/O BME) | 264.92±9.88 |

Cells derived from the human umbilical cord at passage 0 were probed for the production of selected proteins by immunocytochemical analysis. Immediately after isolation (passage 0), cells were fixed with 4% paraformaldehyde and exposed to antibodies for six proteins: von Willebrand Factor, CD34, cytokeratin 18, desmin, alpha-smooth muscle actin, and vimentin. Umbilical cord-derived cells were positive for alpha-smooth muscle actin and vimentin, with the staining pattern consistent through passage 11.

The production of GROalpha, GCP-2, oxidized LDL receptor 1 and reticulon (NOGO-A) in umbilical cord-derived cells at passage 11 was investigated by immunocytochemistry. Umbilical cord-derived cells were GCP-2 positive, but GRO alpha production was not detected by this method. Furthermore, cells were NOGO-A positive.

Accordance between gene expression levels measured by microarray and PCR (both real-time and conventional) has been established for four genes: oxidized LDL receptor 1, renin, reticulon, and IL-8. The expression of these genes was differentially regulated at the mRNA level in umbilical cord-derived cells, with IL-8 also differentially regulated at the protein level. Differential expression of GCP-2 and CXC ligand 3 was not confirmed at the mRNA level. Although this result does not support data originally obtained from the microarray experiment, this may be due to a difference in the sensitivity of the methodologies.

Cells derived from the human umbilical cord at passage 0 were probed for the expression of alpha-smooth muscle actin and vimentin, and were positive for both. The staining pattern was preserved through passage 11.

In conclusion, the complete mRNA data at least partially verifies the data obtained from the microarray experiments.

### EXAMPLE 10

### Immunohistochemical Characterization of Cellular Phenotypes

The phenotypes of cells found within human umbilical cord tissue were analyzed by immunohistochemistry.

Human umbilical cord tissue was harvested and immersion fixed in 4% (w/v) paraformaldehyde overnight at 4°C. Immunohistochemistry was performed using antibodies directed against the following epitopes (*see* Table 10-1): vimentin (1:500; Sigma, St. Louis, MO), desmin (1:150, raised against rabbit; Sigma; or 1:300, raised against mouse; Chemicon, Temecula, CA), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, CA). In addition, the following markers were tested: anti-human GROalpha-PE (1:100; Becton Dickinson, Franklin Lakes, NJ), anti-human GCP-2 (1:100; Santa Cruz Biotech, Santa Cruz, CA), anti-human oxidized LDL receptor 1 (ox-LDL R1; 1:100; Santa Cruz Biotech), and anti-human NOGO-A (1:100; Santa Cruz Biotech). Fixed specimens were trimmed with a scalpel and placed within OCT embedding compound (Tissue-Tek OCT; Sakura, Torrance, CA) on a dry ice bath containing ethanol. Frozen blocks were then sectioned (10 microns thick) using a standard cryostat (Leica Microsystems) and mounted onto glass slides for staining.

Immunohistochemistry was performed similar to previous studies (e.g., Messina, et al. Exper. Neurol., 2003; 184: 816-829). Tissue sections were washed with phosphate-buffered saline (PBS) and exposed to a protein blocking solution containing PBS, 4% (v/v) goat serum (Chemicon, Temecula, CA), and 0.3% (v/v) Triton (Triton X-100; Sigma) for 1 hour to access intracellular antigens. In instances where the epitope of interest would be located on the cell surface (CD34, ox-LDL R1), triton was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the procedure. Primary antibodies, diluted in blocking solution, were then applied to the sections for a period of 4 hours at room temperature. Primary antibody solutions were removed, and cultures washed with PBS prior to application of secondary antibody solutions (1 hour at room temperature) containing block along with goat anti-mouse IgG-Texas Red (1:250; Molecular Probes, Eugene, OR) and/or goat anti-rabbit IgG-Alexa 488 (1:250; Molecular Probes) or donkey anti-goat IgG-FITC (1:150; Santa Cruz Biotech). Cultures were washed, and 10 micromolar DAPI (Molecular Probes) was applied for 10 minutes to visualize cell nuclei.

Following immunostaining, fluorescence was visualized using the appropriate fluorescence filter on an Olympus inverted epifluorescent microscope (Olympus, Melville, NY). Positive staining was represented by fluorescence signal above control staining. Representative images were captured using a digital color videocamera and ImagePro software (Media Cybernetics, Carlsbad, CA). For triple-stained samples, each image was taken using only one emission filter at a time. Layered montages were then prepared using Adobe Photoshop software (Adobe, San Jose, CA).

**Table 10-1: Summary of Primary Antibodies Used**

| **Antibody** | **Concentration** | **Vendor** |
|---|---|---|
| Vimentin | 1:500 | Sigma, St. Louis, MO |
| Desmin (rb) | 1:150 | Sigma |
| Desmin (m) | 1:300 | Chemicon, Temecula, CA |
| alpha-smooth muscle actin (SMA) | 1:400 | Sigma |
| Cytokeratin 18 (CK18) | 1:400 | Sigma |
| von Willebrand factor (vWF) | 1:200 | Sigma |
| CD34 III | 1:100 | DakoCytomation, Carpinteria, CA |
| GROalpha-PE | 1:100 | BD, Franklin Lakes, NJ |
| GCP-2 | 1:100 | Santa Cruz Biotech |
| Ox-LDL R1 | 1:100 | Santa Cruz Biotech |
| NOGO-A | 1:100 | Santa Cruz Biotech |

Vimentin, desmin, SMA, CK18, vWF, and CD34 markers were expressed in a subset of the cells found within umbilical cord (data not shown). In particular, vWF and CD34 expression were restricted to blood vessels contained within the cord. CD34+ cells were on the innermost layer (lumen side). Vimentin expression was found throughout the matrix and blood vessels of the cord. SMA was limited to the matrix and outer walls of the artery & vein, but not contained with the vessels themselves. CK18 and desmin were observed within the vessels only, desmin being restricted to the middle and outer layers.

None of these markers were observed within umbilical cord (data not shown).

Vimentin, desmin, alpha-smooth muscle actin, cytokeratin 18, von Willebrand Factor, and CD 34 are expressed in cells within human umbilical cord. Based on *in vitro* characterization studies showing that only vimentin and alpha-smooth muscle actin are expressed, the data suggests that the current process of umbilical cord-derived cell isolation harvests a subpopulation of cells or that the cells isolated change expression of markers to express vimentin and alpha-smooth muscle actin.

### EXAMPLE 11

### Secretion of Trophic Factors When UTC Are Grown in Culture

The secretion of selected trophic factors from umbilicus-derived cells grown in culture was measured. Factors were selected that have angiogenic activity (*i.e.*, hepatocyte growth factor (HGF) (Rosen et al. Ciba Found. Symp. 1997; 212:215-26), monocyte chemotactic protein 1(also known as monocyte chemoattractant-1) (MCP-1) (Salcedo et al. Blood, 2000; 96;34-40), interleukin-8 (IL-8) (Li et al. J. Immunol., 2003; 170:3369-76), keratinocyte growth factor (KGF), basic fibroblast growth factor (bFGF), vascular endothelial growth factor (VEGF) (Hughes et al. Ann. Thorac. Surg., 2004 77:812-8), tissue inhibitor of matrix metalloproteinase 1 (TIMP1), angiopoietin 2 (ANG2), platelet derived growth factor (PDGFbb), thrombopoietin (TPO), heparin-binding epidermal growth factor (HB-EGF), neurotrophic/neuroprotective activity (brain-derived neurotrophic factor (BDNF) (Cheng et al. Dev. Biol. , 2003; 258;319-33), stromal-derived factor 1alpha (SDF-1alpha), interleukin-6 (IL-6), granulocyte chemotactic protein-2 (GCP-2), transforming growth factor beta2 (TGFbeta2)), or chemokine activity (macrophage inflammatory protein 1alpha (MIP1alpha (MIP1α)), macrophage inflammatory protein 1 beta (MIP1beta (MIP1β)), RANTES (regulated on activation, normal T cell expressed and secreted), 1309, thymus and activation-regulated chemokine (TARC), Eotaxin, macrophage-derived chemokine (MDC).

Cells derived from umbilical cord, as well as human fibroblasts derived from human neonatal foreskin, were cultured in growth medium on gelatin-coated T75 flasks. Cells were cryopreserved at passage 11 and stored in liquid nitrogen. After thawing, growth medium was added to the cells, followed by transfer to a 15 milliliter centrifuge tube and centrifugation of the cells at 150 x g for 5 minutes. The cell pellet was resuspended in 4 milliliters growth medium, and cells were counted. Cells were seeded at 5,000 cells/cm² in T75 flasks each containing 15 milliliters of growth medium, and cultured for 24 hours. The medium was changed to a serum-free medium (DMEM-low glucose (Gibco), 0.1% (w/v) bovine serum albumin (Sigma), penicillin (50 Units/milliliter) and streptomycin (50 micrograms/milliliter, Gibco)) for 8 hours. Conditioned serum-free medium was collected at the end of incubation by centrifugation at 14,000 x g for 5 minutes and stored at -20°C.

To estimate the number of cells in each flask, the cells were washed with phosphate-buffered saline (PBS) and detached using 2 milliliters trypsin/EDTA (Gibco). Trypsin activity was inhibited by addition of 8 milliliters growth medium. The cells were centrifuged at 150 x g for 5 minutes. The supernatant was removed, and cells were resuspended in 1 milliliter growth medium. The cell number was estimated with a hemocytometer.

Cells were grown at 37°C in 5% carbon dioxide and atmospheric oxygen. The amount of MCP-1, IL-6, VEGF, SDF-1alpha, GCP-2, IL-8, and TGF-beta2 produced by each cell sample was determined by ELISA (R&D Systems, Minneapolis, MN). All assays were performed according to the manufacturer's instructions. Values presented are picograms per milliliter per million cells (n=2, sem).

Chemokines (MIP1alpha (MIP1α), MIP1beta (MIP1β), MCP-1, RANTES, 1309, TARC, Eotaxin, MDC, IL-8), BDNF, and angiogenic factors (HGF, KGF, bFGF, VEGF, TIMP1, ANG2, PDGFbb, TPO, HB-EGF were measured using SearchLight™ Proteome Arrays (Pierce Biotechnology Inc.). The Proteome Arrays are multiplexed sandwich ELISAs for the quantitative measurement of two to sixteen proteins per well. The arrays are produced by spotting a 2 x 2, 3 x 3, or 4 x 4 pattern of four to sixteen different capture antibodies into each well of a 96-well plate. Following a sandwich ELISA procedure, the entire plate is imaged to capture the chemiluminescent signal generated at each spot within each well of the plate. The signal generated at each spot is proportional to the amount of target protein in the original standard or sample.

MCP-1 and IL-6 were secreted by umbilicus-derived PPDCs and dermal fibroblasts (Table 11-1). SDF-1alpha (SDF-1α) and GCP-2 were secreted by fibroblasts. GCP-2 and IL-8 were secreted by umbilicus-derived PPDCs. TGF-beta2 was not detected from either cell type by ELISA.

| **Table 11-1.** ELISA Results: Detection of Trophic Factors | | | | | | | |
|---|---|---|---|---|---|---|---|
| | **MCP-1** | **IL-6** | **VEGF** | **SDF-1α** | **GCP-2** | **IL-8** | **TGF-beta2** |
| **Fibroblast** | 17±1 | 61±3 | 29±2 | 19±1 | 21±1 | ND | ND |
| **Umbilical (022803)** | 1150±74 | 4234±289 | ND | ND | 160±11 | 2058±145 | ND |
| **Umbilical (071003)** | 2794±84 | 1356±43 | ND | ND | 2184±98 | 2369±23 | ND |
| Key: ND: Not Detected., =/- sem | | | | | | | |

Searchlight™ Multiplexed ELISA assay. TIMP1, TPO, KGF, HGF, FGF, HBEGF, BDNF, MIP1beta, MCP1, RANTES, 1309, TARC, MDC, and IL-8 were secreted from umbilicus-derived PPDCs (*see* Tables 11-2 and 11-3 below) when grown in culture. No Ang2, VEGF, or PDGFbb were detected.

| **Table 11-2.** Searchlight™ Multiplexed ELISA assay results | | | | | | | | | | |
|---|---|---|---|---|---|---|---|---|---|---|
| | **TIMP1** | **ANG2** | **PDGFbb** | **TPO** | **KGF** | **HGF** | **FGF** | **VEGF** | **HBEGF** | **BDNF** |
| **hFB** | 19306.3 | ND | ND | 230.5 | 5.0 | ND | ND | 27.9 | 1.3 | ND |
| **U1** | 57718.4 | ND | ND | 1240.0 | 5.8 | 559.3 | 148.7 | ND | 9.3 | 165.7 |
| **U3** | 21850.0 | ND | ND | 1134.5 | 9.0 | 195.6 | 30.8 | ND | 5.4 | 388.6 |
| Key: hFB (human fibroblasts), U1 (umbilicus-derived PPDC (022803)), U3 (umbilicus-derived PPDC (071003)). | | | | | | | | | | |
| ND: Not Detected. | | | | | | | | | | |

| **Table 11-3.** Searchlight™ Multiplexed ELISA assay results | | | | | | | | | |
|---|---|---|---|---|---|---|---|---|---|
| | **MIP1α** | **MIP1β** | **MCP1** | **RANTES** | **I309** | **TARC** | **Eotaxin** | **MDC** | **IL8** |
| **hFB** | ND | ND | 39.6 | ND | ND | 0.1 | ND | ND | 204.9 |
| **U1** | ND | 8.0 | 1694.2 | ND | 22.4 | 37.6 | ND | 18.9 | 51930.1 |
| **U3** | ND | 5.2 | 2018.7 | 41.5 | 11.6 | 21.4 | ND | 4.8 | 10515.9 |
| Key: hFB (human fibroblasts), U1 (umbilicus-derived PPDC (022803)), U3 (umbilicus-derived PPDC (071003)). | | | | | | | | | |
| ND: Not Detected. | | | | | | | | | |

Umbilicus-derived cells secreted a number of trophic factors when cultured. Some of these trophic factors, such as HGF, bFGF, MCP-1 and IL-8, play important roles in angiogenesis. Other trophic factors, such as BDNF and IL-6, have important roles in neural regeneration or protection.

### EXAMPLE 12

### In Vitro Immunology

Umbilical cord cell lines were evaluated *in vitro* for their immunological characteristics in an effort to predict the immunological response, if any, these cells would elicit upon *in vivo* transplantation. Postpartum cell lines were assayed by flow cytometry for the expression of HLA-DR, HLA-DP, HLA-DQ, CD80, CD86, and B7-H2. These proteins are expressed by antigen-presenting cells (APC) and are required for the direct stimulation of naive CD4⁺ T cells (Abbas & Lichtman, CELLULAR AND MOLECULAR IMMUNOLOGY, 5th Ed. (2003) Saunders, Philadelphia, p. 171). The cell lines were also analyzed by flow cytometry for the expression of HLA-G (Abbas & Lichtman, *supra*), CD178 (Coumans, et.al., (1999) Journal of Immunological Methods 224, 185-196), and PD-L2 (Abbas & Lichtman, *supra*; Brown, et. al. The Journal of Immunology 170, 2003; 1257-1266). To predict the extent to which postpartum umbilicus-derived cell lines elicit an immune response *in vivo*, the cell lines were tested in a one-way mixed lymphocyte reaction (MLR).

Cells were cultured in Growth Media in T75 flasks (Corning, Corning, NY) coated with 2% gelatin (Sigma, St. Louis, MO) until confluent.

Cells were washed in phosphate buffered saline (PBS) (Gibco, Carlsbad, CA) and detached with Trypsin/EDTA (Gibco, Carlsbad, MO). Cells were harvested, centrifuged, and resuspended in 3% (v/v) FBS in PBS at a cell concentration of 1x10⁷ per milliliter. Antibody (Table 12-1) was added to one hundred microliters of cell suspension as per manufacturer's specifications and incubated in the dark for 30 minutes at 4°C. After incubation, cells were washed with PBS and centrifuged to remove unbound antibody. Cells were re-suspended in five hundred microliters of PBS and analyzed by flow cytometry using a FACSCalibur instrument (Becton Dickinson, San Jose, CA).

| **Table 12-1.** Antibodies | | |
|---|---|---|
| **Antibody** | **Manufacture** | **Catalog Number** |
| HLA-DR,DP,DQ | BD Pharmingen (San Diego, CA) | 555558 |
| CD80 | BD Pharmingen | 557227 |
| CD86 | BD Pharmingen | 555665 |
| B7-H2 | BD Pharmingen | 552502 |
| HLA-G | Abcam (Cambridgeshire, UK) | ab 7904-100 |
| CD178 | Santa Cruz (San Cruz, CA) | sc-19681 |
| PD-L2 | BD Pharmingen | 557846 |
| Mouse IgG2alpha | Sigma (St. Louis, MO) | F-6522 |
| Mouse IgG1kappa | Sigma | P-4685 |

Cryopreserved vials of passage 10 umbilicus-derived PPDCs labeled as cell line "A" were packaged on dry ice and sent to CTBR (Senneville, Quebec) to conduct a mixed lymphocyte reaction using CTBR SOP No. CAC-031. Peripheral blood mononuclear cells (PBMCs) were collected from multiple male and female volunteer donors. Six human volunteer blood donors were screened to identify a single allogeneic donor that exhibited a robust proliferation response in a mixed lymphocyte reaction with the other five blood donors. This donor was selected as the allogeneic positive control donor. The remaining five blood donors were selected as recipients. Stimulator (donor) allogeneic PBMC, autologous PBMC, and postpartum cell lines were treated with mitomycin C. Autologous and mitomycin C-treated stimulator cells were added to responder (recipient) PBMCs and cultured for 4 days. After incubation, [³H]thymidine was added to each sample and cultured for 18 hours. Following harvest of the cells, radiolabeled DNA was extracted, and [³H]-thymidine incorporation was measured using a scintillation counter. Reactions were performed in triplicate using two-cell culture plates with three receivers per plate

The stimulation index for the allogeneic donor (SIAD) was calculated as the mean proliferation of the receiver plus mitomycin C-treated allogeneic donor divided by the baseline proliferation of the receiver. The stimulation index of the postpartum cells was calculated as the mean proliferation of the receiver plus mitomycin C-treated postpartum cell line divided by the baseline proliferation of the receiver.

Six human volunteer blood donors were screened to identify a single allogeneic donor that will exhibit a robust proliferation response in a mixed lymphocyte reaction with the other five blood donors. This donor was selected as the allogeneic positive control donor. The remaining five blood donors were selected as recipients. The allogeneic positive control donor and umbilical cord-derived cell lines were mitomycin C-treated and cultured in a mixed lymphocyte reaction with the five individual allogeneic receivers. Reactions were performed in triplicate using two cell culture plates with three receivers per plate (Table 12-2). The average stimulation index ranged from 6.5 (plate 1) to 9 (plate 2) and the allogeneic donor positive controls ranged from 42.75 (plate 1) to 70 (plate 2) (Table 12-3).

**Table 12-2. Mixed Lymphocyte Reaction Data- Cell Line A (Umbilicus)**

| **DPM for Proliferation Assay (Plate 1)** | | | | | | | |
|---|---|---|---|---|---|---|---|
| Analytical Number | Culture System | Replicate | | | Mean | SD | CV |
| | | 1 | 2 | 3 | | | |
| IM04-2478 | Proliferation baseline of receiver | 1074 | 406 | 391 | 623.7 | 390.07 | 62.5 |
| | Control of autostimulation (Mitomycin C treated autologous cells) | 672 | 510 | 1402 | 861.3 | 475.19 | 55.2 |
| | MLR allogenic donor IM04-2477 (Mitomycin C treated) | 43777 | 48391 | 38231 | 43466.3 | 5087.12 | 11.7 |
| | MLR with cell line (Mitomycin C treated cell type A) | 2914 | 5622 | 6109 | 4881.7 | 1721.36 | 35.3 |
| SI (donor) | | | | | 70 | | |
| SI (cell line) | | | | | 8 | | |
| IM04-2479 | Proliferation baseline of receiver | 530 | 508 | 527 | 521.7 | 11.93 | 2.3 |
| | Control of autostimulation (Mitomycin C treated autologous cells) | 701 | 567 | 1111 | 793.0 | 283.43 | 35.7 |
| | MLR allogenic donor IM04-2477 (Mitomycin C treated) | 25593 | 24732 | 22707 | 24344.0 | 1481.61 | 6.1 |
| | MLR with cell line (Mitomycin C treated cell type A) | 5086 | 3932 | 1497 | 3505.0 | 1832.21 | 52.3 |

| Analytical | Culture System | Replicate | | | Mean | SD | CV |
|---|---|---|---|---|---|---|---|
| SI (donor) | | | | | 47 | | |
| SI (cell line) | | | | | 7 | | |
| IM04-2480 | Proliferation baseline of receiver | 1192 | 854 | 1330 | 1125.3 | 244.90 | 21.8 |
| | Control of autostimulation (Mitomycin C treated autologous cells) | 2963 | 993 | 2197 | 2051.0 | 993.08 | 48.4 |
| | MLR allogenic donor IM04-2477 (Mitomycin C treated) | 25416 | 29721 | 23757 | 26298.0 | 3078.27 | 11.7 |
| | MLR with cell line (Mitomycin C treated cell type A) | 2596 | 5076 | 3426 | 3699.3 | 1262.39 | 34.1 |
| SI (donor) | | | | | 23 | | |
| SI (cell line) | | | | | 3 | | |
| IM04-2481 | Proliferation baseline of receiver | 695 | 451 | 555 | 567.0 | 122.44 | 21.6 |
| | Control of autostimulation (Mitomycin C treated autologous cells) | 738 | 1252 | 464 | 818.0 | 400.04 | 48.9 |
| | MLR allogenic donor IM04-2477 (Mitomycin C treated) | 13177 | 24885 | 15444 | 17835.3 | 6209.52 | 34.8 |
| | MLR with cell line (Mitomycin C treated cell type A) | 4495 | 3671 | 4674 | 4280.0 | 534.95 | 12.5 |
| SI (donor) | | | | | 31 | | |
| SI (cell line) | | | | | 8 | | |

| **DPM for Proliferation Assay (Plate 2)** | | | | | | | |
|---|---|---|---|---|---|---|---|
| Analytical Number | Culture System | Replicates | | | Mean | SD | CV |
| | | 1 | 2 | 3 | | | |
| IM04-2482 | Proliferation baseline of receiver | 432 | 533 | 274 | 413.0 | 130.54 | 31.6 |
| | Control of autostimulation (Mitomycin C treated autologous cells) | 1459 | 633 | 598 | 896.7 | 487.31 | 54.3 |
| | MLR allogenic donor IM04-2477 (Mitomycin C treated) | 24286 | 30823 | 31346 | 28818.3 | 3933.82 | 13.7 |
| | MLR with cell line (Mitomycin C treated cell type A) | 2762 | 1502 | 6723 | 3662.3 | 2724.46 | 74.4 |
| SI (donor) | | | | | 70 | | |
| SI (cell line) | | | | | 9 | | |
| IM04-2477 (allogenic donor) | Proliferation baseline of receiver | 312 | 419 | 349 | 360.0 | 54.34 | 15.1 |
| | Control of autostimulation (Mitomycin C treated autologous cells) | 567 | 604 | 374 | 515.0 | 123.50 | 24.0 |
| | | | | | | | |
| Cell line type A | Proliferation baseline of receiver | 5101 | 3735 | 2973 | 3936.3 | 1078.19 | 27.4 |
| | Control of autostimulation (Mitomycin C treated autologous cells) | 1924 | 4570 | 2153 | 2882.3 | 1466.04 | 50.9 |

| **Table 12-3. Average stimulation index of umbilical cells and an allogeneic donor in a mixed lymphocyte reaction with five individual allogeneic receivers** | | |
|---|---|---|
| | Recipient | Umbilicus |
| Plate 1 (receivers1-4) | 42.75 | 6.5 |
| Plate 2 (receiver 5) | 70 | 9 |

Histograms of umbilical cord-derived cells analyzed by flow cytometry show negative expression of HLA-DR, DP, DQ, CD80, CD86, and B7-H2, as noted by fluorescence value consistent with the IgG control, indicating that umbilical cord-derived cell lines lack the cell surface molecules required to directly stimulate allogeneic PBMCs (*e*.*g*., CD4⁺ T cells).

The umbilical cells analyzed by flow cytometry were positive for expression of PD-L2, as reflected in the increase in fluorescence relative to the IgG control. The cells were negative for expression of CD178 and HLA-G, as noted by fluorescence values consistent with the IgG control.

In the mixed lymphocyte reactions conducted with umbilical cell lines the average stimulation index ranged from 6.5 to 9, while that of the allogeneic positive controls ranged from 42.75 to 70. Umbilical cell lines did not express detectable amounts of the stimulating proteins HLA-DR, HLA-DP, HLA-DQ, CD80, CD86, and B7-H2, as measured by flow cytometry. Umbilical cell lines also did not express the immuno-modulating proteins HLA-G and CD178, but expression of PD-L2 was detected by flow cytometry. Allogeneic donor PBMCs contain antigen-presenting cells expressing HLA-DR, DQ, CD8, CD86, and B7-H2, thereby allowing for the stimulation of allogeneic lymphocytes. The absence on umbilicus-derived cells of antigen-presenting cell surface molecules required for the direct stimulation of naive CD4⁺ T cells, as well as the presence of PD-L2, an immunomodulating protein, could account for the low stimulation index exhibited by these cells in a MLR as compared to allogeneic controls.

### EXAMPLE 13

### Assay for Telomerase Activity

Telomerase functions to synthesize telomere repeats that serve to protect the integrity of chromosomes and to prolong the replicative life span of cells (Liu, K, et al., PNAS, 1999; 96:5147-5152). Telomerase consists of two components, telomerase RNA template (hTER) and telomerase reverse transcriptase (hTERT). Regulation of telomerase is determined by transcription of hTERT but not hTER. Real-time polymerase chain reaction (PCR) for hTERT mRNA thus is an accepted method for determining telomerase activity of cells.

### Cell Isolation

Real-time PCR experiments were performed to determine telomerase production of human umbilical cord tissue-derived cells. Human umbilical cord tissue-derived cells were prepared in accordance with the above Examples and the examples set forth in U.S. Patent No. 7,510,873. Generally, umbilical cords obtained from National Disease Research Interchange (Philadelphia, Pa.) following a normal delivery were washed to remove blood and debris and mechanically dissociated. The tissue was then incubated with digestion enzymes including collagenase, dispase, and hyaluronidase in culture medium at 37°C. Human umbilical cord tissue-derived cells were cultured according to the methods set forth in the examples of the '012 application. Mesenchymal stem cells and normal dermal skin fibroblasts (cc-2509 lot # 9F0844) were obtained from Cambrex, Walkersville, Md. A pluripotent human testicular embryonal carcinoma (teratoma) cell line nTera-2 cells (NTERA-2 cl.Dl) (*See*, Plaia et al., Stem Cells, 2006; 24(3):531-546) was purchased from ATCC (Manassas, Va.) and was cultured according to the methods set forth in U.S. Patent No. 7,510,873.

### Total RNA Isolation

RNA was extracted from the cells using RNeasy® kit (Qiagen, Valencia, Ca.). RNA was eluted with 50 microliters DEPC-treated water and stored at -80°C. RNA was reverse transcribed using random hexamers with the TaqMan® reverse transcription reagents (Applied Biosystems, Foster City, Ca.) at 25°C for 10 minutes, 37°C for 60 minutes and 95°C for 10 minutes. Samples were stored at -20°C.

### Real-time PCR

PCR was performed on cDNA samples using the Applied Biosystems Assays-On-Demand™ (also known as TaqMan® Gene Expression Assays) according to the manufacturer's specifications (Applied Biosystems). This commercial kit is widely used to assay for telomerase in human cells. Briefly, hTert (human telomerase gene) (Hs00162669) and human GAPDH (an internal control) were mixed with cDNA and TaqMan® Universal PCR master mix using a 7000 sequence detection system with ABI prism 7000 SDS software (Applied Biosystems). Thermal cycle conditions were initially 50°C for 2 minutes and 95°C for 10 minutes followed by 40 cycles of 95°C for 15 seconds and 60°C for 1 minute. PCR data was analyzed according to the manufacturer's specifications.

Human umbilical cord tissue-derived cells (ATCC Accession No. PTA-6067), fibroblasts, and mesenchymal stem cells were assayed for hTert and 18S RNA. As shown in Table 13-1, hTert, and hence telomerase, was not detected in human umbilical cord tissue-derived cells.

| **Table 13-1** | | |
|---|---|---|
| | hTert | 18S RNA |
| Umbilical cells (022803) | ND | + |
| Fibroblasts | ND | + |
| ND- not detected; + signal detected | | |

Human umbilical cord tissue-derived cells (isolate 022803, ATCC Accession No. PTA-6067) and nTera-2 cells were assayed and the results showed no expression of the telomerase in two lots of human umbilical cord tissue-derived cells while the teratoma cell line revealed high level of expression (Table 13-2).

| **Table 13-2** | | | | | |
|---|---|---|---|---|---|
| Cell type | hTert | | GAPDH | | hTert norm |
| | Exp. 1 | Exp. 2 | Exp. 1 | Exp. 2 | |
| nTera2 | 25.85 | 27.31 | 16.41 | 16.31 | 0.61 |
| 022803 | - | - | 22.97 | 22.79 | - |

Therefore, it can be concluded that the human umbilical tissue-derived cells of the present invention do not express telomerase.

## Claims

1. Umbilical cord tissue-derived cells for use in treating chronic obstructive pulmonary disease (COPD) by reducing the production of one or more pro-inflammatory mediators involved in the pathology of COPD, wherein the one or more pro-inflammatory mediators is selected from the group consisting of TNF-α, RANTES, IL-1β and combinations thereof, and wherein the cells are isolated from human umbilical cord tissue substantially free of blood, are capable of self-renewal and expansion in culture, lack the production of CD117 and CD45, and do not express hTERT or telomerase.

2. The umbilical cord tissue-derived cells for use according to claim 1, wherein the chronic obstructive pulmonary disease is chronic bronchitis or emphysema.

3. The umbilical cord tissue-derived cells for use according to claim 1, wherein the cells further comprise one or more of the following characteristics:
(a) express CD10, CD13, CD44, CD73, and CD90;
(b) do not express CD31 or CD34;
(c) express, relative to a human fibroblast, mesenchymal stem cell, or iliac crest bone marrow cell, increased levels of interleukin 8 or reticulon 1; and
(d) have the potential to differentiate into cells of at least a lung tissue.

4. The umbilical cord tissue-derived cells for use according to any one of claims 1-3, wherein the reducing comprises administering the umbilical cord tissue-derived cells with at least one other cell type and/or at least one other agent.

5. The umbilical cord tissue-derived cells for use according to claim 4, wherein the other cell type is a lung tissue cell selected from lung progenitor cell, vascular smooth muscle cell, vascular smooth muscle progenitor cell, pericyte, vascular endothelial cell, vascular endothelium progenitor cell, or other multipotent stem cell.

6. The umbilical cord tissue-derived cells for use according to claim 4, wherein the agent is an antithrombogenic agent, an anti-inflammatory agent, an immunosuppressive agent, an immunomodulatory agent, a pro-angiogenic agent, or an antiapoptotic agent.

7. The umbilical cord tissue-derived cells for use according to any one of claims 1-4, wherein modulating comprises administering the cells by injection, infusion, a device implanted in the patient, or by implantation of a matrix or scaffold containing the cells.

8. The umbilical cord tissue-derived cells for use according to any one of claims 1-4, wherein the cells exert a trophic effect on the lung tissue, vascular smooth muscle, or vascular endothelium of the patient.

9. The umbilical cord tissue-derived cells for use according to claim 1, wherein the reducing comprises administering the umbilical cord tissue-derived cells at the sites of the chronic obstructive pulmonary disease.

## Patentansprüche

1. Nabelschnurgewebe-abgeleitete Zellen zur Verwendung bei der Behandlung von chronisch-obstruktiver Lungenerkrankung (COPD) durch Verringern der Produktion von einem oder mehreren proinflammatorischen Mediatoren, die an der Pathologie von COPD beteiligt sind, wobei der eine oder die mehreren proinflammatorischen Mediatoren ausgewählt sind aus der Gruppe, bestehend aus TNF-α, RANTES, IL-1β und Kombinationen davon, und wobei die Zellen aus humanem Nabelschnurgewebe, das im Wesentlichen frei von Blut ist, isoliert werden, zur Selbsterneuerung und Expansion in Kultur imstande sind, keine Produktion von CD117 und CD45 aufweisen, und hTERT oder Telomerase nicht exprimieren.

2. Nabelschnurgewebe-abgeleitete Zellen zur Verwendung nach Anspruch 1, wobei es sich bei der chronisch-obstruktiven Lungenerkrankung um chronische Bronchitis oder Emphysem handelt.

3. Nabelschnurgewebe-abgeleitete Zellen zur Verwendung nach Anspruch 1, wobei die Zellen ferner eine oder mehrere der folgenden Eigenschaften umfassen:
(a) sie exprimieren CD10, CD13, CD44, CD73 und CD90;
(b) sie exprimieren nicht CD31 oder CD34;
(c) sie exprimieren, relativ zu einem menschlichen Fibroblasten, einer mesenchymalen Stammzelle oder einer Beckenkamm-Knochenmarkszelle, erhöhte Spiegel von Interleukin 8 oder Reticulon 1; und
(d) sie weisen das Potential auf, sich zu Zellen von mindestens einem Lungengewebe zu differenzieren.

4. Nabelschnurgewebe-abgeleitete Zellen zur Verwendung nach einem der Ansprüche 1-3, wobei das Verringern die Verabreichung der Nabelschnurgewebe-abgeleiteten Zellen mit mindestens einem anderen Zelltyp und/oder mindestens einem anderen Mittel umfasst.

5. Nabelschnurgewebe-abgeleitete Zellen zur Verwendung nach Anspruch 4, wobei der andere Zelltyp eine Lungengewebszelle ist, ausgewählt aus einer Lungenprogenitorzelle, vaskulären glatten Muskelzelle, vaskulären glatten Muskelprogenitorzelle, Perizyte, vaskulären Endothelzelle, vaskulären Endothelprogenitorzelle oder einer anderen multipotenten Stammzelle.

6. Nabelschnurgewebe-abgeleitete Zellen zur Verwendung nach Anspruch 4, wobei das Mittel ein antithrombogenes Mittel, ein entzündungshemmendes Mittel, ein immunsuppressives Mittel, ein immunmodulatorisches Mittel, ein pro-angiogenes Mittel oder ein antiapoptotisches Mittel ist.

7. Nabelschnurgewebe-abgeleitete Zellen zur Verwendung nach einem der Ansprüche 1-4, wobei das Modulieren die Verabreichung der Zellen durch Injektion, Infusion, eine in den Patienten implantierte Vorrichtung oder durch Implantation einer die Zellen enthaltenden Matrix oder Gerüststruktur umfasst.

8. Nabelschnurgewebe-abgeleitete Zellen zur Verwendung nach einem der Ansprüche 1-4, wobei die Zellen einen trophischen Effekt auf das Lungengewebe, die vaskuläre glatte Muskulatur oder das vaskuläre Endothel des Patienten ausüben.

9. Nabelschnurgewebe-abgeleitete Zellen zur Verwendung nach Anspruch 1, wobei das Verringern die Verabreichung der Nabelschnurgewebe-abgeleiteten Zellen an den Stellen der chronisch-obstruktiven Lungenerkrankung umfasst.

## Revendications

1. Cellules dérivées de tissu ombilical pour utilisation dans le traitement de la bronchopneumopathie chronique obstructive (BPCO) par réduction de la production d'un ou plusieurs médiateurs pro-inflammatoires impliqués dans la pathologie de BPCO, dans lesquelles les un ou plusieurs médiateurs pro-inflammatoire sont choisis dans le groupe constitué de TNF-α, RANTES, IL-1β et des combinaisons de ceux-ci, et les cellules sont isolées à partir de tissu ombilical humain sensiblement exempt de sang, sont capables d'autorenouvellement et d'expansion en culture, ne présentent pas la production de CD117 et CD45, et n'expriment pas hTERT ou la télomérase.

2. Cellules dérivées de tissu ombilical pour utilisation selon la revendication 1, la bronchopneumopathie chronique obstructive étant la bronchite chronique ou l'emphysème.

3. Cellules dérivées de tissu ombilical pour utilisation selon la revendication 1, les cellules comprenant en outre une ou plusieurs des caractéristiques suivantes :
(a) expriment CD10, CD13, CD44, CD73 et CD90 ;
(b) n'expriment pas CD31 ou CD34 ;
(c) expriment, par rapport à un fibroblaste, une cellule souche mésenchymateuse ou une cellule de moelle osseuse de crête iliaque humains, des taux accrus d'interleukine 8 ou de réticulon 1 ; et
(d) ont le potentiel pour se différencier en cellules d'au moins un tissu pulmonaire.

4. Cellules dérivées de tissu ombilical pour utilisation selon l'une quelconque des revendications 1 à 3, la réduction comprenant l'administration des cellules dérivées de tissu ombilical avec au moins un autre type de cellule et/ou au moins un autre agent.

5. Cellules dérivées de tissu ombilical pour utilisation selon la revendication 4, dans lesquelles l'autre type de cellule est une cellule de tissu pulmonaire choisie parmi une cellule progénitrice pulmonaire, une cellule de muscle lisse vasculaire, une cellule progénitrice de muscle lisse vasculaire, un péricyte, une cellule endothéliale vasculaire, une cellule progénitrice d'endothélium vasculaire, ou une autre cellule souche multipotente.

6. Cellules dérivées de tissu ombilical pour utilisation selon la revendication 4, dans lesquelles l'agent est un agent antithrombogène, un agent anti-inflammatoire, un agent immunosuppresseur, un agent immunomodulateur, un agent proangiogénique ou un agent antiapoptotique.

7. Cellules dérivées de tissu ombilical pour utilisation selon l'une quelconque des revendications 1 à 4, dans lesquelles la modulation comprend l'administration des cellules par injection, perfusion, un dispositif implanté dans le patient, ou par implantation d'une matrice ou d'un échafaudage contenant les cellules.

8. Cellules dérivées de tissu ombilical pour utilisation selon l'une quelconque des revendications 1 à 4, les cellules exerçant un effet trophique sur le tissu pulmonaire, le muscle lisse vasculaire ou l'endothélium vasculaire du patient.

9. Cellules dérivées de tissu ombilical pour utilisation selon la revendication 1, la réduction comprenant l'administration des cellules dérivées de tissu ombilical aux sites de la bronchopneumopathie chronique obstructive.
